# EUROPEAN PATENT SPECIFICATION

(11) **EP 1 020 426 B1**
(45) Date of publication and mention of the grant of the patent: **13.07.2005**
(21) Application number: 98936667.9
(22) Date of filing: 05.08.1998
(51) Int. Cl.: C07C 211/26, C07C 209/68, C07C 225/04, C07C 221/00, A61K 31/135

(54) **AMINE DERIVATIVES AND PROCESS FOR PRODUCING THE SAME**
AMINDERIVATE UND VERFAHREN ZU IHRER HERSTELLUNG
DERIVES D'AMINE ET LEUR PROCEDE DE PRODUCTION

(30) Priority: 05.08.1997 JP 22308797; 06.04.1998 JP 9356798
(43) Date of publication of application: 19.07.2000
(60) Divisional application: 05009223.8
(73) Proprietor: POLA CHEMICAL INDUSTRIES INC., Shizuoka-shi, Shizuoka 420-0914 (JP)
(72) Inventor: ITOH, Takao, Pola Chemical Industries Inc., Yokohama-shi, Kanagawa 244-0812 (JP); NAKASHIMA, Takuji, Pola Chemical Industries Inc., Yokohama-shi, Kanagawa 244-0812 (JP); NOZAWA, Akira, Pola Chemical Industries Inc., Yokohama-shi, Kanagawa 244-0812 (JP); YOKOYAMA, Kouji, Pola Chemical Industries Inc., Yokohama-shi, Kanagawa 244-0812 (JP); TAKIMOTO, Hiroyuki, Pola Chemical Industries Inc., Yokohama-shi, Kanagawa 244-0812 (JP); YUASA, Masayuki, Pola Chemical Industries Inc., Yokohama-shi, Kanagawa 244-0812 (JP); KAWAZU, Yukio, Pola Chemical Industries Inc., Yokohama-shi, Kanagawa 244-0812 (JP); SUZUKI, Toshimitsu, Pola Chemical Industries Inc., Yokohama-shi, Kanagawa 244-0812 (JP); MAJIMA, Toshiro, Pola Chemical Industries Inc., Yokohama-shi, Kanagawa 244-0812 (JP)
(74) Representative: Bannerman, David Gardner
(86) International application number: PCT/JP1998/003487
(87) International publication number: WO 1999/007666

(56) References cited:
- CH-A- 656 378
- DE-A- 2 809 211
- DE-A- 3 720 317
- JP-A- 2 209 848
- JP-A- 5 148 137
- US-A- 3 933 803
- STUETZ A ET AL: "SYNTHESIS AND STRUCTURE-ACTIVITY RELATIONSHIPS OF NAFTIFINE-RELATED ALLYLAMINE ANTIMYCOTICS" JOURNAL OF MEDICINAL CHEMISTRY, AMERICAN CHEMICAL SOCIETY. WASHINGTON, US, vol. 29, no. 1, 1986, pages 112-125, XP002037080 ISSN: 0022-2623
- HISASHI TAKAHASHI et al., "Highly Efficient Asymmetric Hydrogenation of Amino Ketone Derivatives Leading to Practical Syntheses of (S)-Propranolol and Related Compounds", JOURNAL OF THE AMERICAN CHEMICAL SOCIETY, Vol. 112, No. 15, pages 5876-5878, XP002914620

## Description

The present invention relates to novel amine derivatives having an excellent antimycotic effect and to a method for producing the same.

An increasing number of patients are suffering from superficial mycosis, whose representative example is athlete's foot (*tinea* pedis), due to a prolonged time in which shoes are put on. However, no certain therapeutical method or remedy therefor has been found and currently it is enumerated as one of diseases that remain to be overcome. So far, great effort has been made to find their remedy and many compounds have been subjected to screening for their antimycotic effect. Although, some substances show antimycotic activity in vitro or vivo using animals, most of them do not show the antimycotic activity in real clinical tests, resulting in stopping the development of the remedy. Therefore, only a limited number of substances showed satisfactory antimycotic results.

Generally-related amine derivatives are shown in J.Med.Chem., 1986, vol.29, p.112-125, and also in DE-A-4216644 and DE-A-3720317.

An object of the present invention is to provide novel compounds having an excellent antimycotic effect and a pharmaceutical composition containing them.

Under the circumstances, the present inventors have made intensive investigation, and as a result of which, the present inventors have found that the amine derivatives represented by general formula (1) set forth below have an excellent antimycotic effect, thus completing the present invention.

That is, the present invention provides amine derivatives represented by general formula (1) below or salts thereof: 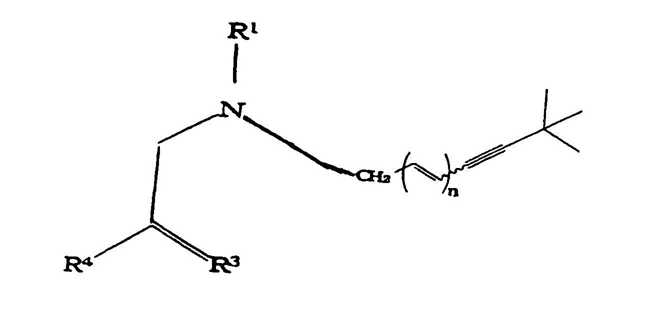 [wherein in the formula (1) above, R¹ represents a linear C₁₋₅ alkyl group, a branched alkyl group having up to 5 carbon atoms, or a cyclic alkyl group having up to 5 carbon atoms, said alkyl group may be halogenated; and " 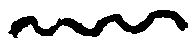 " indicates that the arrangement of double bond may be either *cis* or *trans*, and n is an integer of 1 to 3,
R³ is an oxygen atom or a group represented by the formula (f); 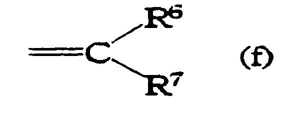 wherein each of R⁶ and R⁷ independently represents a hydrogen atom, a linear C₁₋₄ alkyl group, a branched alkyl group having up to 4 carbon atoms or a cyclic alkyl group having up to 4 carbon atoms; R⁴ represents a group of the formula (g), (h), or (i), 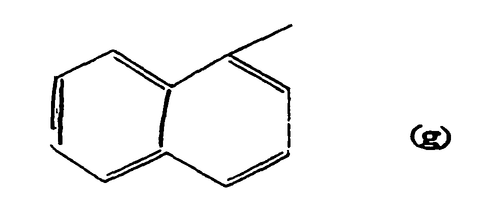 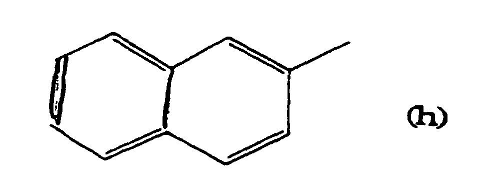 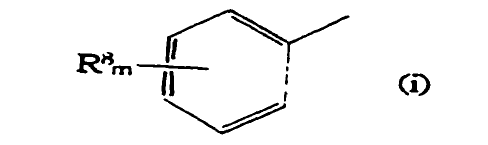 wherein in the formula (i), R⁸ is a substituent which is a linear C₁₋₇ alkyl group, a branched alkyl group having up to 7 carbon atoms, a cyclic alkyl group having up to 7 carbon atoms, a halogen atom, a linear C₁₋₄ alkoxy group, a branched alkoxy group having up to 4 carbon atoms, a cyclic alkoxy group having up to 4 carbon atoms, a nitro group, an amino group, a cyano group, a carboxyl group, a linear C₂₋₅ alkoxycarbonyl group, a branched alkoxycarbonyl group having from 2 to 5 carbon atoms, a cyclic alkoxycarbonyl group having from 2 to 5 carbon atoms, a hydroxyl group, or a group represented by R⁹₃SiO- where R⁹ is a linear C₁₋₄ alkyl group, a branched alkyl group having up to 4 carbon atoms or a cyclic alkyl group having up to 4 carbon atoms, in which the three R⁹ groups may be the same or different; and m is an integer from 0 to 5.

The amine derivatives or salts thereof of the present invention specifically include: amine derivatives represented by the general formula (1) in which R¹ is a linear C₁₋₅ alkyl group, a branched C₁₋₅ alkyl group or a cyclic C₁₋₅ alkyl group; R² is a group represented by the formula (d) or (e); R⁴ is a group represented by the formula (i); and R³, R⁸ in the formula (i), m, and n in the formula (d) or (e) are as defined above, or salts thereof.

Further, the present invention provides a method for producing amine derivatives represented by general formula (1) or salts thereof, comprising condensing a compound represented by general formula (2) 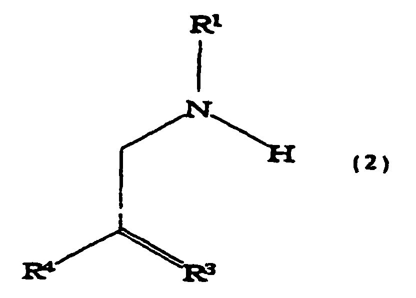 with a compound represented by general formula (3) 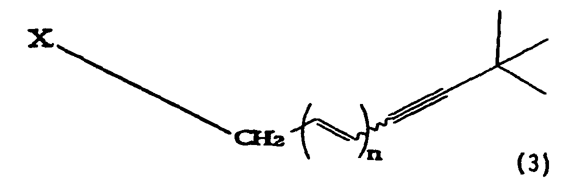 where X represents a halogen atom.

Still further, the present invention provides a method for producing amine derivatives represented by general formula (1) or salts thereof by condensing a compound represented by general formula (4) 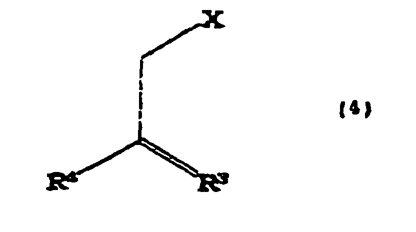 with a compound represented by general formula (5) 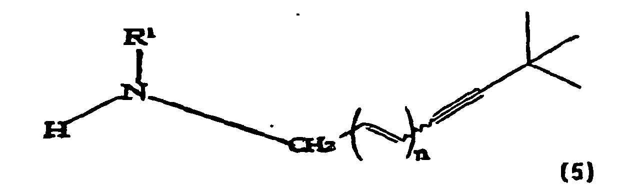 where X represents a halogen atom.

The present invention provides an antimycotic agent consisting of the amine derivatives or salts thereof.

The present invention provides an antimycotic agent or composition comprising the above-defined amine derivatives or salts thereof.

The present invention provides a pharmaceutical composition comprising, as an active ingredient, the above-defined amine derivatives or salts thereof.

Hereinafter, the present invention will be described in detail. First, the amine derivatives of the present invention and salts thereof will be explained.

### (1) Amine Derivatives of the Present Invention and Salts thereof

The amine derivatives of the present invention are compounds represented by the general formula (1) above. R¹, R², R³, and R⁴ in the formula (1) can be selected without restriction from the atoms or groups within the above-described ranges.

The amine derivatives of the invention are compounds represented by the general formula (1) above in which R¹, R³, and R⁴ are selected from the atoms or groups set forth below.

R1 represents a linear C₁₋₅ alkyl group, a branched alkyl group having up to 5 carbon atoms, or a cyclic alkyl group having up to 5 carbon atoms and specific examples thereof include a methyl group, an ethyl group, an n-propyl group, an i-propyl group, a cyclopropyl group, an n-butyl group, an i-butyl group, a sec-butyl group, a tert-butyl group, a cyclobutyl group, etc. Among these, a C₁₋₃ alky group are preferred, in particular, a methyl group, an ethyl group, an i-propyl group, or a cyclopropyl group.

R³ is an oxygen atom or a group represented by the formula (f). In the formula (f), R⁶ and R⁷ independently represent a hydrogen atom, or a linear C₁₋₄ alkyl group, a branched alkyl group having up to 4 carbon atoms, or a cyclic alkyl group having up to 4 carbon atoms. Specific examples of R¹ include, preferably, an oxygen atom, a carbon atom to which two hydrogen atoms are added, a carbon atom to which a hydrogen atom and a methyl group are added, and a carbon atom to which two methyl groups are added. Of these, a carbon atom to which an oxygen atom and two hydrogen atoms are added is particularly preferred.

R⁴ is a group represented by the formula (i). In the formula (i), R⁸ is a substituent group which substitutes for hydrogen atom(s) of the phenyl group in the formula (i) and represents a linear c₁₋₇ alkyl group, a branched alkyl group having up to 7 carbon atoms, a cyclic alkyl group having up to 7 carbon atoms, a halogen atom, a linear C₁₋₄ alkoxy group, a branched alkoxy group having up to 4 carbon atoms, a cyclic alkoxy group having up to 4 carbon atoms, a nitro group, an amino group which may have a substituent group, a cyano group, a carboxyl group, a linear C₂₋₅ alkoxycarbonyl group, a branched alkoxycarbonyl group having up to 5 carbon atoms, a cyclic alkoxycarbonyl group having up to 5 carbon atoms, a hydroxyl group, or a group represented by R⁹₃SiO-. R⁹ represents a C₁₋₄ alkyl group, a branched alkyl group having up to 4 carbon atoms, or a cyclic alkyl group having up to 4 carbon atoms, in which three of R⁹ may be the same or different; m is O or an integer of 1-5; and m of R⁸ may be the same or different. In the present specification, the number of carbons in "C₂₋₅ alkoxycarbonyl group" is counted as including that of carbonyl group part.

More specifically, preferred examples of R⁸ include a methyl group, a tert-butyl group, a fluorine atom, a chlorine atom, a bromine atom, a methoxy group, a nitro group, an amino group, a cyano group, an ethoxycarbonyl group, a hydroxyl group, a tert-butyldimethylsilyl group, and so forth.

Preferred specific examples of the amine derivatives of the invention as described above include the following compounds:
Trans-2-[N-(6,6-dimethyl-2-hepten-4-ynyl)-N-methylamino]acetophenone (hereinafter, sometimes referred to as "Compound PR-1130". Similarly, abbreviation will be described after the name of substance.);
Trans-N-(6,6-dimethyl-2-hepten-4-ynyl)-N-methyl-2-phenyl-2-propenylamine (Compound PR-1257);
Trans-2-[N-(6,6-dimethyl-2-hepten-4-ynyl)-N-methylamino]-2'-methylacetophenone (Compound PR-1531);
Trans-N-(6,6-dimethyl-2-hepten-4-ynyl)-N-methyl-[2-(o-tolyl)-2-propenyl]amine ("Compound PR-1532");
Trans-2-[N-(6,6-dimethyl-2-hepten-4-ynyl)-N-methylamino]-3'-methylacetophenone (Compound PR-1538);
Trans-N-(6,6-dimethyl-2-hepten-4-ynyl)-N-methyl-[2-(m-tolyl)-2-propenyl]amine (Compound PR-1539);
Trans-2-[N-(6,6-dimethyl-2-hepten-4-ynyl)-N-methylamino]-4'-methylacetophenone (Compound PR-1413);
Trans-N-(6,6-dimethyl-2-hepten-4-ynyl)-N-methyl-[2-(p-tolyl)-2-propenyl]amine (Compound PR-1414);
Trans-2-[N-(6,6-dimethyl-2-hepten-4-ynyl)-N-methylamino]-2'-fluoroacetophenone (Compound PR-1489);
Trans-N-(6,6-dimethyl-2-hepten-4-ynyl)-N-methyl-[2-(2-fluorophenyl)-2-propenyl]amine (Compound PR-1490);
Trans-2-[N-(6,6-dimethyl-2-hepten-4-ynyl)-N-methylamino]-3'-fluoroacetophenone (Compound PR-1468);
Trans-N-(6,6-dimethyl-2-hepten-4-ynyl)-N-methyl-[2-(3-fluorophenyl)-2-propenyl]amine (Compound PR-1469);
Trans-2-[N-(6,6-dimethyl-2-hepten-4-ynyl)-N-methylamino]-4'-fluoroacetophenone (Compound PR-1428);
Trans-N-(6,6-dimethyl-2-hepten-4-ynyl)-N-methyl-[2-(4-fluorophenyl)-2-propenyl]amine (Compound PR-1429);
Trans-2'-bromo-2-[N-(6,6-dimethyl-2-hepten-4-ynyl)-N-methylamino]acetophenone (Compound PR-1503);
Trans-N-(6,6-dimethyl-2-hepten-4-ynyl)-N-methyl-[2-(2-bromophenyl)-2-propenyl]amine (Compound PR-1504);
Trans-3'-bromo-2-[N-(6,6-dimethyl-2-hepten-4-ynyl)-N-methylamino]acetophenone (Compound PR-1482);
Trans-N-(6,6-dimethyl-2-hepten-4-ynyl)-N-methyl-[2-(3-bromophenyl)-2-propenyl]amine (Compound PR-1483);
Trans-4'-bromo-2-[N-(6,6-dimethyl-2-hepten-4-ynyl)-N-methylamino]acetophenone (Compound PR-1437);
Trans-N-(6,6-dimethyl-2-hepten-4-ynyl)-N-methyl-[2-(4-bromophenyl)-2-propenyl]amine (Compound PR-1438);
Trans-2'-chloro-2-[N-(6,6-dimethyl-2-hepten-4-ynyl)-N-methylamino]acetophenone (Compound PR-1496);
Trans-N-(6,6-dimethyl-2-hepten-4-ynyl)-N-methyl-[2-(2-chlorophenyl)-2-propenyl]amine (Compound PR-1497);
Trans-4'-chloro-2-[N-(6,6-dimethyl-2-hepten-4-ynyl)-N-methylamino]acetophenone (Compound PR-1416);
Trans-N-(6,6-dimethyl-2-hepten-4-ynyl)-N-methyl-[2-(4-chlorophenyl)-2-propenyl]amine (Compound PR-1417);
Trans-2-[N-(6,6-dimethyl-2-hepten-4-ynyl)-N-methylamino]-2'-methoxyacetophenone (Compound PR-1632);
Trans-N-(6,6-dimethyl-2-hepten-4-ynyl)-N-methyl-[2-(2-methoxyphenyl)-2-propenyl]amine (Compound PR-1633);
Trans-2-[N-(6,6-dimethyl-2-hepten-4-ynyl)-N-methylamino]-3'-methoxyacetophenone (Compound PR-1388);
Trans-N-(6,6-dimethyl-2-hepten-4-ynyl)-N-methyl-[2-(3-methoxyphenyl)-2-propenyl]amine (Compound PR-1389);
Trans-2-[N-(6,6-dimethyl-2-hepten-4-ynyl)-N-methylamino]-2'-nitroacetophenone (Compound PR-1639);
Trans-N-(6,6-dimethyl-2-hepten-4-ynyl)-N-methyl-[2-(2-nitrophenyl)-2-propenyl]amine (Compound PR-1640);
Trans-2-[N-(6,6-dimethyl-2-hepten-4-ynyl)-N-methylamino]-3'-nitroacetophenone (Compound PR-1646);
Trans-N-(6,6-dimethyl-2-hepten-4-ynyl)-N-methyl-[2-(3-nitrophenyl)-2-propenyl]amine (Compound PR-1647);
Trans-2-[N-(6,6-dimethyl-2-hepten-4-ynyl)-N-methylamino]-4'-nitroacetophenone (Compound PR-1393);
Trans-N-(6,6-dimethyl-2-hepten-4-ynyl)-N-methyl-[2-(4-nitrophenyl)-2-propenyl]amine (Compound PR-1394);
Trans-3'-cyano-2-[N-(6,6-dimethyl-2-hepten-4-ynyl)-N-methylamino]acetophenone (Compound PR-1552);
Trans-3-{1-[N-(6,6-dimethyl-2-hepten-4-ynyl)-N-me thylamino]methyl}vinylbenzonitrile (Compound PR-1553);
Trans-4'-cyano-2-[N-(6,6-dimethyl-2-hepten-4-ynyl)-N-methylamino]acetophenone (Compound PR-1559);
Trans-4-{1-[N-(6,6-dimethyl-2-hepten-4-ynyl)-N-me thylamino]methyl}vinylbenzonitrile (Compound PR-1560);
Ethyl trans-4-{2-[N-(6,6-dimethyl-2-hepten-4-ynyl)-N-methylamino]acetyl}benzoate (Compound PR-1685);
Ethyl trans-4-{1-[N-(6,6-dimethyl-2-hepten-4-ynyl)-N-methylamino]methyl}vinylbenzoate (Compound PR-1686);
Trans-2',4'-dichloro-2-[N-(6,6-dimethyl-2-hepten-4-ynyl)-N-methylamino]acetophenone (Compound PR-1517);
Trans-N-(6,6-dimethyl-2-hepten-4-ynyl)-N-methyl-[2-(2,4-dichlorophenyl)-2-propenyl]amine (Compound PR-1518);
Trans-3',4'-dichloro-2-[N-(6,6-dimethyl-2-hepten-4-ynyl)-N-methylamino]acetophenone (Compound PR-1510);
Trans-N-(6,6-dimethyl-2-hepten-4-ynyl)-N-methyl-[2-(3,4-dichlorophenyl)-2-propenyl]amine (Compound PR-1511);
Trans-2',4'-dimethyl-2-[N-(6,6-dimethyl-2-hepten-4-ynyl)-N-methylamino]acetophenone (Compound PR-1710);
Trans-N-(6,6-dimethyl-2-hepten-4-ynyl)-N-methyl-[2-(2,4-dimethylphenyl)-2-propenyl]amine (Compound PR-1711);
Trans-3',4'-dimethyl-2-[N-(6,6-dimethyl-2-hepten-4-ynyl)-N-methylamino]acetophenone (Compound PR-1703);
Trans-N-(6,6-dimethyl-2-hepten-4-ynyl)-N-methyl-[2-(3,4-dimethylphenyl)-2-propenyl]amine (Compound PR-1704);
Trans-3',4'-difluoro-2-[N-(6,6-dimethyl-2-hepten-4-ynyl)-N-methylamino]acetophenone (Compound PR-2171);
Trans-N-(6,6-dimethyl-2-hepten-4-ynyl)-N-methyl-[2-(3,4-difluorophenyl)-2-propenyl]amine (Compound PR-2172);
Trans-3',5'-difluoro-2-[N-(6,6-dimethyl-2-hepten-4-ynyl)-N-methylamino]acetophenone (Compound PR-2157);
Trans-N-(6,6-dimethyl-2-hepten-4-ynyl)-N-methyl-[2-(3,5-difluorophenyl)-2-propenyl]amine (Compound PR-2158);
Trans-4'-tert-butyl-2-[N-(6,6-dimethyl-2-hepten-4-ynyl)-N-methylamino]acetophenone (Compound PR-1717);
Trans-N-(6,6-dimethyl-2-hepten-4-ynyl)-N-methyl-[2-(4-tert-butylphenyl)-2-propenyl]amine (Compound PR-1718);
Trans-2-[N-(6,6-dimethyl-2-hepten-4-ynyl)-N-methylamino]-2'-hydroxyacetophenone (Compound PR-1619);
Trans-4'-tert-butyldimethylsilyloxy-2-[N-(6,6-dimethyl-2-hepten-4-ynyl)-N-methylamino]acetophenone (Compound PR-1604);
Trans-N-(6,6-dimethyl-2-hepten-4-ynyl)-N-methyl-[2-(4-tert-butyldimethylsilyloxyphenyl)-2-propenyl]amine (Compound PR-1605);
Trans-N-(6,6-dimethyl-2-hepten-4-ynyl)-N-methyl-[2-(4-hydroxyphenyl)-2-propenyl]amine (Compound PR-1606);
Trans-N-(6,6-dimethyl-2-hepten-4-ynyl)-N-methyl-[2-(2-aminophenyl)-2-propenyl]amine (Compound PR-1672);
Trans-N-(6,6-dimethyl-2-hepten-4-ynyl)-N-methyl-[2-(3-aminophenyl)-2-propenyl]amine (Compound PR-1676);
N-Cinnamyl-N-methyl-2-phenyl-2-propenylamine (Compound PR-1806);
Trans-N-(6,6-dimethyl-2-hepten-4-ynyl)-N-ethyl-2-phenyl-2-propenylamine (Compound PR-1853);
Trans-N-(6,6-dimethyl-2-hepten-4-ynyl)-N-isopropyl-2-phenyl-2-propenylamine (Compound PR-1855); and
Trans-N-cyclopropyl-N-(6,6-dimethyl-2-hepten-4-ynyl)-2-phenyl-2-propenylamine (Compound PR-1930).

Of these, further preferred examples include the following:
Trans-N-(6,6-dimethyl-2-hepten-4-ynyl)-N-methyl-2-phenyl-2-propenylamine (Compound PR-1257),
Trans-N-(6,6-dimethyl-2-hepten-4-ynyl)-N-methyl-[2-(o-tolyl)-2-propenyl]amine ("Compound PR-1532"),
Trans-N-(6,6-dimethyl-2-hepten-4-ynyl)-N-methyl-[2-(m-tolyl)-2-propenyl]amine (Compound PR-1539),
Trans-N-(6,6-dimethyl-2-hepten-4-ynyl)-N-methyl-[2-(2-fluorophenyl)-2-propenyl]amine (Compound PR-1490),
Trans-N-(6,6-dimethyl-2-hepten-4-ynyl)-N-methyl-[2-(4-fluorophenyl)-2-propenyl]amine (Compound PR-1429),
Trans-N-(6,6-dimethyl-2-hepten-4-ynyl)-N-methyl-[2-(2-bromophenyl)-2-propenyl]amine (Compound PR-1504),
Trans-N-(6,6-dimethyl-2-hepten-4-ynyl)-N-methyl-[2-(3-bromophenyl)-2-propenyl]amine (Compound PR-1483),
Trans-N-(6,6-dimethyl-2-hepten-4-ynyl)-N-methyl-[2-(4-bromophenyl)-2-propenyl]amine (Compound PR-1438),
Trans-N-(6,6-dimethyl-2-hepten-4-ynyl)-N-methyl-[2-(4-chlorophenyl)-2-propenyl]amine (Compound PR-1417),
Trans-N-(6,6-dimethyl-2-hepten-4-ynyl)-N-methyl-[2-(2-nitrophenyl)-2-propenyl]amine (Compound PR-1640),
Trans-3-{1-[N-(6,6-dimethyl-2-hepten-4-ynyl)-N-me thylamino]methyl}vinylbenzonitrile (Compound PR-1553),
Trans-N-(6,6-dimethyl-2-hepten-4-ynyl)-N-methyl-[2-(2,4-dimethylphenyl)-2-propenyl]amine (Compound PR-1711),
Trans-N-(6,6-dimethyl-2-hepten-4-ynyl)-N-methyl-[2-(3,4-dimethylphenyl)-2-propenyl]amine (Compound PR-1704),
Trans-N-(6,6-dimethyl-2-hepten-4-ynyl)-N-methyl-[2-(3,4-difluorophenyl)-2-propenyl]amine (Compound PR-2172),
Trans-N-(6,6-dimethyl-2-hepten-4-ynyl)-N-methyl-[2-(3,5-difluorophenyl)-2-propenyl]amine (Compound PR-2158),
Trans-2-[N-(6,6-dimethyl-2-hepten-4-ynyl)-N-methylamino]-2'-hydroxyacetophenone (Compound PR-1619),
Trans-N-(6,6-dimethyl-2-hepten-4-ynyl)-N-methyl-[2-(4-hydroxyphenyl)-2-propenyl]amine (Compound PR-1606), and
Trans-N-(6,6-dimethyl-2-hepten-4-ynyl)-N-methyl-[2-(2-aminophenyl)-2-propenyl]amine (Compound PR-1672).

### (iii) Salts of Amine Derivatives represented by

### General Formula (1)

In the present invention, the salts of the amine derivatives represented by the general formula (1) above are not limited particularly so far as they are physiologically acceptable. Preferably they include, for example, mineral acid salts such as hydrochloride, sulfate, nitrate, and phosphate, organic acid salts such as citrates, oxalates, fumarates, maleates, formates, acetates, methanesulfonates, benzenesulfonates, and paratoluenesulfonates, and carbonates. Among these, hydrochloride is preferred. These salts can be obtained by a conventional method by using the amine derivative (1) and an acid. For example, they can be obtained by mixing the amine derivative (1) and an acid in a polar or non-polar solvent.

The amine derivatives represented by the general formula (1) of the present invention can be produced, for example, by the method for producing an amine derivatives according to the present invention shown below.

### (2) Method for Producing Amine Derivatives of the Present Invention

The method for producing amine derivatives of the present invention is characterized by following one of the reaction schemes (Method I) or (Method II) set forth below to produce the amine derivatives represented by the general formula (1) above. 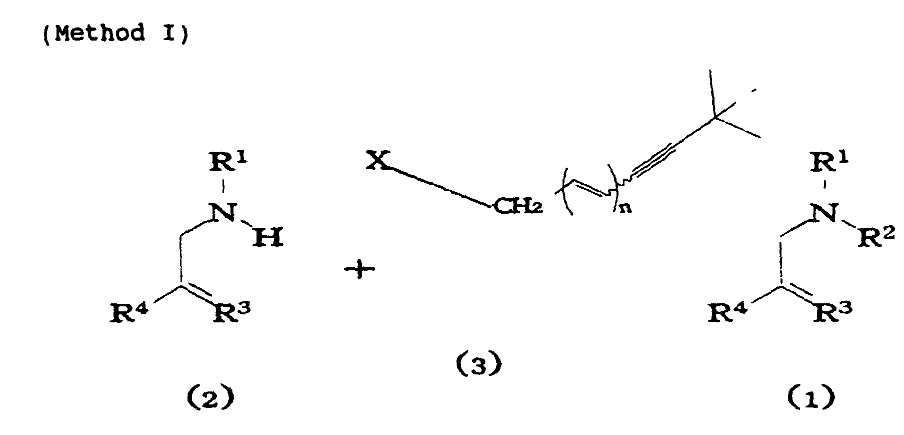 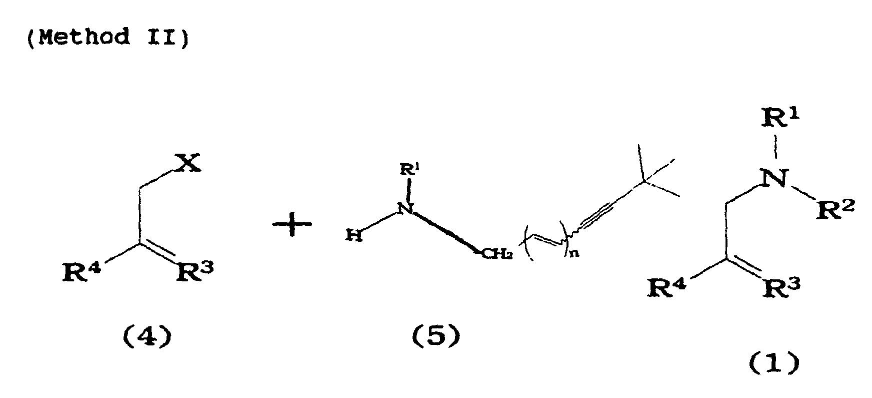 [in the formula, R¹, n, R³, and R⁴ represent the same meanings as explained above concerning the amine derivatives of the present invention represented by the general formula (1) and X represents a halogen atom.]

More specifically, the compounds (1) of the invention can be produced by condensing a secondary amine derivative represented by general formula (2) or salts thereof with a halogenated compound represented by general formula (3) (Method I) or condensing a halogenated compound represented by general formula (4) with a secondary amine derivative represented by general formula (5) or salts thereof (Method II). The condensing reactions can be carried out, for example, by using a condensing agent in the presence of a solvent.

The secondary amine derivatives represented by the general formulae (2), (3), (4), and (5), salts thereof and halogenated compounds can be easily obtained by one skilled in the art by production, purchase, or the like or can be obtained by conversion of substituent group(s) by general techniques.

In (Method I) or (Method II), the ratio of the secondary amine derivative and halogenated compound used as raw materials, is usually 0.1 to 10.0 moles, particularly preferably 1.0 to 2.5 moles of the secondary amine derivative per mole of the halogenated compound. As the condensing agent for the reaction, tertiary organic amines and inorganic bases can be used. Specific examples thereof include triethylamine, N,N-diisopropylethylamine, anhydrous potassium carbonate, anhydrous sodium carbonate and so forth. The condensing agents are used in amounts of usually 0.1 to 30.0 moles, preferably 2.0 to 5.0 moles, for the total mole of the secondary amine and halogenated compound. The reaction can be proceeded in the absence of condensing agents.

The solvent to be used in the reaction is not limited particularly so far as it is non-aqueous solvent which can dissolve the two raw materials and specifically includes N,N-dimethylformamide (hereinafter, sometimes referred to as "DMF") and so forth. The amount of solvent to be used is preferably 5 to 100 times of the total weight of the secondary amine and the halogenated compound. The solvents may be used solely or more than two of them can be used in combination. The solvent may be selected so as to be suited to the physical properties of raw materials and condensing agent used in the reaction.

The reaction temperature may be any temperature of from room temperature to a temperature near the boiling point of the solvent used. Room temperature is preferred. The reaction time may vary depending on various conditions but usually it takes 10 minutes to 30 days. Post-treatment of the reaction and purification method may be carried out by general methods, for example, appropriate combinations of quenching with water, solvent extraction, column chromatography, recrystallization and so forth.

Before or after (Method I) and (Method II), conversion of substituent groups may be carried out, if desired, to obtain other compounds represented by the general formula (1). Specific examples of the conversion of substituent groups include the following:
Halogenation using N-bromosuccinimide, bromine, copper dibromide and so forth;
Conversion of a primary amino group into a secondary amino group using a halogenated alkyl;
Conversion into an acid halide compound by reacting a carboxyl group with a halogenating agent such as thionyl chloride and subsequent reaction with an amine derivative to form an amide bond;
Conversion into an amine derivative by reacting an amide bond with a reducing agent such as lithium aluminum hydride;
Conversion of a nitro group into an amino group using a reducing agent such as zinc metal and so forth;
conversion of a carbonyl group into a carbon-carbon double bond using Wittig reaction;
Silylation using tert-butyldimethylchlorosilane and so forth;
Desilylation using n-butylammonium fluoride and so forth;
Introduction of a formyl group using hexamethylenetetramine and so forth;
Conversion into a secondary amine derivative by reacting a formyl group with a primary amine derivative and subsequent reduction;
Conversion of an alkoxycarbonyl group into carboxyl group by hydrolysis reaction; and the like. One skilled in the art can carry out the conversion of substituent groups by using general techniques.

The amine derivatives represented by the general formula (1) above and salts thereof according to the present invention are excellent in antimycotic effects and are highly useful as an antimycotic agent consisting of the same, an antimycotic composition comprising the same, and a pharmaceutical composition containing the same as an active ingredient.

Hereinafter, explanation will be made of an antimycotic agent consisting of the amine derivative or salts thereof of the present invention and an antimycotic composition and a pharmaceutical composition comprising the amine derivative or salts thereof of the present invention.

### (3) Antimycotic Agent, Antimycotic Composition, and Pharmaceutical composition of the Present Invention.

The antimycotic agent of the present invention consists of the amine derivative represented by the general formula (1) above or salts thereof according to the present invention. The antimycotic agent of the present invention, like conventionally known antimycotic agents, can be used, for example, by compounding a generally used composition which an antimycotic agent is to be added as a compounding ingredient with the antimycotic agent of the present invention, instead of a conventionally known antimycotic agent, with appropriately selecting it compounding amount.

The antimycotic composition and pharmaceutical composition of the present invention (hereinafter, both are sometimes referred to as "the composition of the present invention") can be produced by compounding one or more of the compounds represented by the general formula (1) or salts thereof. The composition of the present invention can be formulated as any type of composition without a particular limit so far as it is known to contain an antimycotic agent, and includes for example, pharmaceutical composition such as external formulations for skin and external formulations for cleaning/sterilization, cloths such as stockings and underwear, plastic products such as a toothbrush and a ball-point pens. Pharmaceutical compositions, in particular, external formulations for skin are most preferred. The compound represented by the general formula (1) or salts thereof can be introduced into the composition of the present invention by a conventional technique. For example, in case of pharmaceutical compositions, the compound (1) or salts thereof together with other ingredients may be emulsified, solubilized or mixed with powder ingredient. For cloths, the compound (1) or salts thereof may be melt-mixed with other ingredients and spun in the step of producing fiber or dipping to cloths. For plastic products, the compound (1) or salts thereof may be melt-mixed. Further, the compound (1) or salts thereof can be dipped to wood for preventing molds.

The composition of the present invention may optionally contain any ingredients that may be usually contained in compositions of these types in addition to the compound represented by the general formula (1) or salts thereof as required. Though such optional ingredients are not limited particularly, in the present invention generally include, for example, when the composition is pharmaceutical formulations, excipients, coloring agents, taste/odor correcting agent, binders, disintegrating agents, coating agents, stabilizers, pH adjusting agents, sweetening agents, and emulsifying/dispersing/solubilizing agents. Particularly, for external formulations for skin, there can be cited, for example, hydrocarbons such as liquid paraffin and vaseline, esters such as spermaceti and beeswax, triglycerides such as olive oil and beef tallow, higher alcohols such as cetanol and oleyl alcohol, fatty acids such as stearic acid and oleic acid, polyhydric alcohols such as propylene glycol and glycerol, nonionic surfactants, anionic surfactants, cationic surfactants, thickening agents, etc. For cloths or plastics, there can be cited, for example, plasticizers, crosslinking agents, coloring agents, antioxidants, ultraviolet absorbents, etc. The amount of the compound represented by the general formula (1) or salts thereof to be compounded in the inventive composition is not limited particularly but the amount is preferred from 0.001 to 20% by weight, more preferred 0.01 to 15% by weight, practically preferred 0.1 to 10% by weight.

### EXAMPLES

Hereinafter, the present invention will be described in more detail by examples. However, the present invention should not be limited thereto.

### <Preparation Examples for Raw Material>

The novel compounds of the present invention can be produced from various halogen compounds and secondary amines as raw materials. Hereinafter, preparation examples for the various halogen compounds and secondary amines which can be used as raw materials of the novel compounds of the invention will be described.

### Preparation Example 1: Preparation of 2-Bromo-4'-fluoroacetophenone

In 25 ml of diethyl ether was dissolved 5.00 g (36.2 mmol) of p-fluoroacetophenone and while stirring the solution on an ice bath, 5.8 g (36.2 mmol) of bromine was dropped. After dropping, the mixture was stirred for 5 minutes and then the solvent was distilled off under reduced pressure. The residue thus obtained was purified by silica gel column chromatography (chloroform 100%). The results of NMR measurement revealed that it contained the target compound, and in addition, the raw materials and a dibromo form as a side product. Yield after adjustment was 6.21 g and % yield was 79.0%.
¹H-NMR (CDCl₃, ppm)
4.42 (s, 2H) , 7.12~8.18 (m, 4H)

### Preparation Example 2: Preparation of 2-bromo-2'-fluoro-acetophenone

2'-fluoroacetophenone was used as a raw material and the target compound was obtained in the same manner as in Preparation Example 1.
¹H-NMR (CDCl₃, ppm)
4.54 (dd, J=2.34Hz, 1.08Hz, 2H), 7.11~7.29 (m, 2H), 7.59 (m, 1H), 7.96 (m, 1H)

### Preparation Example 3: Preparation of 2.2'-dibromoacetophenone

2'-bromoacetophenone was used as a raw material and the target compound was obtained in the same manner as in Preparation Example 1.
¹H-NMR (CDCl₃, ppm)
4.95 (s, 2H), 7. 32~7.66 (m, 4H)

### Preparation Example 4: Preparation of 2.3'-dibromoacetophenone

3'-bromoacetophenone was used as a raw material and the target compound was obtained in the same manner as in Preparation Example 1.
¹H-NMR (CDCl₃, ppm)
4.42 (s, 2H), 7.39 (t, J=7.83Hz, 1H), 7.72~7.76 (m, 1H), 7.92 (m, 1H), 8.13 ( t, J=1.89Hz, 1H)

### Preparation Example 5: Preparation of 2-bromo-3'-methylacetophenone

3'-methylacetophenone was used as a raw material and the target compound was obtained in the same manner as in Preparation Example 1.
¹H-NMR (CDCl₃, ppm)
2.43 (s, 3H), 4.45 (s, 2H) , 7.35~7.44 (m, 3H), 7.80 (s, 1H)

### Preparation Example 6: Preparation of 2-bromo-2'-methylacetophenone

2'-methylacetophenone was used as a raw material, synthesis was carried out in the same manner as in Preparation Example 1 and the product was used as it was in subsequent reaction without isolation and purification.

### Preparation Example 7: Preparation of 2-bromo-2'-methoxyacetophenone

2'-methoxyacetophenone was used as a raw material and the target compound was obtained in the same manner as in Preparation Example 1.
¹H-NMR (CDCl₃, ppm)
3.95 (s, 3H), 4.61 (s, 2H), 6.96~7.11 (m, 2H), 7.52 (m, 1H), 7.85 (m, 1H)

### Preparation Example 8: Preparation of 2-bromo-4'-nitroacetophenone

4'-nitroacetophenone was used as a raw material and the target compound was obtained in the same manner as in Preparation Example 1.
¹H-NMR (CDCl₃, ppm)
4.45 (s, 2H), 8.16 (d, J=8.91Hz, 2H), 8.35 (d, J=8.91Hz, 2H)

### Preparation Example 9: Preparation of 2-bromo-3'-nitroacetophenone

m-nitroacetophenone was used as a raw material and the target compound was obtained in the same manner as in Preparation Example 1.
¹H-NMR (CDCl₃, ppm)
4.50 (s, 2H), 7.74 (t, J=8.10Hz, 1H), 8.33 (d, J=8.10Hz, 1H), 8.48 (d, J=8. 10Hz, 1H), 8.82 (s, 1H)

### Preparation Example 10: Preparation of 2-bromo-3', 4'-dimethylacetophenone

3', 4'-dimethylacetophenone was used as a raw material and the target compound was obtained in the same manner as in Preparation Example 1.
¹H-NMR (CDCl₃, ppm)
2.33 (s, 6H), 4.43 (s, 2H), 7.24 (d, J=9.45Hz, 1H), 7.71 (dd, J=7.83Hz, 1.89Hz, 1H), 7.76 (s, 1H)

### Preparation Example 11: Preparation of 2-bromo-2', 4'-dimethylacetophenone

2', 4'-dimethylacetophenone was used as a raw material and the target compound was obtained in the same manner as in Preparation Example 1.
¹H-NMR (CDCl₃, ppm)
2.37 (s, 3H), 2.47 (s, 3H), 4.41 (s, 2H), 7.06~7.14 (m, 2H), 7.62 (d, J=8.64Hz, 1H)

### Preparation Example 12: Preparation of 2-bromo-4'-tert-butylacetophenone

4'-tert-butylacetophenone was used as a raw material and the target compound was obtained in the same manner as in Preparation Example 1.
¹H-NMR (CDCl₃, ppm)
1.23 (s, 9H) , 4.44 (s, 2H) , 7.51 (d, J=8.64Hz, 2H), 7.93 (d, J=8.64Hz, 2H)

### Preparation Example 13: Preparation of 2-bromo-3'-cyanoacetophenone

m-cyanoacetophenone was used as a raw material and the target compound was obtained in the same manner as in Preparation Example 1.
¹H-NMR (CDCl₃, ppm)
4.42 (s, 2H), 7.66 (t, J=8.10Hz, 1H, 7.90 (d, J=8.10Hz, 1H), 8.22 (t, J=8.10Hz, 1H), 8.28 (s, 1H)

### Preparation Example 14: Preparation of 4'-tert-butyldimethylsilyloxyacetopheone

N,N-dimethylformamide (70 ml) was mixed with 5.00 g (36.7 mmol) of 4'-hydroxyacetophenone, 6.64 g (44.1 mmol) of tert-butyldimethylchlorosilane, and 6.00 (98.1 mmol) of imidazole and the mixture was stirred for 12 hours at room temperature. The reaction mixture was poured into ice water to stop the reaction and extracted with 100 ml of ethyl acetate. The organic extract was washed with 100 ml of water ten times and then with saturated saline and dried with anhydrous sodium sulfate. The solvent was distilled off under reduced pressure and the resulting residue was purified by silica gel column chromatography (chloroform 100%) to obtain 5.23 g (yield: 56.9%) of the target compound.
¹H-NMR (CDCl₃, ppm)
0.23 (s, 3H×2), 0.98 (s, 9H), 2.55 (s, 3H), 6.87 (dd, J=7.02Hz, 1.89Hz, 2H), 7.88 (dd, J=7.02Hz, 1.89Hz, 2H)

### Preparation Example 15: Preparation of 2-bromo-4'-tert-butyldimethylsilyloxyacetophenone

Using 4'-tert-butyldimethylsilyloxyacetophenone obtained in Preparation Example 14 above as a raw material, the target compound was obtained in the same manner as in Preparation Example 1.
¹H-NMR (CDCl₃, ppm)
0.25 (s, 3H × 2), 0.99 (s, 9H), 4.40 (s, 2H), 6.90 (d, J=8.91Hz, 2H), 7.93 (d, J=8.91Hz, 2H)

### Preparation Example 16: Preparation of 2-bromo-2'-hydroxyacetophenone

Ethyl acetate (40 ml) was mixed with 13.8 g (161.8 mmol) of copper dibromide, and a solution of 5.00 g (37.0 mmol) of o-hydroxyacetophenone in 40 ml of chloroform was dropped while refluxing with heating, the mixture being refluxed for additional 4 hours. White crystals precipitated were filtered and the filtrate was washed with water and with saturated saline and dried with anhydrous sodium sulfate. Then the solvent was distilled off under reduced pressure. The raw material, o-hydroxyacetophenone, was removed from the resulting residue by distillation under reduced pressure to obtain 4.12 g (yield: 51.8%) of the target compound as a pale yellow oily substance.
¹H-NMR (CDCl₃, ppm)
4.45 (s, 2H), 6.91~7.01 (m, 2H), 7.53 (m, 1H), 7.74 (m, 1H), 11.7 (s, 1H)

### Preparation Example 17: Preparation of 2-bromo-3', 5'-difluoroacetophenone

3', 5'-difluoroacetophenone was used as a raw material and the target compound was obtained in the same manner as in Preparation Example 1.
¹H-NMR (CDCl₃, ppm)
4.38 (s, 2H), 7.07 (m, 1H), 7.47~7.54 (m, 2H)

### Preparation Example 18: Preparation of 2-bromo-3', 4' difluoroacetophenone

3', 4'-difluoroacetophenone was used as a raw material and the target compound was obtained in the same manner as in Preparation Example 1.
¹H-NMR (CDCl₃, ppm)
4.38 (s, 2H), 7.11 (m, 1H), 7.65~8.02 (m, 2H)

### Preparation Example 19: Preparation of α -bromomethyl-styrene

Benzene (120 ml) was mixed with 9.00 g (76.2 mmol) of α-methylstyrene, 14.2 g (80.0 mmol) of N-bromosuccinimide, and 300 mg of benzoyl peroxide and the mixture was refluxed for 24 hours. After the solvent was distilled off under reduced pressure, carbon tetrachloride was added and the resulting crystals were filtered. The filtrate was concentrated under reduced pressure and the resulting residue was purified by silica gel column chromatography (n-hexane 100%) to obtain the target compound. The yield, % yield and NMR spectrum were as follows.
Yield: 7.72 g, % yield: 51.5%.
¹H-NMR (CDCl₃, ppm)
4.39 (s, 2H), 5.49 (s, 1H), 5.56 (s, 1H), 7.25~7.55 (m, 5H)

### Preparation Example 20: Preparation of N-ethyl-2-phenyl-2-propenylamine

12.42 g (152 mmol) of ethylamine hydrochloride is dissolved in 50ml of methanol, and 15.42 g (152 mmol) of triethylamine was dropped into the solution under ice cooling while stirring. Further, 5 ml of the methanol solution containing 3.0 g (15.2 mmol) of α -bromomethylstyrene obtained in Preparation Example 19 was dropped. Then, after heating to room temperature, the mixture was stirred for 91 hours. After concentration, pH of the reaction mixture was changed to alkaline with sodium hydrogen carbonate and extracted with 150 ml and 100 ml of chloroform. After drying with magnesium sulfate, the solvent was distilled off under reduced pressure and the resulting residue was purified by silica gel column chromatography (chloroform : methanol = 100:1 → 100:2) to obtain 890 mg (yield: 36.3%) of the target compound.
¹H-NMR (CDCl₃, ppm)
1.10 (t, J=7.29Hz, 3H), 2.68 (q, J=7.29Hz, 2H), 3.67 (s, 2H), 5.23 (s, 1H), 5.39 (s, 1H), 7.22~7.48 (m, 5H)

### Preparation Example 21: Preparation of 2-bromo-4'-methylacetophenone

4'-methylacetophenone was used as a raw material and the target compound was obtained in the same manner as in Preparation Example 1.
¹H-NMR (CDCl₃, ppm)
2.43 (s, 3H), 4.43 (s, 2H), 7.29 (d, J=8.10Hz, 2H), 7.88 (d, J=8. 10Hz, 2H)

### Preparation Example 22: Preparation of 2-bromo-3'-fluoro-acetophenone

3'-fluoroacetophenone was used as a raw material and the target compound was obtained in the same manner as in Preparation Example 1.
¹H-NMR (CDCl₃, ppm)
4.43 (s, 2H), 7.32 (m, 1H), 7.49 (m, 1H), 7.68 (m, 1H), 7.77 (m, 1H)

### Preparation Example 23: Preparation of 2-bromo-2'-chloroacetophenone

2'-chloroacetophenone was used as a raw material and the target compound was obtained in the same manner as in Preparation Example 1.
¹H-NMR (CDCl₃, ppm)
4.53 (s, 2H), 7.30~7.52 (m, 3H), 7.57 (d, J=7.56Hz, 1H)

### Preparation Example 24: Preparation of 2-bromo-4'-cyanoacetophenone

4'-cyanoacetophenone was used as a raw material and the target compound was obtained in the same manner as in Preparation Example 1.
¹H-NMR (CDCl₃, ppm)
4.43 (s, 2H), 7.83 (d, J=7. 02Hz, 1H), 8.09 (d, J=7. 02Hz, 2H)

### Preparation Example 25: Preparation of ethyl 4-(2-bromoacetyl)benzoate

Ethyl 4-acetylbenzoate was used as a raw material and the target compound was obtained in the same manner as in Preparation Example 1.
¹H-NMR (CDCl₃, ppm)
1.42 (t, J=7. 29Hz, 3H), 4.42 (q, J=7.29Hz, 2H), 4.47 (s, 2H), 8.04 (d, J=8.91 Hz, 2H), 8.16 (d, J=8.91Hz, 2H)

### Preparation Example 26: Preparation of 2-bromo-2' 4'-dichloroacetophenone

2', 4'-dichloroacetophenone was used as a raw material and the target compound was obtained in the same manner as in Preparation Example 1.
¹H-NMR (CDCl₃, ppm)
4.50 (s, 2H), 7.37 (dd, J=7.02Hz, 1.89Hz, 1H), 7.48 (d, J=1.89Hz, 1H), 7.57 (d, J=7.02Hz, 1H)

### Preparation Example 27: Preparation of 2-bromo-3', 4'-dichloroacetophenone

3', 4'-dichloroacetophenone was used as a raw material and the target compound was obtained in the same manner as in Preparation Example 1.
¹H-NMR (CDCl₃, ppm)
4.38 (s, 2H), 7.59 (d, J=8.37Hz, 1H), 7.82 (dd, J=8.37Hz, 1.89Hz, 1H), 8.07 (d, J=1.89Hz, 1H)

### Preparation Example 28: Preparation of N-isopropyl-2-phenyl-2-propenylamine

9 g of isopropylamine was dissolved in 20 ml of methanol under stirring with ice cooling, and 5 ml of the methanol containing 3.0 g (15.2 mmol) of α -bromomethylstyrene obtained in Preparation Example 19 was dropped into the solution. After heating to room temperature and stirring for 18 hours, the solution was washed by concentration under reduced pressure. 150 ml of chloroform was added to the residue and the solution was washed with saturated sodium hydrogen carbonate. After drying the solution with magnesium sulfate, the solvent was distilled off. Purification was performed by silica gel column chromatography (chloroform ~ chloroform : methanol = 50 : 1).
Yield: 2.14 g, % yield: 80.3%.
¹H-NMR (CDCl₃, ppm)
1.06 (d, J=6.48Hz, 6H), 2.85 (7 doublet, J=6.48Hz, 1H), 3.66 (s, 2H), 5.24 (s, 1H), 5.39 (s, 1H), 7.25~7.50 (m, 5H)

### Preparation Example 29: Preparation of N-cyclopropyl-2-phenyl-2-propenylamine

Using cyclopropylamine instead of isopropylamine as a raw material, the target compound was obtained in the same manner as in Preparation Example 28.
¹H-NMR (CDCl₃, ppm)
0.30~0.50 (m, 4H), 2.15 (m, 1H), 3.73 (s, 2H), 5.23 (s, 1H), 5.39 (s, 1H), 7.23~7.52 (m, 5H)

### Preparation Example 30: Preparation of N-(6, 6-dimethyl-2-hepten-4-ynyl)methylamine (Compound PR-1133)

First, 1-bromo-6,6-dimethyl-2-hepten-4-yne was synthesized by the method described in J. Med. Chem. 1984, Vol. 27, page 1539 and used for the synthesis of the target compound below.

2.02 g of triethylamine was added to 40 ml of a 40% methylamine-methanol solution under ice cooling and while stirring the mixture, 3.5 ml of the methanol solution containing 4.02 g of 1-bromo-6,6-dimethyl-2-hepten-4-yne obtained as above was dropped. After dropping, the ice bath was removed and the reaction mixture was stirred at room temperature for 88 hours. Then, under reduced pressure, the residue obtained by distilling off methanol from the reaction mixture was dissolved in an aqueous 1N hydrochloric acid solution, then was washed with ether. pH of the water extract after washing was changed to alkaline with an aqueous 2N sodium hydroxide solution and extracted with 100 ml of chloroform twice. The organic extract thus obtained was dried with magnesium sulfate and then the solvent was distilled off under reduced pressure. The residue after the distilling off the solvent was purified by silica gel column chromatography (chloroform ~ chloroform : methanol = 100 : 1 ~ 10 : 1) to obtain the target compound as an oily substance.

The yield, % yield and results of measurement on NMR are shown below. Analysis of NMR spectrum revealed that N-(6,6-dimethyl-2-hepten-4-ynyl)methylamine obtained in this Preparation Example was a mixture of a *trans*-form and a *cis*-form (*trans* : *cis* = 3 : 1).
Yield: 2.02 g, % yeild: 66.9%
¹H-NMR (CDCl₃, ppm), *trans* form corresponding peak
1.24 (s, 9H), 2.41 (s, 3H), 3.23 (dd, J=6.48Hz, 1. 35Hz, 2H), 5.62 (dt, J=15.93Hz, 1.35Hz, 1H), 6.07 (dt, J=15.93Hz, 6.48Hz, 1H), 1.53 (broad s , estimated N-H+H₂O) 1H-NMR (CDCl₃, ppm), *cis* form corresponding peak
1.26 (s, 9H), 2.44 (s, 3H), 3.43 (dd, J=6.75Hz, 1.35Hz, 2H), 5.57 (dt, J=10.53Hz, 1.35Hz, 1H), 5.89 (dt, J=10.53Hz, 6.75Hz, 1H), 1.53 (broad s , estimated N-H+H₂O)

### Preparation Example 31: Preparation of N-(4-tert-butylbenzyl)methylamine

Chloroform (100 ml) was mixed with 10.1 g (56.6 mmol) of p-tert-butylbenzoic acid and 20.2 g of thionyl chloride, and the mixture was refluxed for 5 hours. The solvent and excess thionyl chloride were distilled off under reduced pressure and the residue was dissolved in a small amount of methanol. The solution was dropped to 17 ml of a 40% methylamine-methanol solution cooled on an ice bath. After dropping, the solution was taken out of the ice bath and stirred at room temperature for 48 hours. Then, 100 ml of 2N hydrochloric acid was added to the reaction mixture, which was extracted with dichloromethane, and after washing with water and saturated saline, respectively, the organic layer was dried with magnesium sulfate. After distilling off the solvent under reduced pressure, the resulting white crystals were dissolved in dichloromethane and the solution was washed with 1 liter of a saturated aqueous sodium hydrogen carbonate solution to remove p-tert-butylbenzoic acid, raw material. After the organic extract was dried with magnesium sulfate, the solvent was distilled off under reduced pressure to obtain 8.15 g (yield: 74.9%) of N-methyl-4-tert-butylbenzamide as white crystals. This was mixed with 110 ml of diethyl ether, 8.15 g (42.6 mmol) of N-methyl-4-tert-butylbenzamide and 2.88 g (85.2 mmol) of lithium aluminum hydride, and refluxed in nitrogen atmosphere for 6 hours. After the reflux, the reaction mixture was ice cooled and water was added thereto to decompose excess lithium aluminum hydride. Aluminum hydroxide deposited was filtered off and the filtrate was extracted with diethyl ether. After washing with water and saturated saline, respectively, the organic extract was dried with magnesium sulfate. The solvent was distilled off under reduced pressure and the resulting yellow oily substance was distilled under reduced pressure (115 to 118°C/10 mmHg) to obtain 3.69 g (yield: 48.9%) of the target compound as yellow oily substance.
¹H-NMR (CDCl₃, ppm)
1.31 (9H, s), 2.45 (3H, s), 7.24 (2H, d, J=8.37Hz), 7.35 (2H, d, J=8.37Hz)

### Preparation Example 32: Preparation of N-(4-tert-butylbenzyl)methylamine (2)

14.8 g (0.10 mol) of p-tert-butyltoluene was dissolved in carbon tetrachloride, and 17.8 g (0.10 mol) of N-bromosuccinimide and 200 mg of benzoyl peroxide are added to the solution, and the mixture was refluxed for 2 hours. After cooling, insoluble matter was filtered off. The residue was washed with carbon tetrachloride and the filtrate was concentrated under reduced pressure. The residue was dissolved in n-hexane and the solution was dried with magnesium sulfate. Thereafter, the solvent was distilled off under reduced pressure to obtain 22.7 g (yield: 100%, however, the results of ¹H-NMR revealed that the product was a mixture of the target compound : raw material : dibromo form = 10 : 1 : 1) of p-tert-butylbenzylbromide. 10.6 g (0.10 mol) of sodium carbonate was added to 200 ml of a 40% methylamine-methanol solution, and 20 ml of the methanol containing 22.7 g (0.10 mol) of p-tert-butylbenzyl bromide was dropped in an ice bath. After taking out of the ice bath, the solution was stirred at room temperature for 41 hours. The methanol was distilled off under reduced pressure and water was added to the residue, followed by extraction with 400 ml of ether. The ether part was extracted twice with 200 ml and 100 ml, respectively, of 1N hydrochloric acid and the water part was extracted with ethyl acetate. Then, the water extract was changed to alkaline with an aqueous 2N sodium hydroxide solution and extracted with 400 ml of ether. After drying the extracts with magnesium sulfate, the solvent was distilled off under reduced pressure. The residue was purified by silica gel column chromatography (chloroform: methanol= 200: 1 → 100: 1 → 20: 1) to obtain the target substance of 9.51 g (yield: 53.7%).
¹H-NMR (CDCl₃, ppm)
1.31 (9H, s), 2.45 (3H, s), 7.24 (2H, d, J=8.37Hz), 7.35 (2H, d, J=8.37Hz)

### Preparation Example 33: Preparation of N-methyl-(1-naphthylmethyl)amine

1.14 g(11.3 mmol) of triethylamine was mixted with a 40% methylamine-methanol solution and 5 ml of the methanol containing 2.00 g (11.3 mmol) of 1-chloromethylnaphthalene was dropped while stirring in an ice bath. After dropping, the reaction mixture was taken out of the ice bath and stirred at room temperature for 60 hours. The solvent was distilled off under reduced pressure and extracted with ether-2N hydrochloric acid. The organic extract was washed with saturated saline and dried with sodium sulfate. Then, the solvent was distilled off under reduced pressure to obtain 1.78 g (yield: 91.9%) of the target compound as a pale yellow oil.
¹H-NMR (CDCl₃, ppm)
2.56 (3H, s), 4.20 (2H, s), 7.39~7.57 (4H, m), 7.77 (1H, dd, J=2.02Hz, 7.43Hz), 7.86 (1H, dd, J=2.02Hz, 8.23Hz), 8.12 (1H, d, J=7.83Hz)

### Preparation Example 34: Preparation of N-methyl-(2-naphthylmethyl)amine

2-bromomethylnaphthalene was used as a raw material and the target compound was obtained in the same manner as in Preparation Example 19.
¹H-NMR (CDCl₃, ppm)
2.50 (3H, s), 3.92 (2H, s), 7.41~7.50 (3 H, m), 7.80~7.90 (4H, m)

### Preparation Example 35: Preparation of 2-bromo-2'-acetonaphthone

2'-acetonaphthone was used as a raw material and the target compound was obtained in the same manner as in Preparation Example 3.
¹H-NMR (CDCl₃, ppm)
4.59 (2H, s), 7.26~7.67 (2H, m), 7.87~8. 06 (4H, m), 8.50 (1H, m)

### Preparation Example 36: Preparation of N-(2,2,2-trifluoroethyl)-2-phenyl-2-propenylamine

α-bromomethylstyrene obtained in Preparation Example 19 above was reacted with 2,2,2-trifluoroethylamine in the same manner as in Preparation Example 20 to obtain the target compound.
¹H-NMR (CDCl₃, ppm)
3.17 (2H, q, J=9. 45Hz), 3.78 (2H, s), 5.26 (1H, s), 5.45 (1H, s), 7.22~7.45 (5H, m)

### Preparation Example 37: Preparation of 4-(1-methyl-1-phenylethyl)benzaldehyde

Trifluoroacetic acid (35 ml) was mixed with 3.93 g(20.0 mmol) of 2,2-diphenylpropane and 2.80 g (20.0 mmol) of hexamethylenetetramine and heated to reflux for 16 hours. After cooling to room temperature, the reaction mixture was poured into ice water and the mixture was stirred for 1 hour. The solution was adjusted to pH = about 9 with aqueous potassium carbonate solution and extracted with 100 ml of diethyl ether. The organic extract was washed with saturated saline and dried with sodium sulfate and the solvent was distilled off under reduced pressure. The resulting residue was purified by silica gel column chromatography (n-hexane : ethyl acetate = 10 : 1) to obtain 3.61 g (yield: 80.5%) of the target compound as a pale yellow oily substance.
¹H-NMR (CDCl₃, ppm)
1.71 (3H×2, s), 7.17~7.32 (5H, m), 7.40 (2H, d, J=8.37Hz), 7.79 (2H, d, J=8.87Hz), 9.98 (1H, s)

### Prsparation Example 38: Preparation of N-methyl-4-(1-methyl-phenylethyl) benzylamine

3.61 g (16.1 mmol) of 4-(1-methyl-1-phenylethyl)benzaldehyde obtained in Preparation Example 37 was dissolved in 40 ml of a 40% methylamine-methanol solution, and several particles of molecular sieves (4 Å) was added and the mixture was stirred at room temperature overnight. The reaction mixture was filtered, the filtrate was concentrated under reduced pressure, and the residue was extracted with 100 ml of diethyl ether. The organic extract was washed with saturated saline and dried with sodium sulfate and then the solvent was distilled off under reduced pressure. The residue was dissolved in 25 ml of methanol and 0.70 g of sodium borohydride was added thereto, followed by stirring at 50°C for 1 hour. The solvent was distilled off under reduced pressure and the residue was extracted with 100 ml of diethyl ether. The organic exract was washed with saturated saline and dried with sodium sulfate and the solvent was distilled off under reduced pressure. The residue was dissolved in 10 ml of ethanol and an excess amount of 4N hydrogen chloride-ethyl acetate solution was added thereto. The solvent was distilled off under reduced pressure, 100 ml or diisopropyl ether was added to the residue and the crystals precipitated were collected by filtration After neutralizing hydrochloric acid with an aqueous sodium hydroxide solution, the resulting crystals were extracted with ether to obtain 2.49 g (yield; 64.6%) of the target compound as an orange oily substance.
¹H-NMR (CDCl₃, ppm)
1.68 (3H×2. s), 2.46 (3H, s), 3.71 (2H, s), 7.08~7.29 (9H, m)

### <Examples of the inventive novel compounds>

Hereinafter, examples of the compounds represented by the general formula (1) of the present invention will be described. First, for each example, a list of R¹, R², R³ and R⁴ will be shown in Table 1.

In some of the examples, hydrochloride of the compounds are also described in the column of "Compound No." in Tables 1 and 2 are also described.

For the amine derivative of the invention, because all of R⁴ in the general formula (1) are groups represented by the formula (i), they were omitted in the list. In Tables 1 and 2, "-" in the column of R⁸ means that there is no substituent group and the numerals in the round brackets indicate the position of bonding. Further, (a), (b), (c), (g), (h), and (i) mean groups represented by the formulae (a), (b), (c), (g), (h), and (i), respectively. Ph represents a phenyl group.

Further, "PR-1133'" in Table 1 represents "6,6dimethyl-2-hepten-4-ynyl group", the group being represented by the following formula.

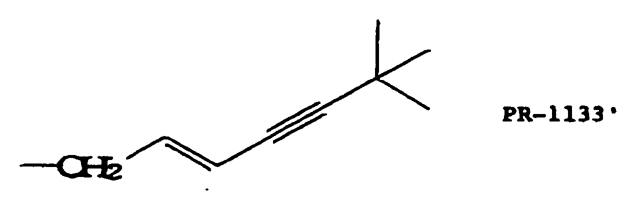

In addition, "PR-1805" represents "a cinnamyl group", which is represented by the following formula.

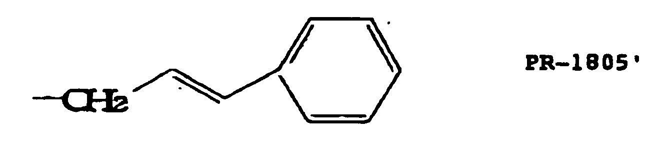

**Table 1-1**

| PR Compounds | | | | | | |
|---|---|---|---|---|---|---|
| Exp. | Comp. No | R¹ | R² | R³ | R⁸ | m |
| 1 | PR-1130 | -CH₃ | PR-1133' | =0 | - | 0 |
| 2 | PR-1257 | -CH₃ | PR-1133' | =CH₂ | - | 0 |
| 3 | PR-1531 | -CH₃ | PR-1133' | =0 | -CH₃ (2) | 1 |
| 3 | PR-1532 | -CH₃ | PR-1133' | =CH₂ | -CH₃ (2) | 1 |
| 4 | PR-1538 | -CH₃ | PR-1133' | =0 | -CH₃ (3) | 1 |
| 4 | PR-1539 | -CH₃ | PR-1133' | =CH₂ | -CH₃ (3) | 1 |
| 5 | PR-1413 | -CH₃ | PR-1133' | =0 | -CH₃ (4) | 1 |
| 5 | PR-1414 | -CH₃ | PR-1133' | =CH₂ | -CH₃ (4) | 1 |
| 6 | PR-1489 | -CH₃ | PR-1133' | =0 | -F (2) | 1 |
| 6 | PR-1490 | -CH₃ | PR-1133' | =CH₂ | -F (2) | 1 |
| 7 | PR-1468 | -CH₃ | PR-1133' | =0 | -F (3) | 1 |
| 7 | PR-1469 | -CH₃ | PR-1133' | =CH₂ | -F (3) | 1 |
| 8 | PR-1428 | -CH₃ | PR-1133' | =0 | -F (4) | 1 |
| 8 | PR-1429 | -CH₃ | PR-1133' | =CH₂ | -F (4) | 1 |
| 9 | PR-1503 | -CH₃ | PR-1133' | =0 | -Br (2) | 1 |
| 9 | PR-1504 | -CH₃ | PR-1133' | =CH₂ | -Br (2) | 1 |
| 10 | PR-1482 | -CH₃ | PR-1133' | =0 | -Br (3) | 1 |
| 10 | PR-1483 | -CH₃ | PR-1133' | =CH₂ | -Br (3) | 1 |
| 11 | PR-1437 | -CH₃ | PR-1133' | =0 | -Br (4) | 1 |
| 11 | PR-1438 | -CH₃ | PR-1133' | =CH₂ | -Br (4) | 1 |
| 12 | PR-1496 | -CH₃ | PR-1133' | =0 | -Cl (2) | 1 |
| 12 | PR-1497 | -CH₃ | PR-1133' | =CH₂ | -Cl (2) | 1 |

**Table 1-2**

| PR Compounds | | | | | | |
|---|---|---|---|---|---|---|
| Exp. | Comp. No | R¹ | R² | R³ | R⁸ | m |
| 13 | PR-1416 | -CH₃ | PR-1133' | =0 | -Cl (4) | 1 |
| 13 | PR-1417 | -CH₃ | PR-1133' | =CH₂ | -Cl (4) | 1 |
| 14 | PR-1632 | -CH₃ | PR-1133' | =0 | -OCH₃ (2) | 1 |
| 14 | PR-1633 | -CH₃ | PR-1133' | =CH₂ | -OCH₃ (2) | 1 |
| 15 | PR-1388 | -CH₃ | PR-1133' | =0 | -OCH₃ (3) | 1 |
| 15 | PR-1389 | -CH₃ | PR-1133' | =CH₂ | -OCH₃ (3) | 1 |
| 16 | PR-1639 | -CH₃ | PR-1133' | =0 | -NO₂ (2) | 1 |
| 16 | PR-1640 | -CH₃ | PR-1133' | =CH₂ | -NO₂ (2) | 1 |
| 17 | PR-1646 | -CH₃ | PR-1133' | =0 | -NO₂ (3) | 1 |
| 17 | PR-1647 | -CH₃ | PR-1133' | =CH₂ | -NO₂ (3) | 1 |
| 18 | PR-1393 | -CH₃ | PR-1133' | =0 | -NO₂ (4) | 1 |
| 18 | PR-1394 | -CH₃ | PR-1133' | =CH₂ | -NO₂ (4) | 1 |
| 19 | PR-1552 | -CH₃ | PR-1133' | =0 | -CN (3) | 1 |
| 19 | PR-1553 | -CH₃ | PR-1133' | =CH₂ | -CN (3) | 1 |
| 20 | PR-1559 | -CH₃ | PR-1133' | =0 | -CN (4) | 1 |
| 20 | PR-1560 | -CH₃ | PR-1133' | =CH₂ | -CN (4) | 1 |
| 21 | PR-1685 | -CH₃ | PR-1133' | =0 | -COOC₂H₅ (4) | 1 |
| 21 | PR-1686 | -CH₃ | PR-1133' | =CH₂ | -COOC₂H₅ (4) | 1 |
| 22 | PR-1517 | -CH₃ | PR-1133' | =0 | -Cl (2,4) | 2 |
| 22 | PR-1518 | -CH₃ | PR-1133' | =CH₂ | -Cl (2,4) | 2 |
| 23 | PR-1510 | -CH₃ | PR-1133' | =0 | -Cl (3,4) | 2 |
| 23 | PR-1511 | -CH₃ | PR-1133' | =CH₂ | -Cl (3,4) | 2 |

**Table 1-3**

| PR Compounds | | | | | | |
|---|---|---|---|---|---|---|
| Exp. | Comp. No | R¹ | R² | R³ | R⁸ | m |
| 24 | PR-1710 | -CH₃ | PR-1133' | =0 | -CH₃ (2,4) | 2 |
| 24 | PR-1711 | -CH₃ | PR-1133' | =CH₂ | -CH₃ (2,4) | 2 |
| 25 | PR-1703 | -CH₃ | PR-1133' | =0 | -CH₃ (3,4) | 2 |
| 25 | PR-1704 | -CH₃ | PR-1133' | =CH₂ | -CH₃ (3,4) | 2 |
| 26 | PR-2171 | -CH₃ | PR-1133' | =0 | -F (3,4) | 2 |
| 26 | PR-2172 | -CH₃ | PR-1133' | =CH₂ | -F (3,4) | 2 |
| 27 | PR-2157 | -CH₃ | PR-1133' | =0 | -F (3,5) | 2 |
| 27 | PR-2158 | -CH₃ | PR-1133' | =CH₂ | -F (3,5) | 2 |
| 28 | PR-1717 | -CH₃ | PR-1133' | =0 | -C(CH₃)₃ (4) | 1 |
| 28 | PR-1718 | -CH₃ | PR-1133' | =CH₂ | -C(CH₃)₃ (4) | 1 |
| 29 | PR-1619 | -CH₃ | PR-1133' | =0 | -OH (2) | 1 |
| 30 | PR-1604 | -CH₃ | PR-1133' | =0 | -OSi(CH₃)₂C(CH₃)₃ (4) | 1 |
| 30 | PR-1605 | -CH₃ | PR-1133' | =CH₂ | -OSi(CH₃)₂C(CH₃)₃ (4) | 1 |
| 30 | PR-1606 | -CH₃ | PR-1133' | =CH₂ | -OSi(CH₃)₂C(CH₃)₃ (4) | 1 |
| 31 | PR-1672 | -CH₃ | PR-1133' | =CH₂ | -NO₂ (2) | 1 |
| 32 | PR-1676 | -CH₃ | PR-1133' | =CH₂ | -NH₂ (3) | 1 |
| 33 | PR-1806 | -CH₃ | PR-1805' | =CH₂ | - | 0 |
| 34 | PR-1853 | -C₂H₅ | PR-1133' | =CH₂ | - | 0 |
| 35 | PR-1855 | -CH(CH₃)₂ | PR-1133' | =CH₂ | - | 0 |
| 36 | PR-1930 | -C₃H₅ | PR-1133' | =CH₂ | - | 0 |

### Example 1: Trans-2-[N-(6,6-dimethyl-2-hepten-4-ynyl)-N-methylamino]acetophenone; (Compound PR-1130) and its hydrochloride; (Compound PR-1254)

N,N-Dimethylformamide (DMF) (35 ml) was mixed with 3.27 g (21.6 mmol) of N-(6,6-dimethyl-2-hepten-4-ynyl)methylamine (Compound PR-1133) and 7.46g (540 mmol) of potassium carbonate and a solution of 3.98 g (20.0 mmol) of 2-bromoacetophenone in 50 ml of DMF was dropped while stirring in an ice bath. After the dropping, the mixture was stirred as it was and the temperature was slowly elevated to room temperature, and stirring was continued at room temperature for additional 12 hours.

The reaction mixture was poured in a saturated aqueous sodium hydrogen carbonate solution with ices therein to stop the reaction. The reaction mixture was extracted with 100 ml of ethyl acetate, and the organic extract was washed with a saturated aqueous sodium hydrogen carbonate solution and with saturated saline and dried with anhydrous sodium sulfate.

The solvent was distilled off under reduced pressure and the residue was purified by silica gel column chromatography (hexane : ethyl acetate = 9 : 1) to obtain Compound PR-1130 (hereinafter, the step described above for obtaining Compound PR-1130 is sometimes referred to as "synthetic step A")

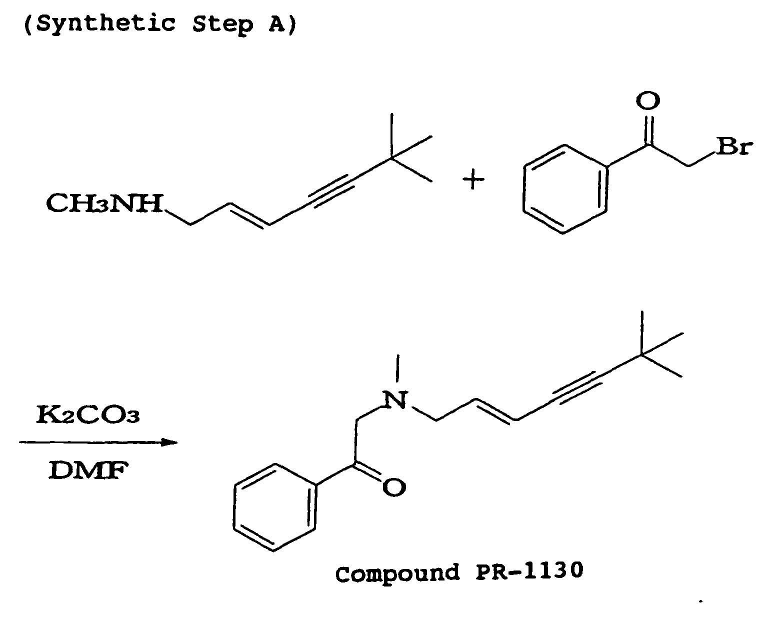

The yield, % yield and NMR spectrum of Compound PR-1130 are as follows.
Yield: 1.90 g, % yield: 32.2%.
¹H-NMR (CDCl₃, ppm)
1.24 (s, 9H), 2.37 (s, 3H), 3.21 (dd, J=6.89Hz, 1.49Hz, 2H), 3.81 (s, 2H), 5.66 (d, J=15. 9Hz, 1H), 6.09 (dt, J=15.9Hz, 6.89Hz, 1H), 7.43~8.00 (m, 5H)

0.51 g (1.89 mmol) of Compound PR-1130 was dissolved in 100 ml of diisopropyl ether (IPE), and 0.47 ml of 4N-hydrogen chloride- ethyl acetate was dropped while stirring at room temperature.

IPE (200 ml) was added and after 60 hours' stirring at room temperature, the crystals precipitated were collected by filtration, washed with IPE and dried under reduced pressure in a desiccator to obtain Compound PR-1254 as white crystals (this step corresponds to the step of hydrochlorination similar to Synthetic Step C described hereinbelow
Yield, % yield, melting point, NMR spectrum, and IR spectrum are as follows.
Yield: 0.31 g, % yield: 53.6%, melting point: 160 to 162°C
¹H-NMR (CDCl₃, ppm)
1.21 (s, 9H), 3.04 (s, 3H), 4.04 (d, J=7.56Hz, 2H), 4.57 (s, 2H), 5.82 (d, J=15.9Hz, 1H), 6.31 (dt, J=15.9Hz, 7.56Hz, 1H), 7.51~7.57 (m, 2H), 7.69 (m, 1H), 7.92 (m, 2H)
IR (KBr tablet, cm⁻¹
3426, 2969, 2931, 1693, 1453, 1250, 969, 758

### Example 2: Trans-N-(6,6-dimethyl-2-hepten-4-ynyl)-N-methyl-2-phenyl-2-propenylamine; (Compound PR-1257) and its hydrochloride; (Compound PR-1258)

### <Synthetic Method 2-1>

Compound PR-1130 was obtained by Synthetic Step A shown in Example 1.

On the other hand, in 15 ml of tetrahydrofuran (THF) was suspended 1.75 g (4.90 mmol) of methyltriphenylphosphonium bromide and while stirring under nitrogen atmosphere at room temperature, 3.6 ml (5.94 mmol) of 1.65 M n-butyllithium-hexane solution was dropped and stirred at room temperature for 15 minutes.

This was cooled in an ice bath and a solution of 1.10 g (4.08 mmol) of Compound PR-1130 dissolved in 20 ml of THF was dropped. After dropping, stirring was continued for 1 hour in an ice bath and for 3 hours at room temperature, followed by pouring into ice water to stop the reaction.

The reaction mixture was extracted with 100 ml of diethyl ether and the organic extract was washed with a saturated aqueous sodium hydrogen carbonate solution and with saturated saline. After drying with anhydrous sodium sulfate, the solvent was distilled off under reduced pressure and the resulting residue was purified by silica gel column chromatography (hexane : ethyl acetate = 9 : 1) to obtain Compound PR-1257 (hereinafter, the step of obtaining Compound PR-1257 from Compound PR-1130 is sometimes referred to as "Synthetic Step B"; Synthetic Step B is a step which utilizes Wittig reaction). 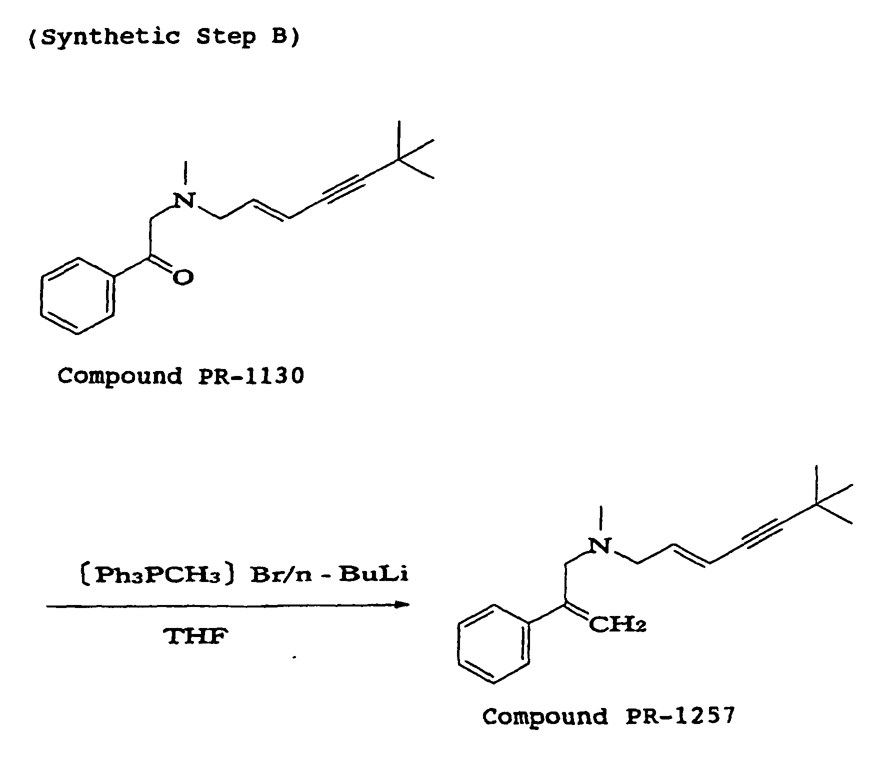 Yield, % yield and NMR spectrum of Compound PR-1257 are as follows.
Yield: 0.39 g, % yield: 28.4 %
¹H-NMR (CDCl₃, ppm)
1.24 (s, 9H), 2.20 (s, 3H), 3.05 (dd, J=1.08Hz, 6.75Hz, 2H), 3.34 (s, 2H), 5.24 (s, 1H), 5.44 (s, 1H), 5.61 (d, J=15. 0Hz, 1H), 6.05 (dt, J=15. 0Hz, 6.75Hz, 1H), 7.29~7. 50 (m, 5H)

0.39 g (1.41 mmol) of Compound 1257 was dissolved in 100 ml of diisopropyl ether, and 0.32 ml of 4N-hydrogen chloride-ethyl acetate solution was dropped while stirring.

After dropping, 150 ml of IPE was added and stirring was continued at room temperature for 72 hours. Thereafter, crystals precipitated were collected by filtration. After washing with IPE, the crystals were dried in a desiccator under reduced pressure to obtain Compound PR-1258 as white crystals (hereinafter, the step of obtaining, from Compound PR-1257, its hydrochloride, i.e., Compound PR-1258 is sometimes referred to as "Synthetic Step C"). 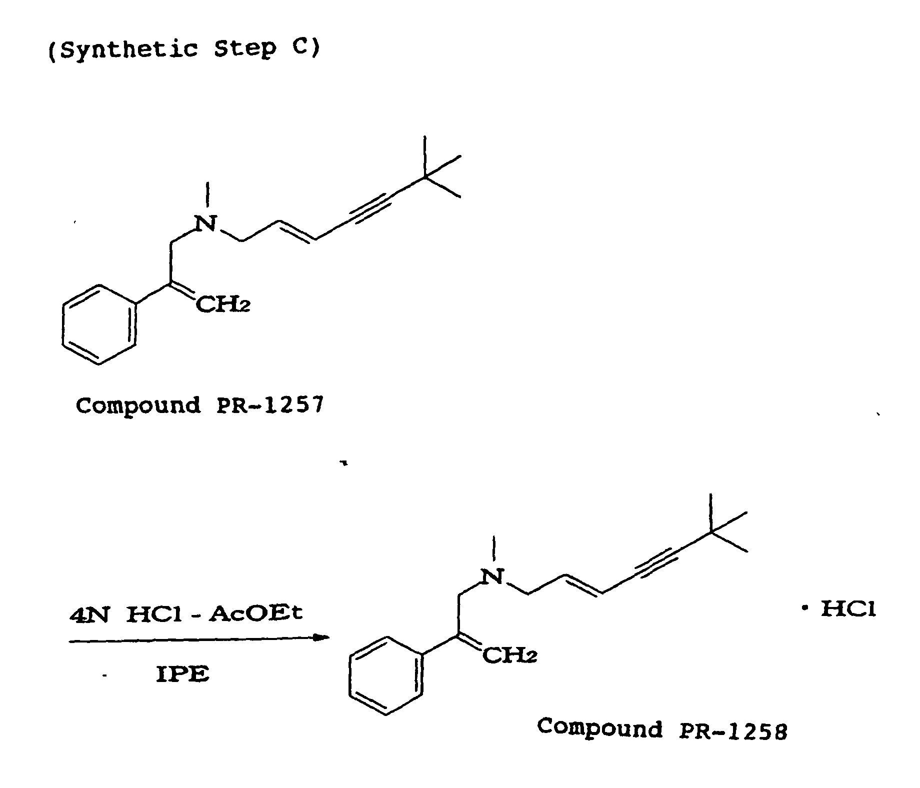 The yield, % yield, melting point, NMR spectrum and IR spectrum of Compound PR-1258 are as follows.
Yield: 0.29 g, % yield: 65.4%, melting point: 183 to 185°C
¹H-NMR (CDCl₃, ppm)
1.25 (s, 9H), 2.58 (d, J=4. 86Hz, 3H), 3.41~3. 65 (m, 2H), 4.00~4.15 (m, 2H), 5.63 (s, 1H), 5.69 (s, 1H), 5.84 (d, J=8. 91Hz, 1H), 6.23 (dt, J=15.7Hz, 7.56Hz, 1H), 7. 39~7. 45 (m, 5H), 1 2. 8 (s, 1H)
IR (KBr tablet, cm⁻¹) :
2971, 2930, 2906, 2690, 2672, 2632, 257 0, 2502, 1465

### <Synthetic Method 2-2>

N,N-Dimethylformamide (30 ml) was mixed with 3.36 g (22.2 mmol) of Compound PR-1133 and 4.19 g (30.3 mmol) of potassium carbonate and a solution of 3.88 g (19.7 mmol) of α-bromomethylstyrene (Compound PR-1392) obtained in Preparation Example 19 in 15 ml of DMF was dropped while stirring in an ice bath.

After dropping, the mixture was stirred for 12 hours at room temperature and the reaction mixture was poured in an ice water to stop the reaction. The reaction mixture was extracted with a mixed solvent containing 80 ml of diethyl ether and 20 ml of ethyl acetate, and the organic layer was washed with a saturated aqueous sodium hydrogen carbonate solution and with saturated saline and dried with anhydrous sodium sulfate.

The solvent was distilled off under reduced pressure and the residue was purified by silica gel column chromatography (hexane : ethyl acetate = 20 : 1) to obtain Compound PR-1257.
The yield, % yield and NMR are as follows.
Yield: 2.76 g, % yield: 46.5 %
¹H-NMR (CDCl₃, ppm)
1.24 (s, 9H), 2.19 (s, 3H), 3.05 (dd, J=1.35Hz, 6.35Hz, 2H), 3.34 (s, 2H), 5.23 (d, J = 1.35Hz, 1H), 5.44 (s, J = 1.35Hz, 1H), 5.61 (dt, J=15.7Hz, 1.49Hz, 1H), 6.05 (dt, J=15.9Hz, 6.48Hz, 1H), 7.23~7.50 (m, 5H)

### Example 3: Trans-2-[N-(6,6-dimethyl-2-henten-4-ynyl)-N-methylamino]-2'-methylacetophenone (Compound PR-1531), trans-N-(6, 6-dimethyl-2-hepten-4-ynyl)-N-methyl-[2-(o-tolyl)-2-propenyl]amine; (Compound PR-1532) and hydrochloride of Compound PR-1532 (Compound PR-1533)

In Synthetic Step A in Example 1, 2-bromo-2'-methylacetophenone obtained in Preparation Example 6 was used instead of 2-bromoacetophenone, and otherwise the procedures in Synthetic Step A were followed to obtain Compound PR-1531. The yield, % yield, NMR spectrum of Compound PR-1531 are as follows.
Yield: 1.74 g, % yield: 61.9%
¹H-NMR (CDCl₃, ppm)
1.24 (s, 9H), 2.36 (s, 3H), 2.49 (s, 3H), 3.19 (dd, J=6.89Hz, 1.35Hz, 2H), 3.73 (s, 2H), 5.64 (d, J=15.9Hz, 1H), 6.06 (dt, J=15.9Hz, 6.89Hz, 1H), 7.22~7.26 (m, 2H), 7.36 (m, 1H), 7.63 (m, 1H)

In Synthetic Step B in Example 2, Compound PR-1531 was used instead of Compound PR-1130 and, otherwise the procedures in Synthetic Step B were followed to obtain Compound PR-1532.
The yield, % yield and NMR spectrum of Compound PR-1532 are as follows.
Yield: 0.49 g, % yield: 28.4 %
¹H-NMR (CDCl₃, ppm)
1.24 (s, 9H), 2.24 (s, 3H), 2.30 (s, 3H), 3.05 (dd, J=6.49Hz, 1.35Hz, 2H), 3.17 (s, 2H), 5.04 (d, J=2.70Hz, 1H), 5.42 (d, J=2.16Hz, 1H), 5.58 (d, J=15.9Hz, 1H), 6.00 (dt, J=15.9Hz, 6.49Hz, 1H), 7.08~7.36 (m, 4H)

Further, in Synthetic Step C in Example 2, Compound PR-1532 was used instead of Compound PR-1257, and otherwise the procedures in Synthetic Step C were followed to obtain Compound PR-1533 as yellowish brown crystals. The yield, % yield, melting point, NMR spectrum, and IR spectrum of Compound PR-1533 are as follows.
Yield: 0.13 g, % yield: 23.5 %, melting point: 165 to 167°C
¹H-NMR (CDCl₃, ppm)
1.23 (s, 9H), 2.33 (s, 3H), 2.63 (d, J=4.59Hz, 3H), 3.44 (m, 2H), 3.95 (s, 2H), 5.44 (d, J=1 5.9Hz, 1H), 5.58 (s, 1H), 5.98 (s, 1H), 6.18 (dt, J=15.9Hz, 7.29Hz, 1H) , 7.1 3~7.31 (m, 4H), 12.8 (broad s, 1H) IR (KBrtablet, cm⁻¹) : 2967,2636

### Example 4: Trans-2-[N-(6,6-dimethyl-2-hepten-4-ynyl)-N-methylamino]-3'-methylacetophenone; (Compound PR-1538), trans-N-(6, 6-dimethyl-2-hepten-4-ynyl)-N-methyl-[2-(m-tolyl)-2-propenyl]amine (Compound PR-1539) and hydrochloride of Compound PR-1539 (Compound PR-1540)

In Synthetic Step A in Example 1, 2-bromo-3'-methylacetophenone obtained in Preparation Example 5 was used instead of 2-bromoacetophenone, and otherwise the procedures in Synthetic Step A were followed to obtain Compound PR-1538. The yield, % yield and NMR spectrum of Compound PR-1538 are as follows.
Yield: 1.11 g, % yield: 39.5 %
¹H-NMR (CDCl₃, ppm)
1.24 (s, 9H), 2.37 (s, 3H), 2.41 (s, 3H), 3.21 (dd, J=7.02Hz, 1.62Hz, 2H), 3.81 (s, 2H), 5.66 (d, J=15.9Hz, 1H), 6.10 (dt, J=15.9Hz, 7.02Hz, 1H), 7.31~7.39 (m, 2H), 7.76~7.78 (m, 2H)

In Synthetic Step B in Example 2, Compound PR-1538 was used instead of Compound PR-1130 and otherwise the procedures in Synthetic Step B were followed to obtain Compound PR-1539.
The yield, % yield and NMR spectrum of Compound PR-1539 are as follows.
Yield: 0.31 g, % yield: 28.1 %
¹H-NMR (CDCl₃, ppm)
1.24 (s, 9H), 2.22 (s, 3H), 2.36 (s, 3H), 3.05 (dd, J=6.35Hz, 1.35Hz, 2H), 3.32 (s, 2H), 5.22 (s, 1H), 5.41 (d, J=1.35Hz, 1H), 5.62 (d, J=15.9Hz, 1H), 6.05 (dt, J=15.9Hz, 6.35Hz, 1H), 7.07~7.35 (m, 4H)

Further, in Synthetic Step C in Example 2, Compound PR-1539 was used instead of Compound PR-1257, and otherwise the procedures in Synthetic Step C were followed to obtain Compound PR-1540 as pale yellow crystals. The yield, % yield, melting point, NMR spectrum and IR spectrum of Compound PR-1540 are as follows.
Yield: 0.18 g, % yield: 51.5 %, melting point: 145 to 147°C
¹H-NMR (CDCl₃, ppm)
1.23 (s, 9H), 2.39 (s, 3H), 2.58 (d, J=5.13Hz, 3H), 3.38~3.67 (m, 2H), 4.02~4.14 (m, 2H), 5.62 (d, J=15.4Hz, 1H), 5.80 (s, 1H×2 (=CH₂)), 6.23 (dt, J=15. 4Hz, 7.56Hz, 1H), 7.14~7.33 (m, 4H), 12.7 (broad s, 1H)
IR (KBrtablet, cm⁻¹)
2970, 2927, 2969

### Example 5: Trans-2-[N-(6,6-dimethyl-2-hepten-4-ynyl)-N-methylamino]-4'-methylacetophenone (Compound PR-1413), trans-N-(6, 6-dimethyl-2-hepten-4ynyl)-N-methyl-[2-p-tolyl)-2-propenyl]amine (Compound PR-1414) and hydrochloride of Compound PR-1414 (Compound PR-1415)

In Synthetic Step A in Example 1, 2-bromo-4'-methylacetophenone obtained in Preparation Example 21 was used instead of 2-bromoacetophenone and triethylamine was used instead of potassium carbonate, and otherwise the procedures in Synthetic Step A were followed to obtain Compound PR-1413. The yield, % yield and NMR spectrum are as follows.
Yield: 0.89 g, % yield: 31.4 %
¹H-NMR (CDCl₃, ppm)
1.23 (s, 9H), 2.36 (s, 3H), 2.41 (s, 3H), 3.20 (dd, J=7.02Hz, 1.08Hz, 2H), 3.79 (s, 2H), 5.56 (d, J=15.66Hz, 1H), 6.09 (dt, J=15.66Hz, 7.02Hz, 1H), 7.25 (d, J=8.37Hz, 2H), 7.89 (d, J=8.37Hz, 2H)

Further, in Synthetic Step B in Example 2, Compound PR-1413 was used instead of Compound PR-1130, and otherwise the procedures in Synthetic Step B were followed to obtain Compound PR-1414. The yield, % yield and NMR spectrum of Compound PR-1414 are as follows.
Yield: 0.50 g, % yield: 56.6 %
¹H-NMR (CDCl₃, ppm)
1.24 (s, 9H), 2.18 (s, 3H), 2.34 (s, 3H), 3.04 (dd, J=6. 48Hz, 1.62Hz, 2H), 3.32 (s, 2H), 5.18 (s, 2H), 5.40 (s, 1H), 5.61 (dt, J=15.93Hz, 1.62Hz, 1H), 6.05 (dt, J=15.93Hz, 6.48Hz, 1H), 7.13 (d, J=8.10Hz, 2H), 7.38 (d, J=8.10Hz, 2H)

Further, in Synthetic Step C in Example 2, Compound PR-1414 was used instead of Compound PR-1257 and otherwise the procedures in Synthetic Step C were followed to obtain Compound PR-1415 as crystals. The yield, % yield, melting point and NMR spectrum of Compound PR-1415 are as follows.
Yield: 0.51 g, % yield: 90.3 %, melting point: 169 to 170.5°C
¹H-NMR (CDCl₃, ppm)
1.23 (s, 9H), 2.37 (s, 3H), 2.57 (d, J=4.05Hz, 3H), 3.33~3.68 (m, 2H), 3.96~4.18 (m, 2H), 5.67 (d, J=15.66Hz, 1H), 5.78 (s, 2H), 6.23 (dt, J=15.66Hz, 7.56Hz, 1H), 7.21 (d, J=8.10Hz, 2H), 7.27 (d, J=8.10Hz, 2H), 12.69 (broad, 1H)

### Example 6: Trans-2-[N-(6,6-dimethyl-2-hepten-4-ynyl)-N-methylamino]-2'-fluoacetophenone (Compound PR-1489), trans-N-(6, 6'-dimethyl-2-hepten-4-ynyl)-N-methyl-[2-(2-fluorophenyl)-2-propenyl]amine (Compound PR-1490) and hydrochloride of Compound PR-1490 (Compound PR-1491)

In Synthetic Step A in Example 1, 2-bromo-2'-fluoroacetophenone obtained in Preparation Example 2 was used instead of 2-bromoacetophenone, and otherwise the procedures in Synthetic Step A were followed to obtain Compound PR-1489.
The yield, % yield and NMR spectrum of Compound PR-1489 are as follows.
Yield: 0.58 g, % yield: 30.5 %
¹H-NMR (CDCl₃, ppm)
1.23 (s, 9H), 2.39 (s, 3H), 3.22 (d, J=7.02Hz, 2H), 3.81 (d, J=2.97Hz, 2H), 5.63 (d, J=15.9Hz, 1H), 6.05 (dt, J=15.9Hz, 7.02Hz, 1H), 7.09~7.26 (m, 2H), 7.53 (m, 1H), 7.87 (m, 1H)

Further, in Synthetic Step B in Example 2, Compound PR-1489 was used instead of Compound PR-1130 and otherwise the procedures in Synthetic Step B were followed to obtain Compound PR-1490. The yield, % yield and NMR spectrum of Compound PR-1490 are as follows.
Yield: 0.14 g, % yield: 24.3%
¹H-NMR (CDCl₃, ppm)
1.23 (s, 9H), 2.18 (s, 3H), 3.02 (d, J=6.21Hz, 2H), 3.34 (s, 2H), 5.34 (s, 1H), 5.43 (s, 1H), 5.57 (d, J=15.9Hz, 1H), 5.98 (dt, J=15.9Hz, 6.21Hz, 1H), 7.02~7.34 (m, 4H)

Further, in Synthetic Step C in Example 2, Compound PR-1490 was used instead of Compound PR-1257 and otherwise the procedures in Synthetic Step C were followed to obtain Compound PR-1491 as white crystals. The yield, % yield, melting point, NMR spectrum, and IR spectrum of Compound PR-1491 are as follows.
Yield: 0.09 g, % yield: 56.9 %, melting point: 165 to 169°C
¹H-NMR (CDCl₃, ppm)
1.23 (s, 9H), 2.64 (d, J=4.32Hz, 3H), 3.50~3.64 (m, 2H), 3.95~4.16 (m, 2H), 5.67 (d, J=15.7Hz, 1H), 5.79 (s, 1H), 6.09 (s, 1H), 6.22 (dt, J=15.7Hz, 2.16Hz, 1H), 7.09~7.39 (m, 4H)
IR (KBrtablet, cm⁻¹) :
2971, 2590, 2569, 2537, 2513, 2481, 1489, 1453, 1412, 768

### Example 7: Trans-2-[N-(6,6-dimethyl-2-hepten-4-ynyl)-N-methylamino]-3'-fluoroacetophenone (Compound PR-1468), trans-N-(6, 6-dimethyl-2-hepten-4-ynyl)-N-methyl-[2-(3-fluorophenyl)-2-propenyl]amine (Compound PR-1469) and hydrochloride of Compound PR-1469 (Compound RR-1470)

In Synthetic Step A in Example 1, 2-bromo-3'-fluoroacetophenone obtained in Preparation Example 22 was used instead of 2-bromoacetaphonone and triethylamine was used instead of potassium carbonate, and otherwise the procedures in Synthetic Step A were followed to obtain compound PR-1468.
The yield, % yield and NMR spectrum of Compound PR-1468 are as follows.
Yield: 1.06 g, % yield: 46.1 %
¹H-NMR (CDCl₃, ppm)
1.25 (s, 9H), 2.36 (s, 3H), 3.19 (dd, J=6.75Hz, 1.08Hz, 2H), 3.77 (s, 2H), 5.65 (dt, J=15.93Hz, 1.08Hz, 1H), 6.07 (dt, J=15.93Hz, 6.75Hz, 1H), 7.27 (m, 1H), 7.44 (m, 1H), 7.69 (m, 1H), 7.78 (m, 1H)

Further, in Synthetic Step B in Example 2, Compound PR-1468 was used instead of Compound PR-1130 and otherwise the procedures in Synthetic Step B were followed to obtain Compound PR-1469. The yield, % yield and NMR spectrum of Compound PR-1469 are as follows.
Yield: 0.60 g, % yield: 57.0 %
¹H-NMR (CDCl₃, ppm)
1.24 (s, 9H), 2.19 (s, 3H), 3.04 (dd, J=6.48Hz, 1.62Hz, 1H), 3.31 (s, 2H), 5.62 (s, 1H), 5.46 (s, 1H), 5.61 (dt, J=15.66Hz, 1.62Hz, 1H), 6.04 (dt, J=15.66Hz, 6.48Hz, 1H), 6.96 (m, 1H), 7.18~7.38 (m, 3H)

Further, in Synthetic Step C in Example 2, Compound PR-1469 was used instead of Compound PR-1257 and otherwise the procedures in Synthetic Step C were followed to obtain Compound PR-1470. The yield, % yield, melting point and NMR spectrum of Compound PR-1470 are as follows.
Yield: 0.61 g, % yield: 90.1 %, melting point: 174 to 175.5°C
¹H-NMR (CDCl₃, ppm)
1.23 (s, 9H), 2.61 (d, 3H, J=4.59Hz), 3.43~3.73 (m, 2H), 3.98 (dd, J=13.77Hz, 4.32Hz, 1H), 4.13 (dd, J=13.77Hz, 2.97Hz, 1H), 5.71 (d, J=15.93Hz, 1H), 5.88 (s, 1H), 5.96 (s, 1H), 6.22 (dt, J=15.93Hz, 7.56Hz, 1H), 7.03~7. 14 (m, 2H), 7.18 (d, J= 7.83Hz, 1H), 7.40 (m, 1H), 12.82 (braod, 1H)

### Example 8: Trans-2-[N-(6,6-dimethyl-2-hepten-4-ynyl)-N-methylamino]-4'-fluoroacetophenone (Compound PR-1428), trans-N-(6, 6-dimethyl-2-hepten-4-ynyl)-N-methyl-[2-(4-fluorophenyl)-2-propenyl]amine; (Compound PR-1429) and hydrochloride of Compound PR-1429 (Compound PR-1430)

In Synthetic Step A in Example 1, 2-bromo-4'-fluorolacetophenone obtained in Preparation Example 1 was used instead of 2-bromoacetophenone and otherwise the procedures in Synthetic Step A were followed to obtain Compound PR-1428.
The yield, % yield and NMR spectrum of Compound PR-1428 are as follows.
Yield: 0.65 g, % yield: 34.2 %
¹H-NMR (CDCl₃, ppm)
1.23 (s, 9H), 2.35 (s, 3H), 3.19 (dd, J=6.75Hz, 1.35Hz, 2H), 3.76 (s, 2H), 5.65 (dd, J=15.7Hz, 1.35Hz, 1H), 6.09 (dt, J=15.7Hz, 6.75Hz, 1H), 7.07~7.13 (m, 2H), 8.01~8.0 8 (m, 2H)

Further, in Synthetic Step B in Example 2, Compound PR-1428 was used instead of Compound PR-1130 and otherwise the procedures in Synthetic Step B were followed to obtain Compound PR-1429. The yield, % yield and NMR spectrum of Compound PR-1429 are as follows.
Yield: 0.27 g, % yield: 41.9 %
¹H-NMR (CDCl₃, ppm)
1.24 (s, 9H), 2.18 (s, 3H), 3.03 (dd, J=6.48Hz, 1.62Hz, 2H), 3.30 (s, 2H), 5.20 (d, J=0.81Hz, 1H), 5.38 (d, J=0.81Hz, 1H), 5.61 (dt, J=15.7Hz, 1.62Hz, 1H), 6.03 (dt, J=15.7Hz, 6.48Hz, 1H), 6.96~7.03 (m, 2H), 7.43~7.50 (m, 2H)

Further, in Synthetic Step C in Example 2, Compound PR-1429 was used instead of Compound PR-1257 and otherwise the procedures in Synthetic Step C were followed to obtain Compound PR-1430 as white crystals. The yield, % yield, melting point, NMR spectrum and IR spectrum of Compound PR-1430 are as follows.
Yield: 0.14 g, % yield: 45.8 %, melting point: 194 to 196°C
¹H-NMR (CDCl₃, ppm)
1.23 (s, 9H), 2.60 (d, 3H, J=4.05Hz), 3.49~3.65 (m, 2H), 3.95~4.14 (m, 2H), 5.67 (s, 1H), 5.73 (s, 1H), 5.82 (d, J=16.7Hz, 1H), 6.22 (dt, J=16.7Hz, 7.56Hz), 7.08~7.16 (m, 2H), 7.36~7.41(m, 2H), 12.8 (broad s, 1H)
IR (KBr tablet, cm⁻¹)
2972, 2956, 2692, 2637, 1511, 1234, 1225, 841

### Example 9: Trans-2'-bromo-2-[N-(6,6-dimethyl-2-hepten-4-ynyl)-N-methylamino]acetophenone (Compound PR-1503), trans-N-(6,6-dimethyl-2-hepten-4-ynyl)-N-methyl-[2-(2-bromophenyl)-2-propenyl]amine (Compound PR-1504) and hydrochloride of Compound PR-1504 (Compound PR-1505)

In Synthetic Step A in Example 1, 2,2'-dibromoacetophenone obtained in Preparation Example 3 was used instead of 2-bromoacetophenone and otherwise the procedures in Synthetic Step A were followed to obtain Compound PR-1503.
The yield, % yield, and NMR spectrum of Compound PR-1503 are as follows.
Yield: 1.91 g, % yield: 55.3 %
¹H-NMR (CDCl₃, ppm)
1.24 (s, 9H), 2.39 (s, 3H), 3.20 (dd, J=6.48Hz, 1.62Hz, 2H), 3.74 (s, 2H), 5.62 (d, J=15.9Hz, 1H), 6.02 (dt, J=15.9Hz, 6.48Hz, 1H), 7.26~7.61 (m, 4H)

Further, in Synthetic Step B in Example 2, Compound PR-1503 was used instead of Compound PR-1130 and otherwise the procedures in Synthetic Step B were followed to obtain Compound PR-1504. The yield, % yield and NMR spectrum of Compound PR-1504 are as follows.
Yield: 0.30 g, % yield: 15.8 %
¹H-NMR (CDCl₃, p pm)
1.22 (s, 9H), 2.24 (s, 3H), 3.06 (dd, J=1.62Hz, 6.48Hz, 2H), 3.29 (s, 2H), 5.15 (s, 1H), 5.47 (d, J=1.89Hz, 1H), 5.57 (d, J=15.9Hz, 1H), 5.99 (dt, J=15.9Hz, 6.48Hz, 1H), 7.09~7.57 (m, 4H)

Further, in Synthetic Step C in Example 2, Compound PR-1504 was used instead of Compound PR-1257 and otherwise the procedures in Synthetic Step C were followed to obtain Compound PR-1505 as yellowish brown crystals. The yield, % yield, melting point, NMR spectrum, and IR spectrum of Compound PR-1505 are as follows.
Yield: 0.24 g, % yield: 72.4%, melting point: 127 to 132°C
¹H-NMR (CDCl₃, ppm)
1.23 (s, 9H), 2.68 (d, J=4.86Hz, 3H), 3.49~3.66 (m, 2H), 3.93~4.08 (m, 2H), 5.59 (d, J=1 5.9Hz, 1H), 5.71 (d, J=1.89Hz, 1H), 6.12 (s, 1H), 6.21 (dt, J=15.9Hz, 7.02Hz, 1H), 7.22~7.65 (m, 4H), 12.7 (broad s, 1H)
IR (KBr tablet, cm⁻¹) :
2969, 2628, 2612, 2499, 1471

### Example 10 : Trans-3'-bromo-2-[N-(6,6-dimethyl-2-hepten-4-ynyl)-N-methylamino]acetophenone (Compound PR-1482),trans-N-(6,6-dimethyl-2-hepten-4-ynyl)-N-meth yl-[2-(3-bromophenyl)-2-propenyl]amine (Compound PR-1483) and hydrochloride of Compound PR-1483; (Compound PR-1484)

In Synthetic Step A in Example 1, 2',3'-dibromoacetophenone obtained in Preparation Example 4 was used instead of 2-bromoacetophenone and otherwise the procedures in Synthetic Step A were followed to obtain Compound PR-1482.
The yield, % yield and NMR spectrum of Compound PR-1482 are as follows.
Yield: 1.20 g, % yield: 43.4 %
¹H-NMR (CDCl₃, ppm)
1.26 (s, 9H), 2.35 (s, 3H), 3.19 (dd, J=1.35Hz, 6.75Hz, 2H), 3.76 (s, 2H), 5.65 (d d, J=15.8Hz, 1.35Hz, 1H), 6.07 (dt, J=15.8Hz, 6.75Hz), 7.34 (t, J=7.83Hz, 1H), 7.69 (m, 1H), 7.92 (m, 1H), 8.13 (m, 1H)

Further, in Synthetic Step B in Example 2, Compound PR-1482 was used instead of Compound PR-1130 and otherwise the procedures in Synthetic Step B were followed to obtain Compound PR-1483. The yield, % yield and NMR spectrum of Compound PR-1483 are as follows.
Yield: 0.82 g, % yield: 68.6 %
¹H-NMR (CDCl₃, ppm)
1.24 (s, 9H), 2.18 (s, 3H), 3.04 (dd, J=1.35Hz, 6.75Hz, 2H), 3.29 (s, 2H), 5.26 (d, J=1.35Hz, 1H), 5.44 (d, J=1.35Hz, 1H), 5.62 (dt, J=15.7Hz, 1.35Hz, 1H), 6.35(dt, J=15.7Hz, 1.35Hz, 1H), 7.18 (t, J=7.83Hz, 1H), 7.33~7.43 (m, 2H), 7.63 (t, J=1.89Hz, 1H)

Further, in Synthetic Step C in Example 2, Compound PR-1483 was used instead of Compound PR-1257 and otherwise the procedures in Synthetic Step C were followed to obtain Compound PR-1484 as white crystals. The yield, % yield, melting point, NMR spectrum and IR spectrum of Compound PR-1484 are as follows.
Yield: 0.70 g, % yield: 77.2 %, melting point: 169 to 171°C
¹H-NMR (CDCl₃, ppm)
1.24 (s, 9H), 2.61 (d, J=4.32Hz, 3H), 3.49~3.66 (m, 2H), 3.93~4.14 (m, 2H), 5.70 (d, J=15.9Hz, 1H), 5.86 (s, 1H), 5.96 (s, 1H), 6.23 (dt, J=15.9Hz, 7.29Hz, 1H), 7.30~7.54 (m, 4H), 12.9 (broad s, 1H)
IR (KBrtablet, cm-1) :
2970, 2487

### Example 11: trans-4'-bromo-2-[N-(6,6-dimethyl-2-hepten-4-ynyl)-N-methylamino]acetophenone (Compound PR-1437), trans-N-(6,6-dimethyl-2-hepten-4-ynyl)-N-methyl-[2-(4-bromophenyl)-2-propenyl]amine (Compound PR-1438) and hydrochloride of Compound PR-1438; (Compound PR-1439)

In Synthetic Step A in Example 1, 2,4'-dibromoacetophenone (manufactured by Aldrich Company) was used instead of 2-bromoacetophenone and otherwise the procedures in Synthetic Step A were followed to obtain Compound PR-1437.
The yield, % yield and NMR spectrum of Compound PR-1437 are as follows.
Yield: 0.92 g, % yield: 40.0 %
¹H-NMR (CDCl₃, ppm)
1.23 (s, 9H), 2.36 (s, 3H), 3.21 (d, 2H, J=6.75Hz), 3.77 (s, 2H), 5.65 (d, J=15.9Hz, 1H), 6.06 (dt, 1H, J=15.9Hz, 6.75Hz), 7.60 (d, J=6.75Hz, 2H), 7.87 (d, J=6.75Hz, 2H)

Further, in Synthetic Step B in Example 2, Compound PR-1437 was used instead of Compound PR-1130 and otherwise the procedures in Synthetic Step B were followed to obtain Compound PR-1438. The yield, % yield and NMR spectrum of Compound PR-1438 are as follows.
Yield: 0.32 g, % yield: 35.0 %
¹H-NMR (CDCl₃, ppm)
1.24 (s, 9H), 2.13 (s, 3H), 3.02 (dd, J=6.48Hz, 1.35Hz, 2H), 3.30 (s, 3H), 5.23 (d,J=0.81Hz, 1H), 5.43 (d, J=0.81Hz, 1H), 5.60 (dt, J=15.7Hz, 1.49Hz, 1H), 6.01(dt, J=15.7Hz, 6.48Hz, 1H), 7.35 (d, J=8.37Hz, 2H), 7.44 (d, J=8.37Hz, 2H)

Further, in Synthetic Step C in Example 2, Compound PR-1438 was used instead of Compound PR-1257 and otherwise the procedures in Synthetic Step C were followed to obtain Compound PR-1439 as white crystals. The yield, % yield, melting point, NMR spectrum and IR spectrum of Compound PR-1439 are as follows.
Yield: 0.30 g, % yield: 84.8 %, melting point: 174.3 to 178.3°C
1.22 (s, 9H), 2.58 (s, 3H), 3.42~3.65 (m, 2H), 3.98~4.12 (m, 2H), 5.70 (d, J=15.7Hz, 1H), 5.83~5.91 (m, 2H), 6.12 (m, 1H), 7.24~7.57 (m, 4H)
IR (KBr tablet, cm⁻¹) :
2969, 2631, 1467, 1395, 1070, 964, 932, 831

### Example 12: Trans-2'-chloro-2-[N-(6,6-dimethylhepten-4-ynyl)-N-methylamino]acetophenone (Compound PR-1496), trans-N-(6,6-dimethyl-2-hepten-4-ynyl)-N-methyl-[2-(2-chlorophenyl)-2-propenyl]amine (Compound PR-1497) and hydrochloride of Compound PR-1497; (Compound PR-1498)

In Synthetic Step A in Example 1, 2-bromo-2'-chloroacetophenone obtained in Preparation Example 23 was used instead of 2-bromoacetophenone and otherwise the procedures in Synthetic Step A were followed to obtain Compound PR-1496.
The yield, % yield and NMR spectrum of Compound PR-1496 are as follows.
Yield: 1.08 g, % yield: 35.6 %
¹H-NMR (CDCl₃, ppm)
1.24 (s, 9H), 2.37 (s, 3H), 3.19 (dd, J=6.75Hz, 1.35Hz, 2H), 3.76 (s, 2H), 5.61 (dt, J=15.66Hz, 1.35Hz, 1H), 6.01 (dt, J=15.66Hz, 6.75Hz, 1H), 7.25~7.48 (m, 4H)

Further, in Synthetic Step B in Example 2, Compound PR-1496 was used instead of Compound PR-1130 and otherwise the procedures in Synthetic Step B were followed to obtain Compound PR-1469. The yield, % yield and NMR spectrum of Compound PR-1497 are as follows.
Yield: 0.49 g, % yield: 45.7 %
¹H-NMR (CDCl₃, ppm)
1.23 (s, 9H), 2.22 (s, 3H), 3.04 (dd, J=6.48Hz, 1.62Hz, 2H), 3.31 (s, 2H), 5.17 (s, 1H), 5.46 (s, 1H), 5.56 (dt, J=15.93Hz, 1.62Hz, 1H), 5.98 (dt, J=15.93Hz, 6.48 Hz, 1H), 7.21 (m, 3H), 7.35 (m, 1H)

Further, in Synthetic Step C in Example 2, Compound PR-1497 was used instead of Compound PR-1257 and otherwise the procedures in Synthetic Step C were followed to obtain Compound PR-1498 as crystals. The yield, % yield, melting point, and NMR spectrum of Compound PR-1498 are as follows.
Yield: 0.45 g, % yield: 81.9 %, melting point: 157 to 159°C
¹H-NMR (CDCl₃, ppm)
1.23 (s, 9H), 2.67 (d, J=5.13Hz, 3H), 3.42~3.70 (m, 2H), 4.01 (dd, J=14.31Hz, 4.32Hz, 1H), 4.10 (dd, J=14.31Hz, 3.51Hz, 1H), 5.60 (d, J=15.93Hz, 1H), 5.71 (s, 1H), 6.13 (s, 1H), 6.21 (dt, J=15.93Hz, 7.56Hz, 1H), 7.28~7.37 (m, 3H), 7.45 (dd, J=8.64Hz, 3.78Hz, 1H), 12.81 (broad, 1H)

### Example 13: Trans-4'-chloro-2-[N-(6,6-dimethyl-2-hepten-4-ynyl)-N-methylamino]acetophenone (Compound PR-1416),trans-N-(6,6-dimethyl-2-hepten-4-ynyl)-N-methyl-[2-(4-chlorophenyl)-2-propenyl]amine (Compound PR 1417) and hydrochloride of Compound PR-1417 (Compound PR-1418)

In Synthetic Step A in Example 1, 2-bromo-4'-chloroacetophenone obtained in Preparation Example 6 was used instead of 2-bromoacetophenone and otherwise the procedures in Synthetic Step A were followed to obtain Compound PR-1416.
The yield, % yield and NMR spectrum of Compound PR-1416 are as follows.
Yield: 1.80 g, % yield: 59.7 %
¹H-NMR (CDCl₃, ppm)
1.24 (s, 9H), 2.35 (s, 3H), 3.19 (dd, J=7.02Hz, 1.35Hz, 2H), 3.75 (s, 2H), 5.65 (dd, J=15.7Hz, 1.35Hz, 1H), 6.21 (dt, J=15.9Hz, 7.02Hz, 1H), 7.43 (d, J=8.91Hz, 2H), 7.95 (d, J=8.91Hz, 2H)

Further, in Synthetic Step B in Example 2, Compound PR-1416 was used instead of Compound PR-1130 and otherwise the procedures in Synthetic Step B were followed to obtain Compound PR-1417. The yield, % yield and NMR spectrum of Compound PR-1417 are as follows.
Yield: 0.89 g, % yield: 49.8 %
¹H-NMR (CDCl₃, p pm)
1.24 (s, 9H), 2.17 (s, 3H), 3.03 (dd, J=6.48Hz, 1.62Hz, 2H), 3.31 (s, 2H), 5.23 (s, 1H), 5.43 (s, 1H), 5.61 (d, J=15.9Hz, 1H), 6.02 (dt, J=15.9Hz, 6.48Hz, 1H), 7.26~7.44 (m, 4H)

Further, in Synthetic Step C in Example 2, Compound PR-1417 was used instead of Compound PR-1257 and otherwise the procedures in Synthetic Step C were followed to obtain Compound PR-1418 as white crystals. The yield, % yield, melting point, NMR spectrum and IR spectrum of Compound PR-1418 are as follows.
Yield: 0.62 g, % yield: 62.1 %, melting point: 180.0 to 182.5°C
¹H-NMR (CDCl₃, ppm)
1.19 (s, 9H), 2.60 (d, J=4.86Hz, 3H), 3.48~3.66 (m, 2H), 3.94~4.10 (m, 2H), 5.66 (s, 1H), 5.73 (s, 1H), 5.88 (d, J=15.4Hz, 1H), 6.21 (dt, J=15.4Hz, 7.29Hz, 1H), 7.33 (d, J=3.65Hz, 2H), 7.40 (d, J=3.65Hz, 2H)
IR (KBr tablet, cm⁻¹) :
2970, 2629, 2502, 1493, 1467, 1396, 964, 835

### Example 14: Trans-2-[N-(6,6-dimethyl-2-hepten-4-ynyl)-N-methylamino]-2'-methoxyacetophenone (Compound PR-1632), trans-N-(6,6-dimethyl-2-hepten-4-ynyl)-N-methyl-[2-(2-methoxyphenyl)-2-propenyl]amine (Compound PR-1633) and hydrochloride of Compound PR-1633 (Compound PR-1634)

In Synthetic Step A in Example 1, 2-bromo-2'-methoxyacetophenone obtained in Preparation Example 7 was used instead of 2-bromoacetophenone and sodium carbonate was used instead of potassium carbonate, and otherwise the procedures in Synthetic Step A were followed to obtain Compound PR-1632. The yield, % yield and NMR spectrum of Compound PR-1632 are as follows.
Yield: 0.65 g, % yield: 14.8 %
¹H-NMR (CDCl₃, ppm)
1.24 (s, 9H), 3.56 (s, 3H), 3.20 (dd, J=6.89Hz, 1.49Hz, 2H), 3.82 (s, 2H), 3.91 (s, 3H), 5.61 (dd, J=1 5.9Hz, 1.49Hz, 1H), 6.06 (dt, J=15.9Hz, 6.89Hz, 1H), 6.94~7.03 (m, 2H), 7.46 (m, 1H), 7.70 (m, 1H)

Further, in Synthetic Step B in Example 2, Compound PR-1632 was used instead of Compound PR-1130 and otherwise the procedures in Synthetic Step B were followed to obtain Compound PR-1633. The yield, % yield and NMR spectrum Compound PR-1633 are as follows.
Yield: 0.17, % yield: 30.6 %
¹H-NMR (CDCl₃, ppm)
1.21 (s, 9H), 2.16 (s, 3H), 2.99 (dd, J=6.48Hz, 1.62Hz, 2H), 3.36 (s, 2H), 3.83 (s, 3H), 5.16 (d, J=2.43Hz, 1H), 5.32 (d, J=2.43Hz, 1H), 5.53 (dt, J=15.9Hz, 1.62Hz, 1H), 5.96 (dt, J=15.9Hz, 6.48Hz, 1H), 6.85~6.94(m, 2H), 7.15~7.28 (m, 2H)

Further, in Synthetic Step C in Example 2, Compound PR-1633 was used instead of Compound PR-1257 and otherwise the procedures in Synthetic Step C were followed to obtain Compound PR-1634 as white crystals. The yield, % yield, melting point, NMR spectrum and IR spectrum of Compound PR-1634 are as follows.
Yield: 0.17 g, % yield: 89.2 %, melting point: 148 to 150°C
¹H-NMR (CDCl₃, ppm)
1.23 (s, 9H), 2.61 (d, J=4.86Hz, 3H), 3.45~3.47 (m, 2H), 3.87 (s, 3H), 4.13 (d, J=3.78Hz, 2H), 5.46 (d, J=15.9Hz, 1H), 5.61 (s, 1H), 5.79 (s, 1H), 6.21 (dt, J=15.9Hz, 7.29Hz, 1H), 6.91~7.02 (m, 2H), 7.18~7.40 (m, 2H), 12.5 (broad s, 1H)
IR (KBrtablet, cm⁻¹) :
2966, 2933, 1459, 1256, 757

### Example 15: Trans-2-[N-(6,6-dimethyl-2-hepten-4-ynyl)-N-methylamino]-3'-methoxyacetophenone (Compound PR-1388), trans-N-(6,6-dimethyl-2-hepten-4-ynyl)-N-methyl-[2-(3-methoxyphenyl)-2-propenyl]amine (Compound PR-1389) and hydrochloride of Compound PR-1389; (Compound PR-1390)

In Synthetic Step A in Example 1, 2-bromo-3'-methoxyacetophenone (manufacutred by Aldrich Company) was used instead of 2-bromoacetophenone and triethylamine was used instead of potassium carbonate, and otherwise the procedures in Synthetic Step A were followed to obtain Compound PR-1388. The yield, % yield and NMR spectrum of Compound PR-1388 are as follows.
Yield: 2.58 g, % yield: 65.1 %
¹H-NMR (CDCl₃, ppm)
1.23 (s, 9H), 2.37 (s, 3H), 3.21 (dd, J=6.75Hz, 1.35Hz, 1H), 3.80 (s, 2H), 3.86 (s, 3H), 5.66 (dt, J=15.66Hz, 1.35Hz, 1H), 6.09 (dt, J=15.66Hz, 6.75Hz, 1H), 7.11 (ddd, J=7.83, 1.62Hz, 1.08Hz, 1H), 7.36 (t, J=7.83Hz, 1H), 7.51 (dd, J=2.43Hz, 1.62Hz, 1H), 7.57 (ddd, J=7. 83Hz, 2.43Hz, 1.08Hz, 1H)

Further, in Synthetic Step B in Example 2, Compound PR-1388 was used instead of Compound PR-1130 and otherwise the procedures in Synthetic Step B were followed to obtain Compound PR-1389. The yield, % yield and NMR spectrum of Compound PR-1389 are as follows.
Yield: 0.44 g, % yield: 17.2 %
¹H-NMR (CDCl₃, ppm)
1.24 (s, 9H), 2.20 (s, 3H), 3.05 (dd, J=6.48Hz, 1.35Hz), 3.31 (s, 2H), 3.83 (s, 3H), 5.23 (s, 1H), 5.44 (s, 1H) , 5.62 (dt, J=15.93Hz, 1.35Hz, 1H), 6.05 (dt, J=15.93Hz, 6.48Hz, 1H), 6.82 (ddd, J=7.83Hz, 1.62Hz, 1.08Hz, 1H), 7.03~7.10 (m, 2H), 7.24 (t, J=7.83Hz)

Further, in Synthetic Step C in Example 2, Compound PR-1389 was used instead of Compound PR-1257 and otherwise the procedures in Synthetic Step C were followed to obtain Compound PR-1390 as crystals. The yield, % yield, melting point, NMR spectrum of Compound PR-1390 are as follows.
Yield: 0.44 g, % yield: 89.1 %, melting point: 142 to 144°C
¹H-NMR (CDCl₃, ppm)
1.23 (s, 9H), 2.59 (d, J=5.13Hz, 3H), 3.45 (m, 1H), 3.61 (m, 1H), 3.85 (s, 3H), 3.96~4.16 (m, 2H), 5.66 (d, J=1 5.93Hz, 1H), 5.83 (s, 1H), 5.85 (s, 1H), 6.23 (dt, J=15.93Hz, 7.56Hz, 1H), 6.86~6.98 (m, 3H), 7.33 (t, J=7.83Hz, 1H), 12.75 (broad, 1H)

### Example 16: Trans-2-[N-(6,6-dimethyl-2-hepten-4-ynyl)-N-methylamino]-2'-nitroacetophenone (Compound PR-1639), trans-N-(6,6-dimethyl-2-hepten-4-ynyl)-N-methyl-[2-(2-nitrophenyl)-2-propenyl]amine (Compound PR-1640) and hydrochloride of Compound PR-1640; (Compound PR-1641)

In Synthetic Step A in Example 1, 2-bromo-2'-nitroacetophenone was used instead of 2-bromoacetophenone and otherwise the procedures in Synthetic Step A were followed to obtain Compound PR-1639.

Further, in Synthetic Step B in Example 2, Compound PR-1639 was used instead of Compound PR-1130 and sodium carbonate was used instead of potassium carbonate, and otherwise the procedures in Synthetic Step B were followed to obtain Compound PR-1640. The yield, % yield and NMR spectrum of Compound PR-1640 are as follows.
Yield: 0.43 g, % yield: 10.6 %
¹H-NMR (CDCl₃, ppm)
1.23 (s, 9H), 2.16 (s, 3H), 2.98 (dd, J=6.35Hz, 1.49Hz, 2H), 3.24 (s, 2H), 5.18 (d, J=0.8 1Hz, 1H), 5.38 (d, J=0.81Hz, 1H), 5.53 (dt, J=15.9Hz, 1.49Hz, 1H), 5.90 (dt, J=15.9Hz, 6.35Hz, 1H), 7.33~7.44 (m, 2H), 7.54 (m, 1H), 1.86 (m, 1H)

Further, in Synthetic Step C in Example 2, Compound PR-1640 was used instead of Compound PR-1257 and otherwise the procedures in Synthetic Step C were followed to obtain Compound PR-1641 as pale brown crystals. The yield, % yield, melting point, NMR spectrum and IR spectrum of Compound PR-1641 are as follows.
Yield: 0.09 g, % yield: 90.0 %, melting point: 162 to 163°C
¹H-NMR (CDCl₃, ppm)
1.19 (s, 9H), 2.78 (s, 3H), 3.72~4.06 (m, -CH₂-×₂), 5.60 (s, 1H), 5.73 (d, J=15.9Hz, 1H), 6.23 (dt, J=15.9Hz, 7.29Hz, 1H), 7.48~7.60 (m, 2H), 7.70 (m, 1H), 8.07 (m, 1H), 12.9 (broad s, 1H)
IR (KBrtablet, cm⁻¹) :
3448, 3426, 2968, 2927, 2866, 2687, 2664, 2632, 2657, 2655, 2495, 1527, 1465, 1408, 1343, 969, 764

### Example 17: Trans-2-[N-6,6-dimethyl-2-hepten-4-ynyl)-N-methylamino]-3'-nitroacetophenone (Compound PR-1646), trans-N-(6, 6-dimethyl-2-hepten-4-ynyl)-N-methyl-[2-(3-nitrophenyl)-2-propenyl]amine (Compound PR-1647) and hydrochloride of Compound PR-1647 (Compound PR-1648)

In Synthetic Step A in Example 1, 2-bromo-3'-nitroacetophenone obtained in Preparation Example 9 was used instead of 2-bromoacetophenone and sodium carbonate was used instead of potassium carbonate, and otherwise the procedures in Synthetic Step A were followed to obtain Compound PR-1646.

Further, in Synthetic Step B in Example 2, Compound PR-1646 was used instead of Compound PR-1130 and otherwise the procedures in Synthetic Step B were followed to obtain Compound PR-1647. The yield, % yield and NMR spectrum of Compound PR-1647 are as follows.
Yield: 0.61 g, % yield: 16.2 %
¹H-NMR (CDCl₃, ppm)
1.24 (s, 9H), 2.19 (s, 3H), 3.05 (dd, J=6.48Hz, 1.35Hz, 2H), 3.37 (s, 2H), 5.36 (d, J=1.08Hz, 1H), 5.57 (s, 1H), 5.62 (d, J=15.7Hz, 1H), 6.03 (dt, J=15.7Hz, 6.48Hz, 1H), 7.49 (t, J=7.83Hz, 1H), 7.83 (d, J=7.02Hz, 1H), 8.13 (d, J=5.94Hz, 1H), 8.38 (t, J=2.03Hz, 1H)

Further, in Synthetic Step C in Example 2, Compound PR-1647 was used instead of Compound PR-1257 and otherwise the procedures in Synthetic Step C were followed to obtain Compound PR-1648 as pale yellow crystals. The yield, % yield, melting point, NMR spectrum and IR spectrum of Compound PR-1648 are as follows.
Yield: 0.12 g, % yield: 89.1 %, melting point: 161 to 164°C
¹H-NMR (CDCl₃, ppm)
1.24 (s, 9H), 2.67 (d, J=4.86Hz, 3H), 3.54~3.63 (m, 2H), 3.94~4.21 (m, 2H), 5.64 (d, J=15.9Hz, 1H), 5.99 (s, 1H), 6.12 (s, 1H), 6.22 (dt, J=15.9Hz, 7.70Hz, 1H), 7.65 (t, J=8.10Hz, 1H), 7.81 (m, 1H), 8.24~8.27 (m, 2H), 13.0 (broad s , 1H)
IR (KBrtablet, cm⁻¹) :
2971, 2631, 1534, 1347

### Example 18: Trans-2-[N-(6,6-dimethyl-2-hepten-4-ynyl)-N-methylamino]-4'-nitroacetophenone (Compound PR-1393), trans-N-(6,6-dimethyl-2-hepten-4-ynyl)-N-methyl-[2-(4-nitrophenyl)-2-propenyl]amine (Compound PR-1394) and hydrochloride of Compound PR-1394 (Compound PR-1395)

In Synthetic Step A in Example 1, 2-bromo-4'-nitroacetophenone obtained in Preparation Example 9 was used instead of 2-bromoacetophenone and sodium carbonate was used instead of potassium carbonate, and otherwise the procedures in Synthetic Step A were followed to obtain Compound PR-1393.

Further, in Synthetic Step B in Example 2, Compound PR-1393 was used instead of Compound PR-1130 and otherwise the procedures in Synthetic Step B were followed to obtain Compound PR-1394. The yield, % yield and NMR spectrum of Compound PR-1394 are as follows.
Yield: 0.18 g, % yield: 6.3 %
¹H-NMR (CDCl₃, ppm)
1.22 (s, 9H), 2.18 (s, 3H), 3.04 (dd, J=6.48Hz, 1.22Hz, 2H), 3.61 (s, 2H), 5.40 (d, J=0.81Hz, 1H), 5.58 (s, 1H), 5.61 (d, 1H, J=14.3Hz), 6.00 (dt, J=14.3Hz, 6.48Hz, 1H), 7.65 (d, J=8.78Hz, 2H), 8.18 (d, J=8.78Hz, 2H)

Further, in Synthetic Step C in Example 2, Compound PR-1394 was used instead of Compound PR-1257 and otherwise the procedures in Synthetic Step C were followed to obtain Compound PR-1395 as yellowish brown crystals. The yield, % yield, melting point, NMR spectrum and IR spectrum of Compound PR-1395 are as follows.
Yield: 0.14 g, % yield: 69.7 %, melting point: 159 to 162°C
¹H-NMR (CDCl₃, ppm)
1.24 (s, 9H), 2.65 (d, 3H), 3.58~3.66 (m, 2H), 3.95~4.24 (m, 2H), 5.75 (d, J=15.4Hz, 2H), 6.00 (s, 1H), 6.14 (s, 1H), 6.21 (dt, J=15.4Hz, 7.56Hz, 2H), 7.60 (d, J=8.64Hz, 2H), 8.29 (d, J=8.64Hz, 2H), 13.0 (broad s, 1H)
IR (KBrtablet, cm⁻¹) :
3470, 2971, 2933, 2908, 2870, 2694, 2676, 2628, 2572, 2504, 1599, 1524, 1471, 1463, 1395, 1345, 963, 936, 859

### Example 19: Trans-3'-cyano-2-[N-(6,6-dimethyl-2-henten-4-ynyl)-N-methylamino]acetophenone (Compound PR-1552), trans-3-{1-[N-(6,6-dimethyl-2-hepten-4-ynyl )-N-methylamino]methyl}vinylbenzonitrile (Compound PR-1553) and hydrochloride of Compound PR-1553 (Compound PR-1554)

In Synthetic Step A in Example 1, 2-bromo-3'-cyanoacetophenone obtained in Preparation Example 13 was used instead of 2-bromoacetophenone and otherwise the procedures in Synthetic Step A were followed to obtain Compound PR-1552. The yield, % yield and NMR spectrum of Compound PR-1552 are as follows.
Yield: 1.05 g, % yield: 56.6 %
¹H-NMR (CDCl₃, ppm)
1.26 (s, 9H), 2.34 (s, 3H), 3.18 (dd, J=6.89Hz, 1.49Hz, 2H), 3.76 (s, 2H), 5.66 (d, 1H, J=15.9Hz), 6.04 (dt, J=15.9Hz, 1.62Hz, 1H), 7.60 (t, J=8.10Hz, 1H), 7.85 (m, 1H), 8.25 (m, 1H), 8.33 (d, 1H, J=1.35Hz)

Further, in Synthetic Step B in Example 2, Compound PR-1552 was used instead of Compound PR-1130 and otherwise the procedures in Synthetic Step B were followed to obtain Compound PR-1553. The yield, % yield and NMR spectrum of Compound PR-1553 are as follows.
Yield: 0.21 g, % yield: 20.0 %
¹H-NMR (CDCl₃, ppm)
1.24 (s, 9H), 2.17 (s, 3H), 3.03 (dd, J=1.35Hz, 6.62Hz, 2H), 3.33 (s, 2H), 5.32 (s, 1H), 5.49 (s, 1H), 5.60 (dd, J=15.9Hz, 1.35Hz, 1H), 6.01 (dt, J=15.9Hz, 6.62Hz, 1H), 7.42 (t, 1H, J=7.56Hz)), 7.55 (dt, J=7.29Hz, 1.35Hz, 1H), 7.72 (dt, J=8.10Hz, 1.35Hz, 1H), 7.80 (d, J=1.35Hz, 1H)

Further, in Synthetic Step C in Example 2, Compound PR-1553 was used instead of Compound PR-1257 and otherwise the procedures in Synthetic Step C were followed to obtain Compound PR-1554 as white crystals. The yield, % yield, melting point, NMR spectrum and IR spectrum of Compound PR-1554 are as follows.
Yield: 0.23 g, % yield: 97.4%, melting point: 150 to 153°C
¹H-NMR (CDCl₃, ppm)
1.24 (s, 9H), 2.66 (d, J=4. 59Hz, 3H), 3.53~3.67 (m, 2H), 3.92~4.20 (m, 2H), 5.75 (d, J=15.1Hz, 1H), 5.92 (s, 1H), 6.08 (s, 1H), 6.21 (dt, J=15.9Hz, 7.56Hz, 1H), 7.57 (m, 1H), 7.69 (m, 1H), 12.9 (broad s, 1H)
IR (KBrtablet, cm⁻¹) :
3425, 2972, 2934, 2908, 2871, 2692, 2676, 2595, 2574, 2530, 2501, 2231, 1469, 1421, 1408, 1395, 963, 935, 802

### Example 20: Trans-4'-cyano-2-[N-(6,6-dimethyl-2-hepten-4-ynyl)-N-methylamino]acetophenone (Compound PR-1559), trans-4-{1-[N-(6,6-dimethyl-2-hepten-4-ynyl)-N-metnylamino]metnyl}vinylbenzonitrile (Compound PR-1560) and hydrochloride of Compound PR-1560 (Compound PR-1561)

In Synthetic Step A in Example 1, 2-bromo-4'-cyanoacetophenone obtained in Preparation Example 24 was used instead of 2-bromoacetophenone and triethylamine was used instead of potassium carbonate, and otherwise the procedures in Synthetic Step A were followed to obtain Compound PR-1559. The yield, % yield and NMR spectrum of Compound PR-1559 are as follows.
Yield: 0.52 g, % yield: 17.7 %
¹H-NMR (CDCl₃, ppm)
1.24 (s, 9H), 2.34 (s, 3H), 3.17 (dd, J=6.75Hz, 1.35Hz, 1H), 3.76 (s, 2H), 5.65 (dt, J=15.66Hz, 1.35Hz, 1H), 6.03 (dt, J=15.66Hz, 6.75Hz, 1H), 7.76 (d, J=7.02Hz, 2H), 8.10 (d, J=7.02Hz, 2H)

Further, in Synthetic Step B in Example 2, Compound PR-1559 was used instead of Compound PR-1130 and otherwise the procedures in Synthetic Step B were followed to obtain Compound PR-1560. The yield, % yield and NMR spectrum of Compound PR-1560 are as follows.
Yield: 0.21 g, % yield: 40.7 %
¹H-NMR (CDCl₃, ppm)
1.26 (s, 9H), 2.17 (s, 3H), 3.03 (dd, J=6.75Hz, 1.35Hz, 1H), 3.34 (s, 2H), 5.36 (s, 1H), 5.54 (s, 1H), 5.60 (dt, J=1 5.66Hz, 1.35Hz, 1H), 6.00 (dt, J=15.66Hz, 1.35Hz, 1H), 7.51 (s, 4H)

Further, in Synthetic Step C in Example 2, Compound PR-1560 was used instead of Compound PR-1257 and otherwise the procedures in Synthetic Step C were followed to obtain Compound PR-1561 as crystals. The yield, % yield, melting point, and NMR spectrum of Compound PR-1561 are as follows.
Yield: 0.23 g, % yield: 97.4%, melting point: 190 to 191.5°C
¹H-NMR (CDCl₃, ppm)
1.24 (s, 9H), 2.64 (d, J=4.32Hz, 3H), 3.45~3.75 (m, 2H), 3.97 (dd, J=14.04Hz, 5.40Hz, 1H), 4.18 (dd, J=14.04Hz, 3.78Hz, 1H), 5.75 (d, J=15.66Hz, 1H), 5.96 (s, 1H), 6.10 (s, 1H), 6.20 (dt, J=15.66Hz, 7.29Hz, 1H), 7.54 (d, J=8.37Hz, 2H), 7.72 (d, J=8.37Hz, 2H), 12.91 (broad, 1H)

### Example 21: Ethyl trans-4-{2-[N-(6,6-dimethyl-2-hepten-4-ynyl)-N-methylamino]acetyl}benzoate (Compound PR-1685), ethyl trans-4-{1-[N-(6,6-dimethyl-2-hepten-4-ynyl)-N-methylamino]methyl}vinylbenzoate (Compound PR-1686) and hydrochloride of Compound PR-1686; (Compound PR-1687)

In Synthetic Step A in Example 1, ethyl 4-(2-bromoacetyl)benzoate obtained in Example 25 was used instead of 2-bromoacetophenone and triethylamine was used instead of potassium carbonate, and otherwise the procedures in Synthetic Step A were followed to obtain Compound PR-1685. The yield, % yield and NMR spectrum of Compound PR-1685 are as follows.
Yield: 0.56 g, % yield: 11.8%
¹H-NMR (CDCl₃, ppm)
1.24 (s, 9H), 1.42 (t, J=7.02Hz, 3H), 2.36 (s, 3H), 3.21 (dd, J=6.75Hz, 1.35Hz, 2H), 3.82 (s, 2H), 4.41 (q, J=7.02Hz, 2H), 5.66 (dt, J=1 5.66Hz, 1.35Hz, 1H), 6.07 (dt, J=15.66Hz, 6.75Hz), 8.03 (d, J=8.91Hz, 2H), 8.12 (d, J=8.91Hz, 2H)

Further, in Synthetic Step B in Example 2, Compound PR-1685 was used instead of Compound PR-1130 and otherwise the procedures in Synthetic Step B were followed to obtain Compound PR-1686. The yield, % yield and NMR spectrum of Compound PR-1686 are as follows.
Yield: 0.14 g, % yield: 25.1 %
¹H-NMR (CDCl₃, ppm)
1.24 (s, 9H), 1.39 (t, J=7.02Hz, 3H), 2.18 (s, 3H), 3.04 (dd, J=6.48Hz, 1.62Hz, 2H), 3.35 (s, 2H), 3.37 (q, J=7.02Hz, 2H), 5.32 (s, 1H), 5.52 (s, 1H), 5.61 (dt, J=15.66Hz, 1.62Hz, 1H), 6.02 (dt, J=15.66Hz, 6.48Hz, 1H), 5.54 (d, J=8.37Hz, 2H), 7.99 (d, J=8.37Hz, 2H)

Further, in Synthetic Step C in Example 2, Compound PR-1686 was used instead of Compound PR-1257 and otherwise the procedures in Synthetic Step C were followed to obtain Compound PR-1687 as crystals. The yield, % yield, melting point, and NMR spectrum of Compound PR-1687 are as follows.
Yield: 92 mg, % yield: 59.3 %, melting point: 136.5 to 138°C
¹H-NMR (CDCl₃, ppm)
1.23 (s, 9H), 1.41 (t, J=7. 02Hz, 3H), 2.61 (d, J=4.86Hz, 3H), 3.49 (m, 1H), 3.67(m, 1H), 4.04 (dd, J=13.77Hz, 4.86Hz, 1H), 4.18 (dd, J=13.77Hz, 3.78Hz, 1H), 4.39 (q, J=7.02Hz, 2H), 5.70 (d, J=15.66Hz, 1H), 5.93 (s, 1H), 6.02 (s, 1H), 6.22 (dt, J=15.66Hz, 7.29Hz, 1H), 7.47 (d, J=8.37Hz, 2H), 8.09 (d, J=8.37Hz, 2H), 12.87 (broad, 1H)

### Example 22: Trans-2',4'-dichloro-2-[N-(6,6-dimethyl-2-hepten-4-ynyl)-N-methylamino]acetophenone (Compound PR-1517), trans-N-(6,6-dimethyl-2-hepten-4-ynyl)-N-methyl-[2-(2,4-dichlorophenyl)-2-propenyl]amine (Compound PR-1518) and hydrochloride of Compound PR-1518 (Compound PR-1519)

In Synthetic Step A in Example 1, 2-bromo-2',4'-dichloroacetophenone obtained in Example 26 was used instead of 2-bromoacetophenone and triethylamine was used instead of potassium carbonate, and otherwise the procedures in Synthetic Step A were followed to obtain Compound PR-1517. The yield, % yield and NMR spectrum of Compound PR-1517 are as follows.
Yield: 0.93 g, % yield: 27.8 %
¹H-NMR (CDCl₃, ppm)
1.24 (s, 9H), 2.34 (s, 3H), 3.16 (dd, J=6.75Hz, 1.35Hz, 2H), 3.72 (s, 2H), 5.60 (dt, J=15.93Hz, 1.35Hz, 1H), 5.98 (dt, J=15.93Hz, 6.75Hz, 1H), 7.31 (dd, J=8.37Hz, 1.62Hz, 1H), 7.43 (d, J=1.62Hz, 1H), 7.44 (d, J=8.37Hz, 1H)

Further, in Synthetic Step B in Example 2, Compound PR-1517 was used instead of Compound PR-1130 and otherwise the procedures in Synthetic Step B were followed to obtain Compound PR-1518. The yield, % yield and NMR spectrum of Compound PR-1518 are as follows.
Yield: 0.25 g, % yield: 27.0 %
¹H-NMR (CDCl₃, ppm)
1.24 (s, 9H), 2.20 (s, 3H), 3.02 (dd, J=6.48Hz, 1.62Hz, 2H), 3.28 (s, 2H), 5.16 (s,1H), 5.46 (s, 1H), 5.55 (dt, J=1 5. 66Hz, 1.62Hz, 1H), 5.95 (dt, J=15.66Hz, 6.48Hz, 1H), 7.13 (d, J=7.83Hz, 1H), 7.20 (dd, J=7.83Hz, 1.89Hz, 1H), 7.37 (d, J=1.89Hz, 1H)

Further, in Synthetic Step C in Example 2, Compound PR-1518 was used instead of Compound PR-1257 and otherwise the procedures in Synthetic Step C were followed to obtain Compound PR-1519 as crystals. The yield, % yield, melting point, and NMR spectrum of Compound PR-1519 are as follows.
Yield: 0.24 g, % yield: 86.5 %, melting point: 179 to 180°C
¹H-NMR (CDCl₃, ppm)
1.23 (s, 9H), 2.68 (d, J=4.59Hz, 3H), 3.45~3.75 (m, 2H), 3.94 (dd, J=14.31Hz, 4.86Hz, 1H), 4.08 (dd, J=14.31Hz, 3.51Hz, 1H), 5.67 (d, J=15.66Hz, 1H), 5.71 (s, 1H), 6.18 (s, 1H), 6.20 (dt, J=15.66Hz, 7.56Hz, 1H), 7.27 (d, J=8.10Hz, 1H), 7.33 (dd, J=8.10Hz, 1.89Hz, 1H), 7.46 (d, J=1.89Hz, 1H), 12.88 (broad, 1H)

### Example 23: Trans-3',4'-dichloro-2-[N-(6,6-dimethyl-2-h epten-4-ynyl)-N-methylamino]acetophenone (Compound PR-1510), trans-N-(6,6-dimethyl-2-hepten-4-ynyl)-N-methyl-[2-(3,4-dichlorophenyl)-2-propenyl]amine (Compound PR-1511) and hydrochloride of Compound PR-1511 (Compound PR-1512)

In Synthetic Step A in Example 1, 2-bromo-3',4'-dichloroacetophenone obtained in Preparation Example 27 was used instead of 2-bromoacetophenone and triethylamine was used instead of potassium carbonate, and otherwise the procedures in Synthetic Step A were followed to obtain Compound PR-1510. The yield, % yield and NMR spectrum of Compound PR-1510 are as follows.
Yield: 0.92 g, % yield: 27.2 %
¹H-NMR (CDCl₃, ppm)
1.23 (s, 9H), 2.33 (s, 3H), 3.18 (dd, J=6.75Hz, 1.62Hz, 1H), 3.72 (s, 2H), 5.66 (dt, J=15.66Hz, 1.62Hz, 1H), 6.05 (dt, J=15.66Hz, 6.75Hz, 1H), 7.52 (d, J=8.37Hz, 1H), 7.83 (dd, J=8.37Hz, 1.89Hz, 1H), 8.11 (d, J=1.89Hz, 1H)

Further, in Synthetic Step B in Example 2, Compound PR-1510 was used instead of Compound PR-1130 and otherwise the procedures in Synthetic Step B were followed to obtain Compound PR-1511. The yield, % yield and NMR spectrum of Compound PR-1511 are as follows.
Yield: 0.32 g, % yield: 35 %
¹H-NMR (CDCl₃, ppm)
1.24 (s, 9H), 2.17 (s, 3H), 3.03 (dd, J=6.75Hz, 1.62Hz, 1H), 3.28 (s, 2H), 2.62 (s, 1H), 5.45 (s, 1H), 5.62 (dt, J=15.66Hz, 1.62Hz, 1H), 6.02 (dt, J=15.66Hz, 6.75Hz, 1H), 7.32 (dd, J=8.37Hz, 1.89Hz, 1H), 7.38 (d, J=15.66Hz, 1H), 7.59 (d, J=1.89Hz, 1H)

Further, in Synthetic Step C in Example 2, Compound PR-1511 was used instead of Compound PR-1257 and otherwise the procedures in Synthetic Step C were followed to obtain Compound PR-1512 as crystals. The yield, % yield, melting point, and NMR spectrum and of Compound PR-1512 are as follows.
Yield: 0.29 g, % yield: 81.7 %, melting point: 202.5 to 205.5°C
¹H-NMR (CDCl₃, ppm)
1.24 (s, 9H), 2.64 (d, J=3.78Hz, 3H), 3.47~3.73 (m, 2H), 3.96 (m, 1H), 4.12 (m, 1H), 5.74 (d, J = 5.93Hz, 1H), 5.88 (s, 1H), 6.00 (s, 1H), 6.22 (dt, J=15.93Hz, 7.56Hz, 1H), 7.27 (dd, J=8.37Hz, 1.89Hz, 1H), 7.49 (d, J=1.89Hz, 1H), 7.50 (d, J=8.37Hz, 1H), 12.89 (broad, 1H)

### Example 24: Trans-2',4'-dimethyl-2-[N-(6,6-dimethyl-2-hepten-4-ynyl)-N-methylaminolacetophenone, (Compound PR-1710), trans-N-(6,6-dimethyl-2-hepten-4-ynyl)-N-methyl-[2-(2,4-dimethylphenyl)-2-propenyl]amine (Compound PR-1711) and hydrochloride of Compound PR-1711 (Compound PR-1712)

In Synthetic Step A in Example 1, 2-bromo-2',4'-dimethylacetophenone obtained in Preparation Example 11 was used instead of 2-bromoacetophenone and sodium carbonate was used instead of potassium carbonate, and otherwise the procedures in Synthetic Step A were followed to obtain Compound PR-1710. The yield, % yield and NMR spectrum of Compound PR-1710 are as follows.
Yield: 1.07 g, % yield: 57.1 %
¹H-NMR (CDCl₃, ppm)
1.24 (s, 9H), 2.35 (s, 3H×2), 2.48 (s, 3H), 3.18 (dd, J=6.89Hz, 0.95Hz, 2H), 3.68 (s, 2H), 5.63 (d, J=15.9Hz, 1H), 6.06 (dt, J=15.9Hz, 6.89Hz, 1H), 7.03~7.06 (m, 2H), 7.57 (d, J=8.64Hz, 1H)

Further, in Synthetic Step B in Example 2, Compound PR-1710 was used instead of Compound PR-1130 and otherwise the procedures in Synthetic Step B were followed to obtain Compound PR-1711. The yield, % yield and NMR spectrum of Compound PR-1711 are as follows.
Yield: 0.21 g, % yield: 19.7%.
¹H-NMR (CDCl₃, ppm)
1.24 (s, 9H), 2.23 (s, 3H), 2.27 (s, 3H), 2.30 (s, 3H), 3.04 (dd, J=6.48Hz, 1.08Hz, 2H), 3.16 (s, 2H), 5.02 (d, J=1.89Hz, 1H), 5.40 (d, J=1.89Hz, 1H), 5.58 (d, J=15.9Hz, 1H), 6.00 (dt, J=15.9Hz, 6.48Hz, 1H), 6.94~7.02 (m, 3H)

Further, in Synthetic Step C in Example 2, Compound PR-1711 was used instead of Compound PR-1257 and otherwise the procedures in Synthetic Step C were followed to obtain Compound PR-1712 as white crystals. The yield, % yield, melting point, NMR spectrum and IR spectrum of Compound PR-1712 are as follows.
Yield: 0.19 g, % yield: 80.5 %, melting point: 180 to 183°C
¹H-NMR (CDCl₃, ppm)
1.23 (s, 9H), 2.29 (s, 3H), 2.33 (s, 3H), 2.61 (d, J=4.86Hz, 3H), 3.39~3.52 (m, 2H), 3.93~3.94 (m, 2H), 5.47 (d, J=15.7Hz, 1H), 5.54 (s, 1H), 5.92 (s, 1H), 6.18 (dt, J=15.9Hz, 7.29Hz, 1H), 7.03~7.06 (m, 3H), 12.8 (broad s, 1H)
IR (KBrtablet, cm⁻¹) :
3457, 2968, 2950, 2924. 2868. 2698, 263 8, 1460, 969, 819

### Example 25: Trans-3',4'-dimethyl-2-[N-(6,6-diethyl-2-hepten-4-ynyl)-N-methylamino]acetophenone (Compound PR-1703), trans-N-(6,6-dimethyl-2-hepten-4-ynyl)-N-methyl-[2-(3,4-dimethylphenyl)-2-propenyl]amine (Compound PR-1704) and hydrochloride of Compound PR-1704 (Compound PR-1705)

In Synthetic Step A in Example 1, 2-bromo-3',4'-dimethylacetophenone obtained in Preparation Example 10 was used instead of 2-bromoacetophenone and sodium carbonate was used instead of potassium carbonate, and otherwise the procedures in Synthetic Step A were followed to obtain Compound PR-1703. The yield, % yield and NMR spectrum of Compound PR-1703 are as follows.
Yield: 0.88 g, % yield: 46.4 %
¹H-NMR (CDCl₃, ppm)
1.23 (s, 9H), 2.31 (s, 3H×2), 2.38 (s, 3H), 3.23 (dd, J=6.62Hz, 1.49Hz, 2H), 3.80 (s, 2H), 5.66 (d, J=15.9Hz, 1H), 6.10 (dt, J=15.9Hz, 6.62Hz, 1H), 7.20 (d, J=7.29Hz, 1H), 7.70~7.74(m, 2H)

Further, in Synthetic Step B in Example 2, Compound PR-1703 was used instead of Compound PR-1103 and otherwise the procedures in Synthetic Step B were followed to obtain Compound PR-1704. The yield, % yield and NMR spectrum of Compound PR-1704 are as follows.
Yield: 0.57 g, % yield: 66.1 %
¹H-NMR (CDCl₃, ppm)
1.24 (s, 9H), 2.19 (s, 3H), 2.25 (s, 3H), 2.27 (s, 3H), 3.04 (dd, J=6.48Hz, 1.62Hz, 2H), 3.31 (s, 2H), 5.17 (d, J=1.35Hz, 1H), 5.38 (d, J=1.35Hz, 1H), 5.64 (dt, J=15.9Hz, 1.49Hz, 1H), 6.06 (dt, J=15.9Hz, 6.48Hz, 1H), 7.08 (d, J=7.83Hz, 1H), 7.20~7.38 (m, 2H)

Further, in Synthetic Step C in Example 2, Compound PR-1704 was used instead of Compound PR-1257 and otherwise the procedures in Synthetic Step C were followed to obtain Compound PR-1705 as white crystals. The yield, % yield, melting point, NMR spectrum and IR spectrum of Compound PR-1705 are as follows.
Yield: 0.52 g, % yield: 81.2 %, melting point: 155 to 156°C
¹H-NMR (CDCl₃, ppm)
1.23 (s, 9H), 2.28 (s, 3H), 2.29 (s, 3H), 2.57 (d, J=3.51Hz, 3H), 3.41~3.6 7 (broad m, 2H), 4.06 (broad s, 2H), 5.63 (d, J = 15.4Hz, 1H), 5.73 (s, 1H), 5.76 (s, 1H), 6.23 (dt, J=15.4Hz, 2.30Hz, 1H), 7.08~7.26 (m, 3H), 12.7 (broad s, 1H)
IR (KBrtablet, cm⁻¹) :
2969, 2931, 2696, 2645, 2623, 1462, 139 7, 965, 928

### Example 26: Trans-3',4'-difluoro-2-[N-(6,6-dimethyl-2-hepten-4-ynyl)-N-methylamino]acetophenone (Compound PR-2171), trans-N-(6,6-dimethyl-2-hepten-4-ynyl)-N-methyl-[2-(3,4-difluorophenyl)-2-propenyl]amine (Compound PR-2172) and hydrochloride of Compound PR-2172 (Compound PR-2173)

In Synthetic Step A in Example 1, 2-bromo-3',4'-difluoroacetophenone obtained in Preparation Example 18 was used instead of 2-bromoacetophenone and otherwise the procedures in Synthetic Step A were followed to obtain Compound PR-2171. The yield, % yield and NMR spectrum of Compound PR-2171 are as follows.
Yield: 0.42 g, % yield: 27.3 %
¹H-NMR (CDCl₃, ppm)
1.24 (s, 9H), 2.33 (s, 3H), 3.17 (dd, J=1.62Hz, 6.75Hz, 2H), 3.71 (s, 2H), 5.65 (d, J=15.7Hz, 1H), 6.05 (dt, J=15.7Hz, 6.75Hz, 1H), 7.23 (m, 1H), 7.77~7.93 (m, 2H)

Further, in Synthetic Step B in Example 2, Compound PR-2171 was used instead of Compound PR-1130 and otherwise the procedures in Synthetic Step B were followed to obtain Compound PR-2172. The yield, % yield and NMR spectrum of Compound PR-2172 are as follows.
Yield: 0.18 g, % yield: 43.0%
¹H-NMR (CDCl₃, ppm)
1.24 (s, 9H), 2.17 (s, 3H), 3.03 (dd, J=6.75Hz, 1.35Hz, 2H), 3.28 (s, 2H), 5.23 (s, 1H), 5.41 (s, 1H), 5.61 (d, J=15. 9Hz, 1H), 6.02 (dt, J=15.9Hz, 6.75Hz, 1H), 7.09 (m, 1H), 7.19~7.39 (m, 2H)

Further, in Synthetic Step C in Example 2, Compound PR-2172 was used instead of Compound PR-1257 and otherwise the procedures in Synthetic Step C were followed to obtain Compound PR-2173 as white crystals. The yield, % yield, melting point, NMR spectrum and IR spectrum of Compound PR-2173 are as follows.
Yield: 0.18 g, % yield: 89.4 %, melting point: 197 to 199°C
¹H-NMR (CDCl₃, ppm)
1.24 (s, 9H), 2.63 (d, 3H, J=4.32Hz), 3.51~3.70 (m, 2H), 3.89~4.14 (m, 2H), 5.74 (d, J=15.4Hz, 1H), 5.83 (s, 1H), 5.94(s, 1H), 6.21 (dt, J=15.4Hz, 7.56Hz, 1H), 7.13~7.27 (m, 3H), 12.9 (broad s, 1H) IR (KBrtablet, cm⁻¹) :
2974, 2695, 2639, 1519, 1398, 1271

### Example 27 : Trans-3',5'-difluoro-2-[N-6,6-dimethyl-2-hepten-4-ynyl)-N-methylamino]acetophenone (Compound PR-2157), trans-N-(6,6-dimethyl-2-hepten-4-ynyl)-N-methyl-[2-(3,5-difluorophenyl)-2-propenyl]amine (Compound PR-2158) and hydrochloride of Compound PR-2158 (Compound PR-2159)

In Synthetic Step A in Example 1, 2-bromo-3',5'-difluoroacetophenone obtained in Preparation Example 17 was used instead of 2-bromoacetophenone and otherwise the procedures in Synthetic Step A were followed to obtain Compound PR-2157. The yield, % yield and NMR spectrum of Compound PR-2157 are as follows.
Yield: 0.59 g, % yield: 30.7 %
¹H-NMR (CDCl₃, p pm)
1.25 (s, 9H), 2.35 (s, 3H), 3.18 (dd, J=6.75Hz, 1.35Hz, 2H), 3.72 (s, 2H), 5.65 (dd, J=15.7Hz, 1.35Hz, 1H), 6.05 (dt, J=15.7Hz, 6.75Hz, 1H), 7.02 (m, 1H), 7.26~7.58 (m, 2H)

Further, in Synthetic Step B in Example 2, Compound PR-2157 was used instead of Compound PR-1130 and otherwise the procedures in Synthetic Step B were followed to obtain Compound PR-2158. The yield, % yield and NMR spectrum of Compound PR-2158 are as follows.
Yield: 0.14 g, % yield: 23.9 %
¹H-NMR (CDCl₃, ppm)
1.24 (s, 9H), 2.18 (s, 3H) , 3.04 (dd, J=6.48Hz, 1.35Hz, 2H) , 3.27 (s, 2H) , 5.29 (s, 1H), 5.48 (d, J=0.81Hz, 1H), 5.61 (d t, J=15.7Hz, 1.35Hz, 1H), 6.03 (dt, J=15.7Hz, 6.48Hz, 1H), 6.70 (m, 1H), 6.99~7.2 2 (m, 2H)

Further, in Synthetic Step C in Example 2, Compound PR-2158 was used instead of Compound PR-1257 and otherwise the procedures in Synthetic Step C were followed to obtain Compound PR-2159 as white crystals. The yield, % yield, melting point, NMR spectrum and IR spectrum of Compound PR-2159 are as follows.
Yield: 0.10 g, % yield: 63.8 %, melting point: 182 to 184°C
¹H-NMR (CDCl₃, p pm)
1.24 (s, 9H), 2.64 (d, 3H, J=4.86Hz), 3.51~3.66 (m, 2H), 3.71~4.13 (m, 2H), 5.75 (d, J=15.1Hz, 1H), 5.92 (s, 1H), 6.08 (d, J=2.97Hz, 1H), 6.22 (dt, J=15.1Hz, 7.56Hz, 1H) 6.80~7.24 (m, 4H), 13.0 (broad s, 1H)
IR (KBrtablet, cm⁻¹) :
3450, 2974, 2935, 1622, 1589, 1398, 133 6, 1 1 21

### Example 28: Trans-4'-tert-butyl-2-[N-(6,6-dimethyl-2-hepten-4-ynyl)-N-methylamino]acetophenone (Compound PR-1717), trans-N-(6,6-dimethyl-2-hepten-4-ynyl)-N-methyl-[2-(4-tert-butylphenyl)-2-propenyl]amine (Compound PR-1718) and hydrochloride of Compound PR-1718 (Compound PR-1719)

In Synthetic Step A in Example 1, 2-bromo-4'-tert-butylacetophenone obtained in Preparation Example 12 was used instead of 2-bromoacetophenone and sodium carbonate was used instead of potassium carbonate, and otherwise the procedures in Synthetic Step A were followed to obtain Compound PR-1717. The yield, % yield and NMR spectrum of Compound PR-1717 are as follows.
Yield: 0.96 g, % yield: 39.7 %
¹H-NMR (CDCl₃, ppm)
1.23 (s, 9H), 1.34 (s, 9H), 2.36 (s, 3H), 3.21 (d, J=5.40Hz, 2H), 3.80 (s, 2H), 5.65 (d, J=15.4Hz, 1H), 6.08 (dt, J=15.4Hz, 5.40Hz, 1H), 7.47 (d, J=8.37Hz, 2H), 7.93 (d, J=8.37Hz, 2H)

Further, in Synthetic Step B in Example 2, Compound PR-1717 was used instead of Compound PR-1130 and otherwise the procedures in Synthetic Step B were followed to obtain Compound PR-1718. The yield, % yield and NMR spectrum Compound PR-1718 are as follows.
Yield: 0.42 g, % yield: 43.7%
¹H-NMR (CDCl₃, ppm)
1.24 (s, 9H), 1.33 (s, 9H), 2.20 (s, 3H), 3.05 (d, J=7.02Hz, 2H), 3.33 (s, 2H), 5.19 (s, 1H), 5.43 (s, 1H), 5.61 (dd, J=15.8Hz, 1.49Hz, 1H), 6.06 (dt, J=15.8Hz, 7.02Hz, 1H), 7.34 (d, J=6.48Hz, 2H), 7.44 (d, J=6.48Hz, 2H)

Further, in Synthetic Step C in Example 2, Compound PR-1718 was used instead of Compound PR-1257 and otherwise the procedures in Synthetic Step C were followed to obtain Compound PR-1719 as white crystals. The yield, % yield, melting point, NMR spectrum and IR spectrum of Compound PR-1719 are as follows.
Yield: 0.38 g, % yield: 81.4%, melting point: 157 to 160°C
¹H-NMR (CDCl₃, ppm)
1.23 (s, 9H), 1.33 (s, 9H), 2.59 (d, J=4.86Hz, 3H), 3.38~3.65 (m, 2H), 4.00~4.15 (m, 2H), 5.59 (d, J=15.9Hz, 1H), 5.81 (s, 1H×2), 6.23 (dt, J=15.9Hz, 7.56Hz, 1H), 7.30 (d, J=8.91Hz, 2H), 7.42 (d, J=8.91Hz, 2H), 12.4 (broad s, 1H)
IR (KBrtablet, cm⁻¹) :
3437, 3427, 2966, 2933, 2906, 2868, 262 5, 2604, 2574, 1463, 1363

### Example 29: Trans-2-[N-(6,6-dimethyl-2-hepten-4-ynyl)-N-methylamino]-2'-hydroxyacetophenone (Compound PR-1619) and hydrochloride of Compound PR-1619 (Compound PR-1620)

In Example 1, 2-bromo-2'-hydroxyacetophenone obtained in Preparation Example 16 was used instead of 2-bromoacetophenone and sodium carbonate was used instead of potassium carbonate, and otherwise the procedures in Example 1 were followed to obtain Compounds PR-1619 and PR-1620. Compound PR-1620 was obtained as pale orange crystals.
The yield, % yield and NMR spectrum of Compounds PR-1619 are as follows.
Yield: 0.73 g, % yield: 25.9 %
¹H-NMR (CDCl₃, ppm)
1.22 (s, 9H), 3.05 (s, 3H), 4.08 (d, J=7.29Hz, 2H), 4.81 (s, 2H), 5.92 (d, J=15.9Hz, 1H), 6.25 (dt, J=15.4Hz, 7.56Hz, 1H), 6.93 (t, J=7.70Hz, 1H), 7.09 (d, J=8.37Hz, 1H), 7.51 (t, J=7.70Hz, 1H), 7.68 (d, J=8.37Hz, 1H)

The yield, % yield, melting point, NMR spectrum and IR spectrum of Compound PR-1620 are as follows. Yield: 0.62 g, % yield: 75.2 %, melting point: 75 to 130°C
¹H-NMR (CDCl₃, ppm)
1.21 (s, 9H), 3.05 (s, 3H), 4.06 (d, J=7.02Hz, 2H), 4.66 (s, 2H), 5.88 (d, J=15.7Hz, 1H), 6.29 (dt, J=15.9Hz, 7.83Hz, 1H), 6.97 (dt, J=7.29Hz, 1.08Hz, 1H), 7.48 (dd, J=8.51Hz, 1.08Hz, 1H), 7.55~7.65 (m, 2H), 11.3 (s, 1H), 13.2 (s, 1H)
IR (KBrtablet, cm⁻¹)
3430, 2970, 2932, 1648, 1617, 1604, 1458, 1363, 1291

### Example 30 : Trans-4'-tert-butyldimethylsilyloxy-2-[N-(6,6-dimethyl-2-hepten-4-ynyl)-N-methylamino]acetophenone (Compound PR-1604), trans-N-(6,6-dimethyl-2-hepten-4-ynyl)-N-methyl-[2-(4-tert-butyldimethylsilyloxyphenyl)-2-propenyl]amine (Compound PR-1605), and trans-N-(6,6-dimethyl-2-hepten-4-ynyl)-N-methyl-[2-(4-hydroxyphenyl)-2-propenyl]amine (Compound PR-1606)

In Synthetic Step A in Example 1, 2-bromo-4'-tert-butyldimethylsilyloxyacetophenone obtained in Preparation Examples 14 and 15 was used instead of 2-bromoacetophenone and otherwise the procedures in Synthetic Step A were followed to obtain Compound PR-1604. The yield, % yield and NMR spectrum of Compound PR-1604 are as follows.
Yield: 1.22 g, % yield: 21.7 %
NMR spectrum (CD₃CN) :
0.00 (s, 3H×2), 0.73 (s, 9H), 0.97 (s, 9H), 2.02 (s, 3H), 2.90 (dd, J=6.48Hz, 1.35Hz, 2H), 3.48 (s, 2H), 5.40 (dd, J=1 5.7Hz, 1.08Hz, 1H), 5.70~5.81 (m, 1H), 6.69 (d, J=9.99Hz, 2H), 7.68 (d, J=9. 99Hz, 2H)

Further, in Synthetic Step B in Example 2, Compound PR-1604 was used instead of Compound PR-1130 and otherwise the procedures in Synthetic Step B were followed to obtain Compound PR-1605. The yield, % yield and NMR spectrum of Compound PR-1605 are as follows.
Yield: 0.61 g, % yield: 61.2 %
¹H-NMR (CDCl₃, ppm)
0.00 (s, 3H×2), 0.80 (s, 9H), 1.04 (s, 9H), 1.99 (s, 3H), 2.83 (dd, J=6.48Hz, 1.62Hz, 2H), 3.09 (s, 2H), 4.93 (d, J=1.08 Hz, 1H), 5.16 (d, J=1.89Hz, 1H), 5.81 (dd, J=16.6Hz, 1.35Hz, 1H), 5.84 (dt, J=16.6Hz, 6.48Hz, 1H), 6.58 (d, J=8.91Hz, 2H), 7.17 (d, J=8.91Hz, 2H)

0.61 g (1.53 mmol) of Compound PR-1605 and 1.45 g (4.60 mmol) of tetrabutylammonium fluoride trihydrate were dissolved in 10 ml of THF, and the mixture was stirred at room temperature for 18 hours. The reaction mixture was poured into ice water and extracted with 100 ml of diethyl ether. The organic extract was washed with water and then with saturated saline and dried with anhydrous sodium sulfate.

The solvent was distilled off under reduced pressure and the residue was purified by silica gel column chromatography (developing solvent: hexane : ethyl acetate = 5 : 1) to obtain Compound PR-1606 as pale yellow crystals. The yield, % yield, melting point, NMR spectrum and IR spectrum of Compound PR-1606 are as follows.
Yield: 0.32 g, % yield: 73.8 %, melting point: 105.5 to 108°C
¹H-NMR (CDCl₃, ppm)
1.24 (s, 9H), 2.20 (s, 3H), 3.08 (d, J=6.48Hz, 2H), 3.33 (s, 2H), 5.13 (s, 1H), 5.32 (d, J=1.35Hz, 1H), 5.63 (d, J=15.9Hz, 1H), 6.06 (dt, J=8.91Hz, 1.62Hz , 1H), 6.62 (d, J=8.64Hz, 2H), 7.29 (d, J=8.64Hz, 2H)
IR (KBrtablet, cm⁻¹) :
3081, 3036, 2968, 2926, 2906, 2868, 2844, 2790, 2746, 2662, 2599, 1609, 1513, 1458, 1373, 1314, 1271, 1243, 997, 961, 869, 830

### Example 31: Trans-N-(6,6-dimethyl-2-hepten-4-ynyl)-N-methyl-[2-(2-aminophenyl)-2-propenyl]amine (Compound PR-1672)

0.43 g (1.38 mmol) of trans-N-(6,6-dimethyl-2-hepten-4-ynyl)-N-methyl-[2-(2-nitrophenyl)-2-propenyl] amine (Compound PR-1640) obtained in Example 16 and 0.27 g (4.13 mmol) of zinc powder were mixed in the solution containing 19 ml of acetic acid and 1 ml of Millipore water, and the solution was heated at 80°C for 3 hours. After the solvent was distilled off under reduced pressure, the residue was neutralized with a saturated aqueous sodium hydrogen carbonate solution and extracted with 100 ml of diethyl ether. The organic extract was washed with a saturated aqueous sodium hydrogen carbonate solution and with saturated saline and dried with anhydrous sodium sulfate.

The solvent was distilled off under reduced pressure and the residue was purified by silica gel column chromatography (hexane : ethyl acetate = 3 : 1) to obtain Compound PR-1672. The yield, % yield and NMR spectrum of Compound PR-1672 are as follows.
Yield: 0.14 g, % yield: 35.9 %
¹H-NMR (CDCl₃, ppm)
1.24 (s, 9H), 2.27 (s, 3H), 3.10 (dd, J=1. 35Hz, 6.48Hz, 2H), 3.18 (s, 2H), 5.30 (d, J=1.89Hz, 1H), 5.38 (d, J=1.89Hz, 1H), 5.61 (d, J=15.7Hz, 1H), 5.53 (dt, J=15.7Hz, 6.48Hz, 1H), 6.64~6.71 (m, 2H), 7.01~7.09 (m, 2H) IR (KBrtablet, cm⁻¹)
3444, 3381, 3216, 3199, 3152, 2969, 292 9, 2790, 1616, 1494, 1452, 914, 742

### Example 32: Trans-N-(6,6-dimethyl-2-hepten-4-ynyl)-N-methyl-[2-(3-aminochenyl)-2-propenyl]amine (Compound PR-1676) and hydrochloride of Compound PR-1676 (Compound PR-1725)

In Example 31, Compound PR-1646 obtained in Example 17 was used instead of compound PR-1640 and otherwise the procedures in Example 31 were followed to obtain compound PR-1676. The yield, % yield and NMR spectrum of Compound PR-1676 are as follows.
Yield: 0 08 g, % yield: 18.0 %
¹H-NMR (CDCl₃, ppm)
1.24 (s, 9H), 2.17 (s, 3H), 3.04 (dd, J=6.75Hz, 1.35Hz, 2H), 3.29 (s, 2H), 3. 65 (broad s, 2H), 5.19 (d, J=1.62Hz, 1H), 5.40 (d, J=1. 35Hz, 1H), 5.62 (d, J= 15.9Hz, 1H), 6.06 (dt, J=15.9Hz, 6.48Hz, 1H), 6.61 (m, 1H), 6.82 (t, J=1.89Hz, 1H), 6,88 (dd, J=6.08Hz, 1.08Hz, 1H), 7.11 (t, J=7.56Hz, 1H)

Further, in Synthetic Step C in Example 2, Compound PR-1676 was used instead of Compound PR-1257 and otherwise the procedures, in Synthetic Step C were followed to obtain Compound PR-1725 as brown crystals. The yield, % yield, melting point, NMR spectrum and IR spectrum of Compound PR-1725 are as follows.
Yield: 0.0467 g, % yield: 51.7 %, melting point: 150 to 192°C
¹H-NMR (CDCl₃, ppm)
1.23 (s, 9H), 2.66 (s, 3H), 3.72 (broad s, 2H), 4.20~4.29 (m, 2H), 5.72 (s, 1H), 5.79 (s, 1H), 5.85 (d, 1H, J=15.9Hz), 6.06 (dt, 1H, J=15.9Hz, 7.29Hz), 7.38~7.40 (broad m, 2H), 7.58~7.59 (broad m, 1H), 7.85 (broad s, 1H)
IR (KBr tablet, cm⁻¹)
3421, 2969, 2937, 2903, 2868, 2732, 2707, 2625, 2602, 1460

### Example 33: N-cinnamyl-N-methyl-2-Rhenyl-2-propenylamine (Compound PR-1806) and hydrochloride of Compound PR-1806: (Compound PR-1807)

0.66 g (6.55 mmol) of triethylamine was dropped in 20 ml of a 40% methylamine-methanol solution.

After dropping, the mixture was stirred at room temperature for 20 hours and excess methylamine and methanol were removed under reduced pressure. The residue was extracted with diethyl ether/2N hydrochloric acid (100 ml/100 ml) and the water extract after neutralization with an aqueous sodium hydroxide solution was extracted with 100 ml of chloroform.

The organic extract was washed with a saturated aqueous sodium hydrogen carbonate solution and with saturated saline. After the organic extract was dried with sodium sulfate, the solvent was distilled off under reduced pressure to obtain N-cinnamylmethylamine as yellow oily substance.
The NMR spectrum of N-cinnamylmethylamine is as follows.
Yield: 0.80 g, % yield: 83.0 %
¹H-NMR (CDCl₃, ppm)
2.48 (s, 3H), 3.38 (dd, J=6.21Hz, 1.22Hz, 2H), 6.29 (dt, J=16.5Hz, 6.21Hz, 1H), 6.54 (d, J=16.5Hz, 1H), 7.19~7.40 (m, 5H)

In Synthetic Step A in Example 1, N-cinnamylmethylamine obtained above was used instead of Compound PR-1133, sodium carbonate was used instead of potassium carbonate, and α-bromomethylstyrene obtained in Preparation Example 19 was used instead of 2-bromoacetophenone, and otherwise the procedures in Synthetic Step A were followed to obtain Compound PR-1806. The yield, % yield and NMR spectrum of Compound PR-1806 are as follows.
Yield: 0.34 g, % yield: 66.5 %
¹H-NMR (CDCl₃, ppm)
2.25 (s, 3H), 1.69 (dd, J=9.45Hz, 6.35Hz, 2H), 3.40 (s, 2H), 5.26 (d, J=1.35Hz, 1H), 5.45 (d, J=1.62Hz, 1H), 6.27 (dt, J=15.7Hz, 6.48Hz, 1H), 6.51 (d, J=15.7Hz, 1H), 7.19~7.39 (m, 8H), 7.47~7.51 (m, 2H)

Further, in Synthetic Step C in Example 2, Compound PR-1806 was used instead of Compound PR-1257 and otherwise the procedures in Synthetic Step C were followed to obtain Compound PR-1807 as white crystals. The yield, % yield, melting point, NMR spectrum and IR spectrum of Compound PR-1807 are as follows.
Yield: 0.31 g, % yield: 80.1 %, melting point: 137 to 139°C
¹H-NMR (CDCl₃, ppm)
2.64 (d, J=7.56Hz, 3H), 3.47~3.77 (m, 2H), 4.13~4.16 (m, 2H), 5.87 (d, J=9.99Hz, 2H), 6.39~6.48 (m, 1H×2), 7.32~7.42 (m, 10H)
IR (KBr tablet, cm⁻¹)
2969, 2889, 2822, 2751, 2709, 2673, 2643, 2596, 1497, 1465, 1449, 1414, 977, 967, 944, 783, 743, 722

### Example 34: Trans-N-(6,6-dimethyl-2-hepten-4-ynyl)-N-ethyl-2-phenyl-2-propenylamine (Compound PR-1853) and hydrochloride of Compound PR-1853 (Compound PR-1854)

N-ethyl-2-phenyl-2-propenylamine obtained in Preparation Example 20 was dissolved in 6 ml of DMF and 390 mg of sodium carbonate was added thereto. While stirring the mixture under ice cooling, a solution of 740 mg of 1-bromo-6,6-dimethylhepten-4-yn (*trans* : *cis* = 3 : 1) in 2 m of DMF was dropped. After the temperature was elevated to room temperature, the mixture was stirred for 68 hours. DMF was concentrated, and the residue was extracted with 100 ml of ether after the water was added. The ether extract was washed with water and dried with magnesium sulfate, and the solvent was distilled off under reduced pressure. The residue was purified by silica gel chromatography (SiO₂ 20 g, hexane : ethyl acetate = 30 : 1) to obtain Compound PR-1853.
Yield: 0.35g, % yield: 34.0%
¹H-NMR (CDCl₃, ppm)
1.01 (t, J=7.02Hz, 3H), 1.24 (s, 9H), 2.53 (q, J=7.02Hz, 2H), 3.11 (dd, J=6.48Hz, 1.62Hz, 2H), 3.40 (s, 2H), 5.27 (s, 1H), 5.42 (s, 1H), 5.60 (dt, J=1 5.93Hz, 1.62Hz, 1H), 6.03 (dt, J=15.93Hz, 6.48Hz, 1H), 7.25~7.38 (m, 3H), 7.49 (m, 2H)

Further, in Synthetic Step C in Example 2, Compound PR-1853 was used instead of Compound PR-1257 and otherwise the procedures in Synthetic Step C were followed to obtain Compound PR-1854 as crystals. 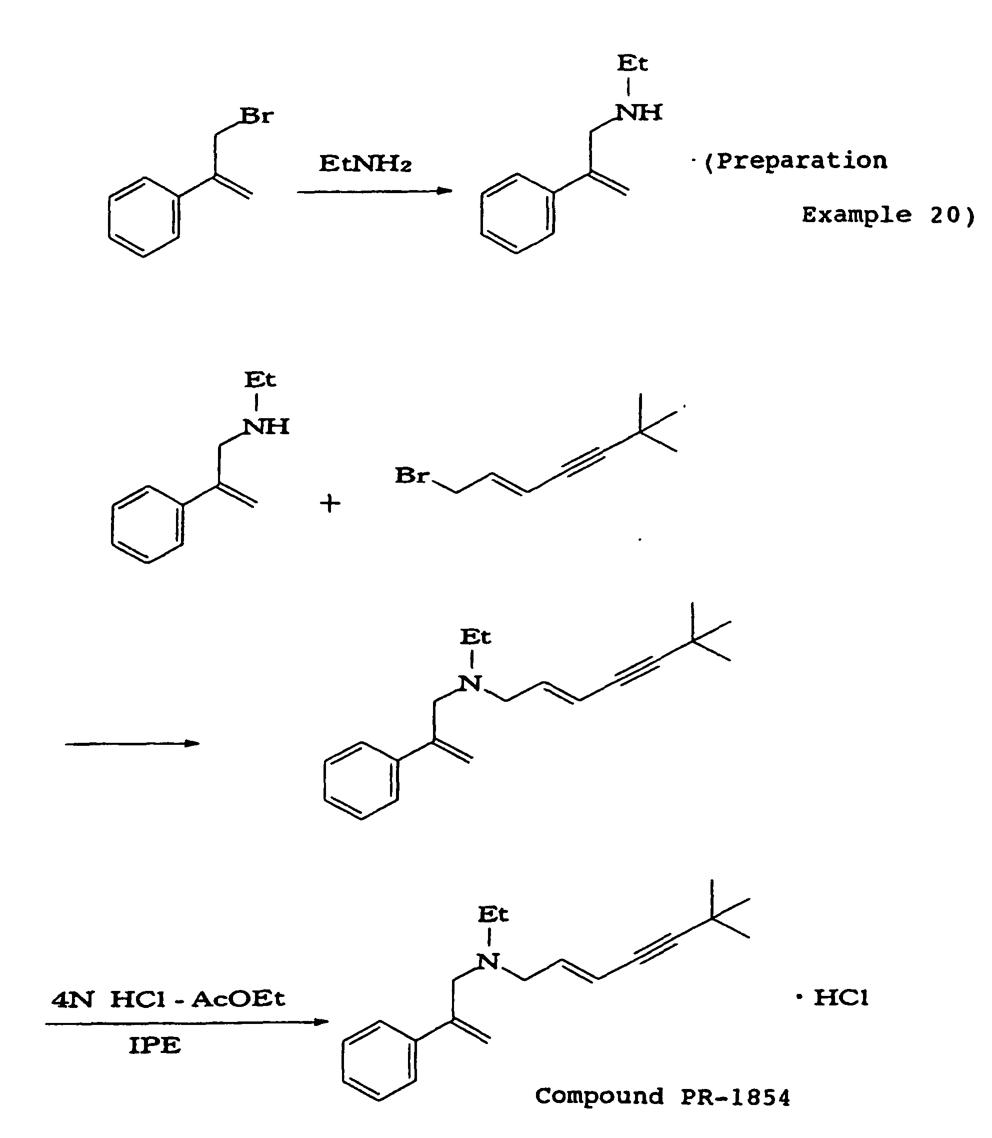 (Et is the abbreviation of an ethyl group)

The yield, % yield, melting point and NMR spectrum of Compound PR-1854 are as follows.
Yield: 0.34 g, % yield: 86.0 %, melting point: 130.5 to 133°C
¹H-NMR (CDCl₃, ppm)
1.23 (s, 9H), 1.39 (t, J=7.29Hz, 3H), 2.98 (m, 2H), 3.55 (m, 2H), 4.08 (m, 2H), 5.61 (d, J=15.66Hz, 1H), 5.81 (d, J=1.35Hz, 1H), 5.93 (d, J=2.97Hz, 1H), 6.24 (dt, J=15.66Hz, 7.56Hz, 1H), 7.32~7.48 (m, 5H), 12.56 (broad, 1H)

### Example 35: Trans-N-(6,6-dimethyl-2-hepten-4-ynyl)-N-isopropyl-2-phenyl-2-propenylamine (Compound PR-1855) and hydrochloride of Compound PR-1855 (Compound PR-1856)

In Example 34, N-isopropyl-2-phenyl-2-propenylamine obtained in Example 28 was used instead of N-ethyl-2-phenyl-2-propenylamine, and otherwise the procedures in Example 34 were followed to obtain Compound PR-1855, and its hydrochloride, Compound PR-1856.
The yield, % yield and NMR spectrum of Compound PR-1855 are as follows.
Yield: 0.48 g, % yield: 47.5 %
¹H-NMR (CDCl₃, ppm)
0.97 (d, J=7.02Hz, 6H), 1.23 (s, 9H), 3.01 (7 doublet, J=7.02Hz, 1H), 3.09 (dd, J=6.48Hz, 1.62Hz, 2H), 3.37 (s, 2H), 5.32 (s, 1H), 5.38 (s, 1H), 5.59 (dt, J=15.66Hz, 1.62Hz, 1H), 5.97 (dt, J=15.66Hz, 6.48Hz, 1H), 7.24~7.38 (m, 3H), 7.45 (m, 2H)

The yield, % yield, melting point and NMR spectrum of Compound PR-1856 are as follows.
Yield: 0.49 g, % yield: 90.8 %, melting point: 157.5 to 159.5°C
¹H-NMR (CDCl₃, ppm)
δ 1.23 (s, 9H), 1.39 (d, J=7.02Hz, 3H), 1.42 (d, J=7. 02Hz, 3H), 3.42~3.78 (m, 3H), 3.90~4.10 (m, 2H), 5.64 (dd, J=15.66Hz, 1.35Hz, 1H), 5.86 (d, J=1.62Hz, 1H), 6.17 (d, J=2.97Hz, 1H), 6.43 (dt, J=15.66Hz, 7.56Hz, 1H), 7.30~7.47 (m, 5H), 12.35 (broad, 1H)

### Example 36: Trans-N-cyclopropyl-N-(6,6-dimethyl-2-hepten-4-ynyl)-2-phenyl-2-propenylamine (Compound PR-1930) and hydrochloride of Compound PR-1930 (Compound PR-1931)

In Example 34, N-cyclopropyl-2-phenyl-2-propenylamine obtained in Preparation Example 29 was used instead of N-ethyl-2-phenyl-2-propenylamine and otherwise the procedures in Example 34 were followed to obtain Compound PR-1930 and its hydrochloride, Compound PR-1931.
The yield, % yield and NMR spectrum of compound PR-1930 are as follows.
Yield: 0.77 g, % yield: 45.5 %
¹H-NMR (CDCl₃, ppm)
0.30~0.44 (m, 4H), 1.25 (s, 9H), 1.88 (m, 1H), 3.19 (dd, J=7.02Hz, 1.62Hz, 2H), 3.61 (s, 2H), 5.22 (s, 1H), 5.39 (s, 1H), 5.55 (dt, J=15.66Hz, 1.62Hz, 1H), 6.09 (dt, J=15.66Hz, 7.02Hz, 1H), 7.22~7.87 (m, 3H), 7.44 (m, 2H)

The yield, % yield, melting point and NMR spectrum of Compound PR-1931 are as follows.
Yield: 0.71 g, % yield: 82.0 %, melting point: 155 to 156.5°C(decomp)
¹H-NMR (C DC l₃, ppm)
0.53 (m, 1H), 0.80 (m, 1H), 1.23 (s, 9H), 1.45~1.65 (m, 2H), 2.07 (m, 1H), 3.51~3.77 (m, 2H) , 4.08~4.30 (m, 2H), 5.58 (d, J=15.66Hz, 1H), 5.75 (s, 1H), 5.79 (s, 1H), 6.27 (d t, J=15.66Hz, 7.56Hz, 1H), 7.40 (s, 5H), 12.44 (broad, 1H)

### Examples 37, 38: Haft of Toothbrush

According to the formulations shown in Table 3, small balls of polystyrene and Compound PR-1258 or PR-1540 were mixed and melt molded to produce a haft of toothbrush.

**Table 3**

| (Formulation) | | |
|---|---|---|
| Ingredient | Compounding Amount (part by weight) | |
| | Example 37 | Example 38 |
| Small balls of polystyrene | 99 | 99 |
| Compound PR-1258 | 1 | - |
| Compound PR-1540 | - | 1 |

### Examples 39, 40; Ointment for athlete's foot (tinea pedis)

According to the formulations shown in Table 4, the respective formulation ingredients were weighed and mixed in a kneader to produce an ointment for athlete's food (*tinea* pedis).

**Table 4**

| (Formulation) | | |
|---|---|---|
| Ingredient | Compounding Amount (part by weight) | |
| | Example 39 | Example 40 |
| Vaseline | 99 | - |
| Water-absorbing ointment | - | 99 |
| Compound PP-1484 | 1 | - |
| Compound PR-1418 | - | 1 |

### Examples 41, 42: Liquid Preparation

The formulation ingredients shown in Table 5 were stirred and solubilized to obtain a liquid preparation.

**Table 5**

| (Formulation) | | |
|---|---|---|
| Ingredient | Compounding Amount (part by weight) | |
| | Example 41 | Example 42 |
| Ethanol | 92 | 92 |
| Alkyl methacrylate copolymer | 2 | 2 |
| Propylene glycol | 5 | 5 |
| Compound PR-1554 | 1 | - |
| Compound PR-2159 | - | 1 |

### <Test Example>

The novel amine derivatives or salts thereof obtained in the examples described above in accordance with the present invention were evaluated for their antimycotic activity by test examples as follows.

### Test Example 1: Measurement of Antimycotic Activity (Measurement of Minimum Inhibitory Concentration (MIC))

The antimycotic activity of the compounds of the invention against dermatophyte was examined.

More particularly, the test dermatophyte strains shown in Table 6 below were cultivated in advance on a slant culture of Sabouraud's agar medium (manufactured by Nissui Seiyaku Co., Ltd.; 1.0% peptone, 4.0% glucose, 1.5% agar, pH=5.9) at 27°C for 2 weeks to allow them to grow conidia sufficiently. Then, sterilized physiological saline containing 0.05 weight/volume % of Tween 80 was added to the culture and while rubbing the surface of the culture with a platinum loop, the conidia were separated and caused to float. Using a hemocytometer, the filtrate was adjusted with sterilized physiological saline such that the concentration of conidia was 10⁶ cells/ml, which was used as a test microbe liquid.

On the other hand, compounds (test drugs) of the present invention obtained in the examples described above were taken each in an amount of 10 mg, and 1 ml of dimethyl sulfoxide was added thereto to form a stock solution, of which 500 µl was taken and 500 µl of dimethyl sulfoxide was added thereto to prepare a 2-fold dilution. The same operation was repeated to prepare 13-stages ranging from 10 to 0.0025 mg/ml (final concentration of test system 100 to 0.025 µg/ml) of diluted drug solutions. Solutions with various diluted concentrations of test drug were each dispensed in sterilized petri dish with an amount of 100µl. Then, 10 ml of sterile-dissolved Sabouraud's agar medium (1.0% peptone, 4.0% glucose, 1.5% agar, pH=5.9) was added, well mixed and solidified. Then, 5 µl of the test microbe solution previously adjusted were added using a microplanter, respectively.

The cultivation was carried out at 27°C for 1 week and the minimum drug concentration (µg/ml) which clearly inhibits visible growth was regarded as an MIC value. The results are shown in Table 6.

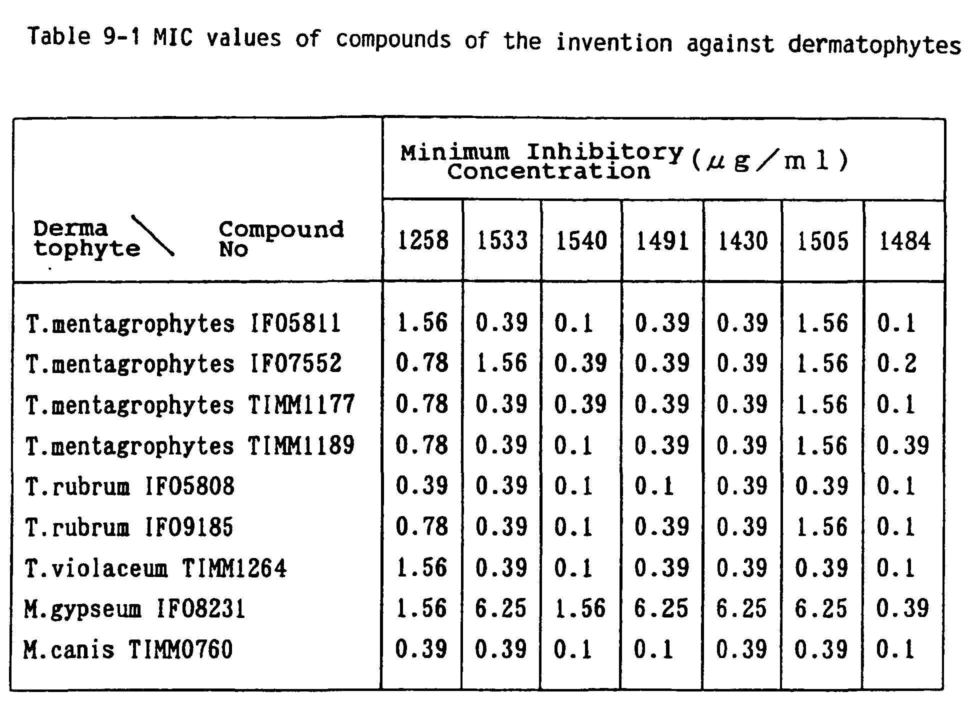

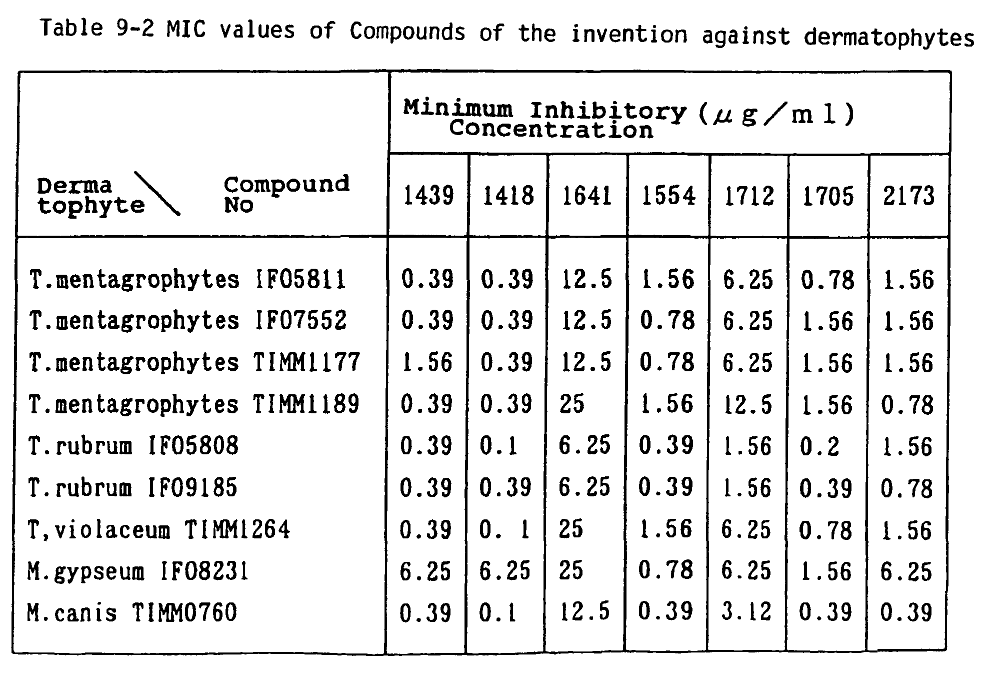

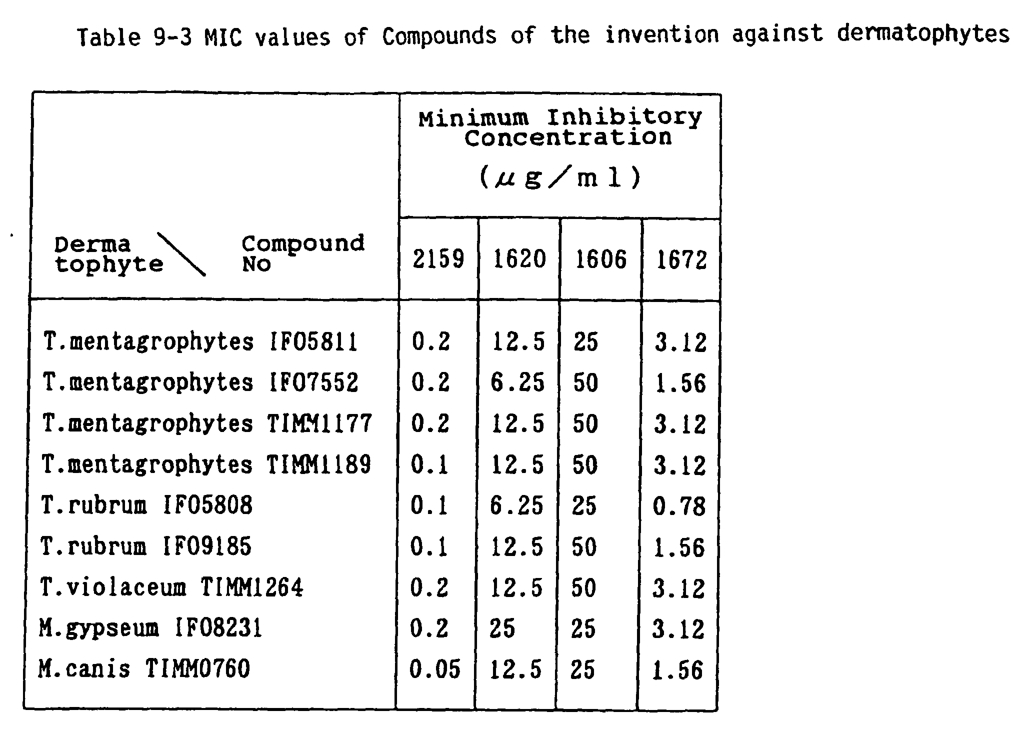

The amine derivative represented by general formula (1) and salts thereof of the present invention have an excellent antimycotic effect and are very useful as an antimycotic agent, an antimycotic composition, a pharmaceutical composition and so forth.

## Claims

1. Amine derivatives represented by general formula (I) below or salts thereof: 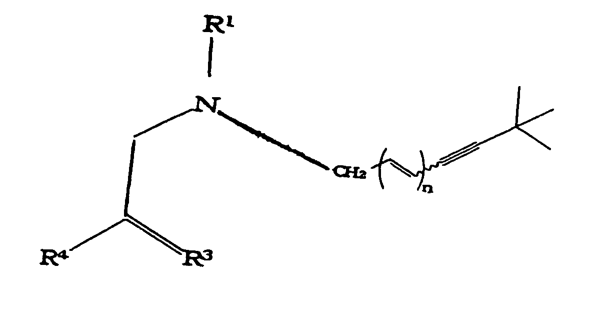 [wherein in the formula (1) above, R¹ represents a linear C₁₋₅ alkyl group, a branched alkyl group having up to 5 carbon atoms, or a cyclic alkyl group having up to 5 carbon atoms, said alkyl group may be halogenated; and " 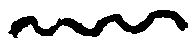 " indicates that the arrangement of double bond may be either *cis* or *trans,* and n is an integer of 1 to 3,
R³ is an oxygen atom or a group represented by the formula (f); 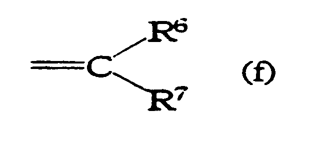 wherein each of R⁶ and R⁷ independently represents a hydrogen atom, a linear C₁₋₄ alkyl group, a branched alkyl group having up to 4 carbon atoms or a cyclic alkyl group having up to 4 carbon atoms; R⁴ represents a group of the formula (g), (h), or (i), 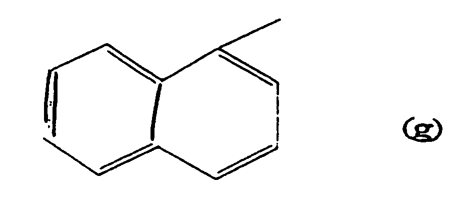 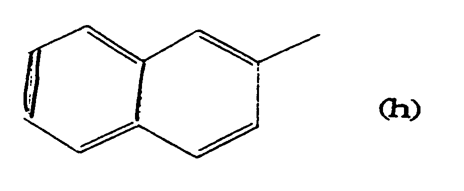 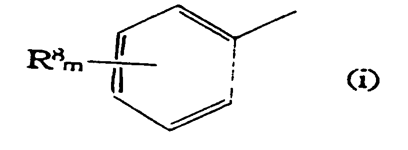 wherein in the formula (i), R⁸ is a substituent which is a linear C₁₋₇ alkyl group, a branched alkyl group having up to 7 carbon atoms, a cyclic alkyl group having up to 7 carbon atoms, a halogen atom, a linear C₁₋₄ alkoxy group, a branched alkoxy group having up to 4 carbon atoms, a cyclic alkoxy group having up to 4 carbon atoms, a nitro group, an amino group, a cyano group, a carboxyl group, a linear C₂₋₅ alkoxycarbonyl group, a branched alkoxycarbonyl group having up to 5 carbon atoms, a cyclic alkoxycarbonyl group having up to 5 carbon atoms, a hydroxyl group, or a group represented by R⁹₃SiO- where R⁹ is a linear C₁₋₄ alkyl group, a branched alkyl group having up to 4 carbon atoms or a cyclic alkyl group having up to 4 carbon atoms, in which the three R⁹ groups may be the same or different; and m is an integer from 0 to 5.

2. Amine derivatives or salts thereof as claimed in claim 1, wherein R¹ is a linear C₁₋₅ alkyl group, a branched alkyl group having up to 5 carbon atoms or a cyclic alkyl group having up to 5 carbon atoms, and R⁴ is a group represented by the formula (i).

3. Amine derivatives or salts thereof as claimed in claim 2, wherein R¹ is a methyl group, an ethyl group, an isopropyl group, or a cyclopropyl group, R¹ is an oxygen atom, a carbon atom to which two hydrogen atoms are attached, and R⁸ is a methyl group, a tert-butyl group, a fluorine atom, a chlorine atom, a bromine atom, a methoxy group, a nitro group, an amino group, a cyano group, an ethoxycarbonyl group, a hydroxyl group, or a tert-butyldimethylsilyl group.

4. Amine derivatives represented by general formula (1) or salts thereof as claimed in claim 2 or 3, which is selected from the following compounds:
Trans-2-[N-(6,6-dimethyl-2-hepten-4-ynyl)-N-methylamino]acetophenone;
Trans-N-(6,6-dimethyl-2-hepten-4-ynyl)-N-methyl-2-phenyl-2-propenylamine;
Trans-2-[N-(6,6-dimethyl-2-hepten-4-ynyl)-N-methylamino]-2'-methylacetophenone;
Trans-N-(6,6-dimethyl-2-hepten-4-ynyl)-N-methyl-[2-(o-tolyl)-2-propenyl]amine;
Trans-2-[N-(6,6-dimethyl-2-hepten-4-ynyl)-N-methylamino]-3'-methylacetophenone;
Trans-N-(6,6-dimethyl-2-hepten-4-ynyl)-N-methyl-[2-(m-tolyl)-2-propenyl]amine;
Trans-2-[N-(6,6-dimethyl-2-hepten-4-ynyl)-N-methylamino]-4'-methylacetophenone;
Trans-N-(6,6-dimethyl-2-hepten-4-ynyl)-N-methyl-[2-(p-tolyl)-2-propenyl]amine;
Trans-2-[N-(6,6-dimethyl-2-hepten-4-ynyl)-N-methylamino]-2'-fluoroacetophenone;
Trans-N-(6,6-dimethyl-2-hepten-4-ynyl)-N-methyl-[2-(2-fluorophenyl)-2-propenyl]amine;
Trans-2-[N-(6,6-dimethyl-2-hepten-4-ynyl)-N-methylamino]-3'-fluoroacetophenone;
Trans-N-(6,6-dimethyl-2-hepten-4-ynyl)-N-methyl-[2-(3-fluorophenyl)-2-propenyl]amine;
Trans-2-[N-(6,6-dimethyl-2-hepten-4-ynyl)-N-methylamino]-4'-fluoroacetophenone;
Trans-N-(6,6-dimethyl-2-hepten-4-ynyl)-N-methyl-[2-(4-fluorophenyl)-2-propenyl]amine;
Trans-2'-bromo-2-[N-(6,6-dimethyl-2-hepten-4-ynyl)-N-methylamino]acetophenone;
Trans-N-(6,6-dimethyl-2-hepten-4-ynyl)-N-methyl-[2-(2-bromophenyl)-2-propenyl]amine;
Trans-3'-bromo-2-[N-(6,6-dimethyl-2-hepten-4-ynyl)-N-methylamino]acetophenone;
Trans-N-(6,6-dimethyl-2-hepten-4-ynyl)-N-methyl-[2-(3-bromophenyl)-2-propenyl]amine;
Trans-4'-bromo-2-[N-(6,6-dimethyl-2-hepten-4-ynyl)-N-methylamino]acetophenone;
Trans-N-(6,6-dimethyl-2-hepten-4-ynyl)-N-methyl-[2-(4-bromophenyl)-2-propenyl]amine;
Trans-2'-chloro-2-[N-(6,6-dimethyl-2-hepten-4-ynyl)-N-methylamino]acetophenone;
Trans-N-(6,6-dimethyl-2-hepten-4-ynyl)-N-methyl-[2-(2-chlorophenyl)-2-propenyl]amine;
Trans-4'-chloro-2-[N-(6,6-dimethyl-2-hepten-4-ynyl)-N-methylamino]acetophenone;
Trans-N-(6,6-dimethyl-2-hepten-4-ynyl)-N-methyl-[2-(4-chlorophenyl)-2-propenyl]amine;
Trans-2-[N-(6,6-dimethyl-2-hepten-4-ynyl)-N-methylamino]-2'-methoxyacetophenone;
Trans-N-(6,6-dimethyl-2-hepten-4-ynyl)-N-methyl-[2-(2-methoxyphenyl)-2-propenyl]amine;
Trans-2-[N-(6,6-dimethyl-2-hepten-4-ynyl)-N-methylamino]-3'-methoxyacetophenone;
Trans-N-(6,6-dimethyl-2-hepten-4-ynyl)-N-methyl-[2-(3-methoxyphenyl)-2-propenyl]amine;
Trans-2-[N-(6,6-dimethyl-2-hepten-4-ynyl)-N-methylamine]-2'-nitroacetophenone;
Trans-N-(6,6-dimethyl-2-hepten-4-ynyl)-N-methyl-[2-(2-nitrophenyl)-2-propenyl]amine;
Trans-2-[N-(6,6-dimethyl-2-hepten-4-ynyl)-N-methylamine]-3'-nitroacetophenone;
Trans-N-(6,6-dimethyl-2-hepten-4-ynyl)-N-methyl-[2-(3-nitrophenyl)-2-propenyl]amine;
Trans-2-[N-(6,6-dimethyl-2-hepten-4-ynyl)-N-methylamine]-4'-nitroacetophenone;
Trans-N-(6,6-dimethyl-2-hepten-4-ynyl)-N-methyl-[2-(4-nitrophenyl)-2-propenyl]amine;
Trans-3'-cyano-2-[N-(6,6-dimethyl-2-hepten-4-ynyl)-N-methylamino]acetophenone;
Trans-3-{1-[N-(6,6-dimethyl-2-hepten-4-ynyl)-N-me thylamino]methyl}vinylbenzonitrile;
Trans-4'-cyano-2-[N-(6,6-dimethyl-2-hepten-4-ynyl)-N-methylamino]acetophenone;
Trans-4-{1-[N-(6,6-dimethyl-2-hepten-4-ynyl)-N-me thylamino]methyl}vinylbenzonitrile;
Ethyl trans-4-{2-[N-(6,6-dimethyl-2-hepten-4-ynyl)-N-methylamino]acetyl}benzoate;
Ethyl trans-4-{1-[N-(6,6-dimethyl-2-hepten-4-ynyl)-N-methylamino]methyl}vinylbenzoate;
Trans-2',4'-dichloro-2-[N-(6,6-dimethyl-2-hepten-4-ynyl)-N-methylamino]acetophenone;
Trans-N-(6,6-dimethyl-2-hepten-4-ynyl)-N-methyl-[2-(2,4-dichlorophenyl)-2-propenyl]amine;
Trans-3',4'-dichloro-2-[N-(6,6-dimethyl-2-hepten-4-ynyl)-N-methylamino]acetophenone;
Trans-N-(6,6-dimethyl-2-hepten-4-ynyl)-N-methyl-[2-(3,4-dichlorophenyl)-2-propenyl]amine;
Trans-2',4'-dimethyl-2-[N-(6,6-dimethyl-2-hepten-4-ynyl)-N-methylamino]acetophenone;
Trans-N-(6,6-dimethyl-2-hepten-4-ynyl)-N-methyl-[2-(2,4-dimethylphenyl)-2-propenyl]amine;
Trans-3',4'-dimethyl-2-[N-(6,6-dimethyl-2-hepten-4-ynyl)-N-methylamino]acetophenone;
Trans-N-(6,6-dimethyl-2-hepten-4-ynyl)-N-methyl-[2-(3,4-dimethylphenyl)-2-propenyl]amine;
Trans-3',4'-difluoro-2-[N-(6,6-dimethyl-2-hepten-4-ynyl)-N-methylamino]acetophenone;
Trans-N-(6,6-dimethyl-2-hepten-4-ynyl)-N-methyl-[2-(3,4-difluorophenyl)-2-propenyl]amine;
Trans-3',5'-difluoro-2-[N-(6,6-dimethyl-2-hepten-4-ynyl)-N-methylamino]acetophenone;
Trans-N-(6,6-dimethyl-2-hepten-4-ynyl)-N-methyl-[2-(3,5-difluorophenyl)-2-propenyl]amine;
Trans-4'-tert-butyl-2-[N-(6,6-dimethyl-2-hepten-4-ynyl)-N-methylamino]acetophenone;
Trans-N-(6,6-dimethyl-2-hepten-4-ynyl)-N-methyl-[2-(4-tert-butylphenyl)-2-propenyl]amine;
Trans-2-[N-(6,6-dimethyl-2-hepten-4-ynyl)-N-methylamino]-2'-hydroxyacetophenone;
Trans-4'-tert-butyldimethylsilyloxy-2-[N-(6,6-dimethyl-2-hepten-4-ynyl)-N-methylamino]acetophenone;
Trans-N-(6,6-dimethyl-2-hepten-4-ynyl)-N-methyl-[2-(4-tert-butyldimethylsilyloxyphenyl)-2-propenyl]amine;
Trans-N-(6,6-dimethyl-2-hepten-4-ynyl)-N-methyl-[2-(4-hydroxyphenyl)-2-propenyl]amine;
Trans-N-(6,6-dimethyl-2-hepten-4-ynyl)-N-methyl-[2-(2-aminophenyl)-2-propenyl]amine;
Trans-N-(6,6-dimethyl-2-hepten-4-ynyl)-N-methyl-[2-(3-aminophenyl)-2-propenyl]amine;
Trans-N-(6,6-dimethyl-2-hepten-4-ynyl)-N-ethyl-2-phenyl-2-propenylamine;
Trans-N-(6,6-dimethyl-2-hepten-4-ynyl)-N-isopropyl-2-phenyl-2-propenylamine; and
Trans-N-cyclopropyl-N-(6,6-dimethyl-2-hepten-4-ynyl)-2-phenyl-2-propenylamine.

5. Hydrochloride salts of amine derivatives as claimed in any one of claims 1 to 4.

6. A method for producing amine derivatives or salts thereof according to claim 1 comprising condensing a compound represented by general formula (2) 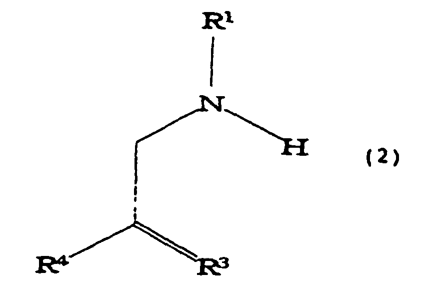 with a compound represented by general formula (3) 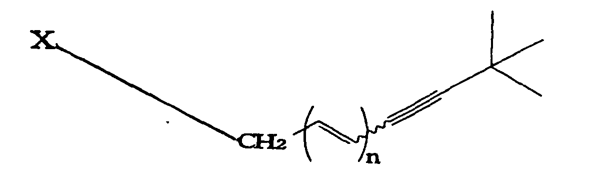 where X represents a halogen atom.

7. A method for producing amine derivatives or salts thereof according to claim 1 comprising condensing a compound represented by general formula (4) 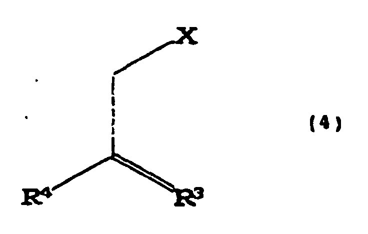 with a compound represented by general formula (5) 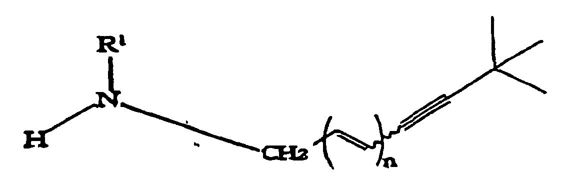 where X represent a halogen atom.

8. An antimycotic agent consisting of the amine derivatives or salts thereof as claimed in any one of claims 1 to 4.

9. An antimycotic composition comprising the amine derivatives or salts thereof as claimed in any one of claims 1 to 4.

10. A pharmaceutical composition comprising, as an active ingredient, the amine derivatives or salts thereof as claimed in any one of claims 1 to 4.

## Patentansprüche

1. Aminderivate die durch die folgende Formel (1) dargestellt werden, oder Salze davon 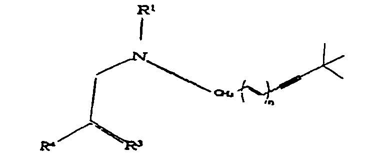 wobei in der oberen Formel (1) R¹ eine lineare C₁₋₅ Alkylgruppe, eine verzweige Alkylgruppe mit bis zu 5 Kohlenstoffatomen oder eine cyclische Alkylgruppe mit bis zu 5 Kohlenstoffatomen darstellt und die besagte Alkylgruppe halogeniert sein kann; und " 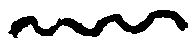 " anzeigt, dass die Anordnung der Doppelbindung sowohl *cis* als auch *trans* sein kann, und n eine ganze Zahl zwischen 1 und 3 ist
R³ ist ein Sauerstoffatom oder eine durch die Formel (f) dargestellte Gruppe, 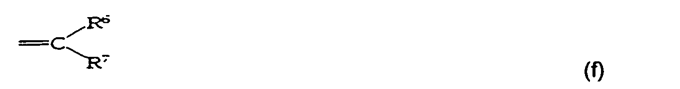 In der R⁶ and R⁷ unabhängig voneinander jeweils ein Wasserstoffatom, eine lineare C₁₋₄-Gruppe, eine verzweige Alkylgruppe mit bis zu 4 Kohlenstoffatomen oder eine cyclische Alkylgruppe mit bis zu 4 Kohlenstoffatomen darstellt, R⁴ stellt eine Gruppe mit der Formel (g), (h) oder (i) dar, 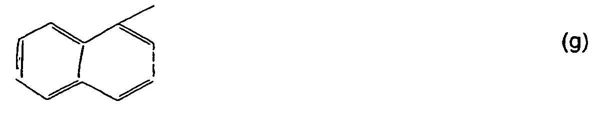 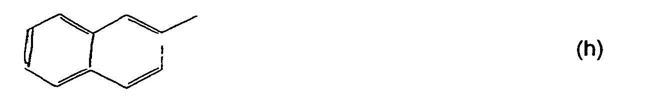 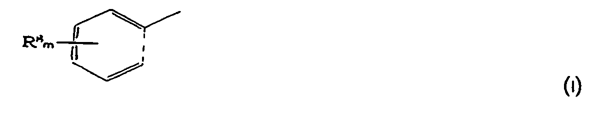 wobei in der Formel (i) R⁸ ein Substituent ist, der eine lineare C₁₋₇ Alkylgruppe, eine verzweige Alkylgruppe mit bis zu 7 Kohlenstoffatomen oder eine cyclische Alkylgruppe mit bis zu 7 Kohlenstoffatomen, ein Halogenatom,eine lineare C₁₋₄-Alkoxigruppe, eine verzweigte Alkokxiguppe mit bis zu 4 Kohlenstoffatomen, eine cyclische Alkoxigruppe mit bis zu 4 Kohlenstoffatomen, eine Nitrogruppe, eine Aminogruppe, eine Cyanogruppe, eine Carboxylgruppe, eine lineare C₂₋₅ Alkoxicarbonylguppe, eine verzweige Alkoxicarbonylguppe mit bis zu 5 Kohlenstoffatomen, eine cyclische Alkoxicarbonylguppe mit bis zu 5 Kohlenstoffatomen, eine Hydroxylgruppe oder eine durch R⁹₃SiO- dargestellte Gruppe ist, in der R⁹ eine lineare C₁₋₄ Gruppe, eine verzweige Alkylgruppe mit bis zu 4 Kohlenstoffatomen oder eine cyclische Alkylgruppe mit bis zu 4 Kohlenstoffatomen darstellt, in der die drei R⁹-Gruppen gleich oder unterschiedlich sein können; und m eine ganze Zahl zwischen 0 und 5 ist.

2. Aminderivate oder Salze davon wie in Anspruch 1 beansprucht, in denen R¹ eine lineare C₁₋₅-Alkylgruppe, eine verzweige Alkylgruppe mit bis zu 5 Kohlenstoffatomen oder eine cyclische Alkylgruppe mit bis zu 5 Kohlenstoffatomen darstellt, und R⁴ eine durch die Formel (i) dargestellte Gruppe darstellt.

3. Aminderivate oder Salze davon wie in Anspruch 2 beansprucht, in denen R¹ eine Methylgruppe, eine Ethylgruppe, eine Isopropylgruppe oder eine Cyclopropylgruppe ist und R³ ein Sauerstoffatom oder ein Kohlenstoffatom ist, an das zwei Wasserstoffatome gebunden sind, und R⁸ eine Methylgruppe, eine tert.-Butylgruppe, ein Fluoratom, ein Chloratom, ein Brumatom, eine Mathoxigruppe, eine Nitrogruppe, eine Aminogruppe, eine Cyanogruppe, eine Ethoxicarbonylgruppe, eine Hydroxylgruppe oder eine tert.-Butyldimethylsilygruppe darstellt.

4. Aminderivate, die durch die allgemeine Formel (1) dargestellt werden, oder Salze davon, wie in den Ansprüchen 2 oder 3 beansprucht, die aus den folgenden Verbindungen ausgewählt werden:
Trans-2-[N-(6,6-dimethyl-2-hepten-4-ynyl)-N-methylamino]acetophenon;
Trans-N-(6,6-dimethyl-2-hepten-4-ynyl)-N-methyl-2-phenyl-2-propenylamin;
Trans-2-[N-(6,6-dimethyl-2-hepten-4-ynyl)-N-methylamino]-2'-methylacetophenon;
Trans-N-(6,6-dimetyl-2-hepten-4-ynyl)-N-methyl-[2-(o-tolyl)-2-propenyl]amin;
Trans-2-[N-(6,6-dimethyl-2-hepten-4-ynyl)-N-methylamino]-3'-methylacetophenon;
Trans-N-(6,6-dimethyl-2-hepten-4-ynyl)-N-methyl-[2-(m-tolyl)-2-propenyl]amin;
Trans-2-[N-(6,6-dimethyl-2-hepten-4-ynyl)-N-methylamino]-4'-methylacetophenon
Trans-N-(6,6-dimethyl-2-hepten-4-ynyl)-N-methyl-[2-(p-tolyl)-2-propenyl]amin;
Trans-2-[N-(6,6-dimethyl-2-hepten-4-ynyl)-N-methylamino]-2'-fluoracetophenon;
Trans-N-(6,6-dimethyl-2-hepten-4-ynyl)-N-methyl-[2-(2-fluorphenyl)-2-propenyl]amin;
Trans-2-[N-(6,6-dimethyl-2-hepten-4-ynyl)-N-methylamino]-3'-fluoracetophenon;
Trans-N-(6,6-dimethyl-2-hepten-4-ynyl-N-methyl-[2-(3-fluorphenyl)-2-propenyl]amin;
Trans-2-[N-(6,6-dimethyl-2-hepten-4-ynyl)-N-methylamino]-4'-fluoracetophenon;
Trans-N-(6,6-dimethyl-2-hepten-4-ynyl)-N-methyl-[2-(4-fluorphenyl)-2-propenyl]amin;
Trans-2'-brom-2-[N-(6,6-dimethyl-2-hepten-4-ynyl)-N-methylamino]acetophenon;
Trans-N-(6,6-dimethyl-2-hepten-4-ynyl)-N-methyl-[2-(2-bromphenyl)-2-propenyl]amin;
Trans-3'-brom-2-[N-(6,6-dimethyl-2-hepten-4-ynyl)-N-methylamino]acetophenon;
Trans-N-(6,6-dimethyl-2-hepten-4-ynyl)-N-methyl-[2-(3-bromphenyl)-2-propenyl]amin;
Trans-4'-brom-2-[N-(6,6-dimethyl-2-hepten-4-ynyl)-N-methylamino]acetophenon;
Trans-N-(6,6-dimethyl-2-hepten-4-ynyl)-N-methyl-[2-(4-bromphenyl)-2-propenyl]amin;
Trans-2'-chlor-2-[N-(6,6-dimethyl-2-hepten-4-ynyl)-N-methylamino]acetophenon;
Trans-N-(6,6-dimethyl-2-hepten-4-ynyl)-N-methyl-[2-(2-chlorphenyl)-2-propenyl]amin;
Trans-4'-chloro-2-[N-(6,6-dimethyl-2-hepten-4-ynyl)-N-methylamino]acetophenon;
Trans-N-(6,6-dimethyl-2-hepten-4-ynyl)-N-methyl-[2-(4-chlorphenyl)-2-propenyl]amin;
Trans-2-[N-(6,6-dimethyl-2-hepten-4-ynyl)-N-methylamino]-2'-methoxiacetophenon;
Trans-N-(6,6-dimethyl-2-hepten-4-ynyl)-N-methyl-[2-(2-methoxiphenyl)-2-propenyl]amin;
Trans-2-[N-(6,6-dimethyl-2-hepten-4-ynyl)-N-methylamin]-3'-methoxiacetophenon;
Trans-N-(6,6-dimethyl-2-hepten-4-ynyl)-N-methyl-[2-(3-dimethoxiphenyl)-2-propenyl]amin;
Trans-2-[N-(6,6-dimethyl-2-hepten-4-ynyl)-N-methylamin]-2'-nitroacetophenon,
Trans-N-(6,6-dimethyl-2-hepten-4-ynyl)-N-methyl-[2-(2-nitrophenyl)-2-propenyl]amin;
Trans-2-[N-(6,6-dimethyl-2-hepten-4-ynyl)-N-methylamin]-3'-nitroacetophenon,
Trans-N-(6,6-dimethyl-2-hepten-4-ynyl)-N-methyl-[2-(3-nitrophenyl)-2-propenyl]amin;
Trans-2-[N-(6,6-dimethyl-2-hepten-4-ynyl)-N-methylamin]-4'-nitroacetophenon;
Trans-N-(6,6-dimethyl-2-hepten-4-ynyl)-N-methyl-[2-(4-nitrophenyl)-2-propenyl]amin,
Trans-3'-cyano-2-[N-(6,6-dimethyl-2-hepten-4-ynyl)-N-methylamino)acetophenon,
Trans-3-{I-[N-(6,6-dimethyl-2-hepten-4-ynyl)-N-methylamino]methyl}vinylbenzonitril;
Trans-4'-cyano-2-[N-(6,6-dimethyl-2-hepten-4-ynyl)-N-methylamino]acetophenon,
Trans-4-{I-[N-(6,6-dimethyl-2-hepten-4-ynyl)-N-methylamino]methyl}vinylbenzonitril,
Ethyl-trans-4-{2-[N-(6,6-dimethyl-2-hepten-4-ynyl)-N-methylamino]acetyl}benzoat,
Ethyl-trans-4-{I-[N-(6,6-dimethyl-2-hepten-4-ynyl)-N-methylamino]methyl}vinylbenzoat,
Trans-2',4'-dichloro-2-[N-(6,6-dimethyl-2-hepten-4-ynyl)-N-methylamino]acetophenon,
Trans-N-(6,6-dimethyl-2-hepten-4-ynyl)-N-methyl-[2-(2,4-dichlorphenyl)-2-propenyl]amin;
Trans-3',4'-dichlor-2-[N-(6,6-dimethyl-2-hepten-4-ynyl)-N-methylamino]acetophenon,
Trans-N-(6,6-dimethyl-2-hepten-4-ynyl)-N-methyl-[2-(3,4-dichlorphenyl)-2-propenyl]amin,
Trans-2',4'-dimethyl-2-[N-(6,6-dimethyl-2-hepten-4-ynyl)-N-methylamino]acetophenon,
Trans-N-(6,6-dimethyl-2-hepten-4-ynyl)-N-methyl-[2-(2,4-dimethylphenyl)-2-propenyl]amin;
Trans-3',4'-dimethyl-2-[N-(6,6-dimethyl-2-hepten-4-ynyl)-N-methylamino)acetophenon,
Trans-[N-(6,6-dimethyl-2-hepten-4-ynyl)-N-methyl-[2-(3,4-dimethylphenyl)-2-propenyl]amin,
Trans-3',4'-difluor-2-[N-(6,6-dimethyl-2-hepten-4-ynyl)-N-methylamino]acetophenon,
Trans-[N-(6,6-dimethyl-2-hepten-4-ynyl)-N-methyl-[2-(3,4-difluorphenyl)-2-propenyl]amin;
Trans-N-(6,6-dimethyl-2-hepten-4-ynyl)-N-methyl[2-(3,5-dilfuorphenyl)-2-propenyl]amin,
Trans-3',5'-difluor-2-[N-(6,6-dimethyl-2-hepten-4-ynyl)-N-methylamino]acetophenon
Trans-4'-tert -butyl-2-N-(6,6-dimethyl-2-hepten-4-ynyl)-N-methylamino]acetophenon,
Trans-N-(6,6-dimethyl-2-hepten-4-ynyl)-N-methyl-[2-(4-tert.-butylphenyl)-2-propenyl]amin,
Trans-2-[N-(6,6-dimethyl-2-hepten-4-ynyl)-N-methylamino-2'-hydroxiacetophenon,
Trans-4'-tert -butyldimethylsiloxi-2-[N-(6,6-dimethyl-2-hepten-4-ynyl)-N-methylamino]acetophenon,
Trans-N-(6,6-dimethyl-2-hepten-4-ynyl)-N-methyl-[2-(4-tert-butyldimethylsilyloxiphenyl)-2-propenyl]amin;
Trans-N-(6,6-dimethyl-2-hepten-4-ynyl)-N-methyl-[2-(4-hydroxiphenyl)-2-propenyl]amin,
Trans-N-(6,6-dimethyl-2-hepten-4-ynyl)-N-methyl-[2-(2-aminophenyl)-2-propenyl]amin;
Trans-N-(6,6-dimethyl-2-hepten-4-ynyl)-N-methyl-[2-(3-aminophenyl)-2-propenyl]amin,
Trans-N-(6,6-dimethyl-2-hepten-4-ynyl)-N-ethyl-2-phenyl-2-propenylamin,
Trans-N-(6,6-dimethyl-2-hepten-4-ynyl)-N-isopropyl-2-phenyl-2-propenylamin und
Trans-N-cyclopropyl-N-(6,6-dimethyl-2-hepten-4-ynyl)-2-phenyl-2-propenyl]amin.

5. Hydrochlondsalze von Aminderivaten, wie sie in irgendeinem der Ansprüche 1 bis 4 beansprucht werden.

6. Verfahren zur Herstellung von Amindenvaten oder ihren Salzen entsprechend Anspruch 1, das das Kondensieren einer durch die allgemeine Formel (2) dargestellten Verbindung 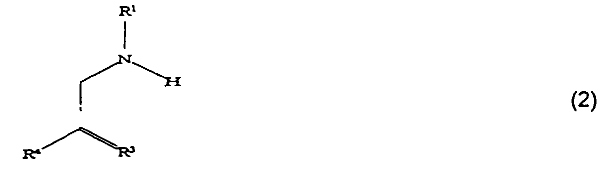 mit einer durch die allgemeine Formel (3) dargestellten Verbindung umfasst, 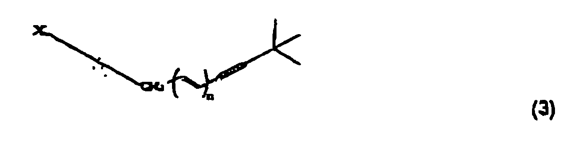 wobei X ein Halogenatom darstellt.

7. Verfahren zur Herstellung von Aminderivaten oder ihren Salzen entsprechend Anspruch 1, das das Kondensieren einer durch die allgemeine Formel (4) dargestellten Verbindung 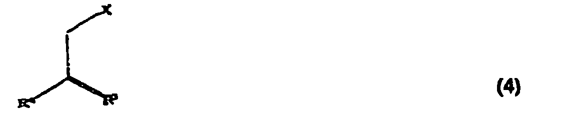 mit einer durch die allgemeine Formel (5) dargestellten Verbindung umfasst, 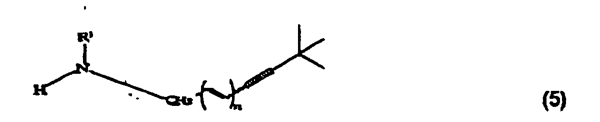 wobei X ein Halogenatom darstellt.

8. Antimycotischer Wirkstoff bestehend aus Aminderivativen oder ihren Salzen, wie sie in irgendeinem der Ansprüche 1 bis 4 beansprucht werden.

9. Antimycotische Zubereitung, der Aminderivate oder ihre Salze, wie sie in irgendeinem der Ansprüche 1 bis 4 beansprucht werden, umfasst.

10. Pharmazeulische Zubereitung, die als einen aktiven Bestandteil die Aminderivate oder ihre Salze, wie sie in irgendeinem der Ansprüche 1 bis 4 beansprucht werden, umfasst.

## Revendications

1. Dérivés d'amine représentés par la formule générale (I) ci-dessous ou des sels de ceux-ci : 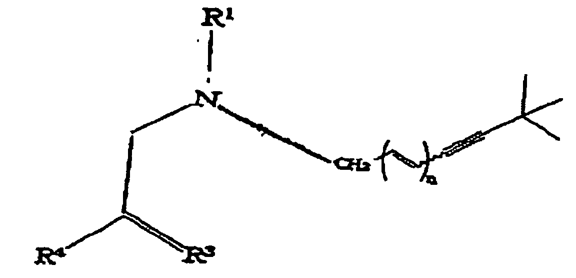 dans laquelle R¹ représente un groupe alkyle en C₁-C₅ linéaire, un groupe alkyle ramifié ayant jusqu'à 5 atomes de carbone, ou un groupe alkyle cyclique ayant jusqu'à 5 atomes de carbone, ledit groupe alkyle pouvant être halogéné ; et « 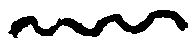 » indique que l'arrangement de la liaison double peut être soit cis soit trans, et n est un nombre entier de 1 à 3,
R³ est un atome d'oxygène ou un groupe représenté par la formule (f) ; 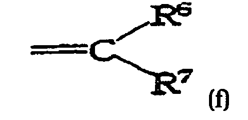 dans laquelle chacun de R⁶ et R⁷ représente indépendamment un atome d'hydrogène, un groupe alkyle en C₁-C₄ linéaire, un groupe alkyle ramifié ayant jusqu'à 4 atomes de carbone et un groupe alkyle cyclique ayant jusqu'à 4 atomes de carbone ; R⁴ représente un groupe de formule (g), (h), ou (i), 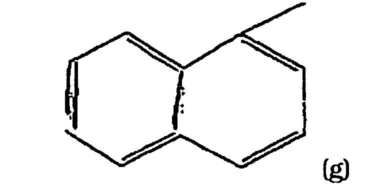 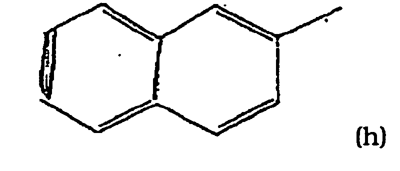 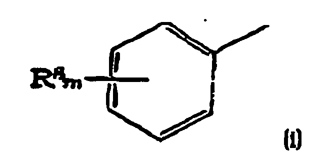 dans lesquelles R⁸ est, dans la formule (i), un substituant qui représente un groupe alkyle en C₁-C₇ linéaire, un groupe alkyle ramifié ayant jusqu'à 7 atomes de carbone, un groupe alkyle cyclique ayant jusqu'à 7 atomes de carbone, un atome d'halogène, un groupe alcoxy en C₁-C₄ linéaire, un groupe alcoxy ramifié ayant jusqu'à 4 atomes de carbone, un groupe alcoxy cyclique ayant jusqu'à 4 atomes de carbone, un groupe nitro, un groupe amino, un groupe cyano, un groupe carboxyle, un groupe alcoxycarbonyle en C₂-C₅ linéaire, un groupe alcoxycarbonyle ramifié jusqu'à 5 atomes de carbone, un groupe alcoxycarbonyle cyclique ayant jusqu'à 5 atomes de carbone, un groupe hydroxyle, ou un groupe représenté par la formule R⁹₃SiO-, dans laquelle R⁹ est un groupe alkyle en C₁-C₄ linéaire, un groupe alkyle ramifié ayant jusqu'à 4 atomes de carbone ou un groupe cyclique ayant jusqu'à 4 atomes de carbone, où les trois groupes R⁹ peuvent être identiques ou différents ; et m représente un nombre entier de 0 à 5.

2. Dérives d'amine ou sels de ceux-ci selon la revendication 1,
**caractérisés en ce que**
R¹ est un groupe alkyle en C₁₋C₅ linéaire, un groupe alkyle ramifié ayant jusqu'à 5 atomes de carbone ou un groupe alkyle cyclique ayant jusqu'à 5 atomes de carbone, et R⁴ est un groupe représenté par la formule (i).

3. Dérivés d'amine ou sels de ceux-ci selon la revendication 2,
**caractérisés en ce que**
R¹ est un groupe méthyle, un groupe éthyle, un groupe isopropyle, ou un groupe cyclopropyle, R³ est un atome d'oxygène ou un atome de carbone auquel deux atomes d'hydrogène sont attachés, et R⁸ est un groupe méthyle, un groupe tert-butyle, un atome de fluor, un atome de chlore, un atome de brome, un groupe méthoxy, un groupe nitro, un groupe amino, un groupe cyano, un groupe éthoxycarbonyle, un groupe hydroxyle, ou un groupe tert-butyldiméthylsilyle.

4. Dérivés d'amine représentés par la formule générale (1) ou des sels de celui-ci selon la revendication 2 ou 3, qui sont sélectionnés parmi les composés suivants :
- trans-2-[N-(6,6-diméthyl-2-heptèn-4-ynyl)-N-méthylamino]acétophénone ;
- trans-N-(6,6-diméthyl-2-heptèn-4-ynyl)-N-méthyl-2-phényl-2-propénylamine ;
- trans-2-[N-(6,6-diméthyl-2-heptèn-4-ynyl)-N-méthylamino]-2'-méthylacétophénone ;
- trans-N-(6,6-diméthyl-2-heptèn-4-ynyl)-N-méthyl-[2-(o-tolyl)-2-propényl]amine ;
- trans-2-[N-(6,6-diméthyl-2-heptèn-4-ynyl)-N-méthylamino]-3'-méthylacétophénone ;
- trans-N-(6,6-diméthyl-2-heptèn-4-ynyl)-N-méthyl-[2-(m-tolyl)-2-propényl]amine ;
- trans-2-[N-(6,6-diméthyl-2-heptèn-4-ynyl)-N-méthylamino]-4'-méthylacétophénone ;
- trans-N-(6,6-diméthyl-2-heptèn-4-ynyl)-N-méthyl-[2-(p-tolyl)-2-propényl]amine ;
- trans-2-[N-(6,6-diméthyl-2-heptèn-4-ynyl)-N-méthylamino]-2'-fluoroacétophénone ;
- trans-N-(6,6-diméthyl-2-heptèn-4-ynyl)-N-méthyl-[2-(2-fluorophényl)-2-propényl]amine ;
- trans-2-[N-(6,6-diméthyl-2-heptèn-4-ynyl)-N-méthylamino]-3'-fluoroacétophénone ;
- trans-N-(6,6-diméthyl-2-heptèn-4-ynyl)-N-méthyl-[2-(3-fluorophényl)-2-propényl]amine ;
- trans-2-[N-(6,6-diméthyl-2-heptèn-4-ynyl)-N-méthylamino]-4'-fluoroacétophénone ;
- trans-N-(6,6-diméthyl-2-heptèn-4-ynyl)-N-méthyl-[2-(4-fluorophényl)-2-propényl]amine ;
- trans-2'-bromo-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(2-bromophényl)-2-propényl]amine ;
- trans-3'-bromo-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(3-bromophényl)-2-propényl]amine ;
- trans-4'-bromo-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(4-bromophényl)-2-propényl]amine ;
- trans-2'-chloro-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(2-chlorophényl)-2-propényl]amine ;
- trans-4'-chloro-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(4-chlorophényl)-2-propényl]amine ;
- trans-2-[N-(6,6-diméthyl-2-heptèn-4-ynyl)-N-méthylamino]-2'-méthoxyacétophénone ;
- trans-N-(6,6-diméthyl-2-heptèn-4-ynyl)-N-méthyl-[2-(2-méthoxyphényl)-2-propényl]amine ;
- trans-2-[N-(6,6-diméthyl-2-heptèn-4-ynyl)-N-méthylamino]-3'-méthoxyacétophénone ;
- trans-N-(6,6-diméthyl-2-heptèn-4-ynyl)-N-méthyl-[2-(3-méthoxyphényl)-2-propényl]amine ;
- trans-2-[N-(6,6-diméthyl-2-heptèn-4-ynyl)-N-méthylamine]-2'-nitroacétophénone ;
- trans-N-(6,6-diméthyl-2-heptèn-4-ynyl)-N-méthyl-[2-(2-nitrophényl)-2-propényl]amine ;
- trans-2-[N-(6,6-diméthyl-2-heptèn-4-ynyl)-N-méthylamine]-3'-nitroacétophénone ;
- trans-N-(6,6-diméthyl-2-heptèn-4-ynyl)-N-méthyl-[2-(3-nitrophényl)-2-propényl]amine ;
- trans-2-[N-(6,6-diméthyl-2-heptèn-4-ynyl]-N-méthylamine]-4'-nitroacétophénone ;
- trans-N-(6,6-diméthyl-2-heptèn-4-ynyl)-N-méthyl-[2-(4-nitrophényl)-2-propényl]amine ;
- trans-3'-cyano-2-[N-(6,6-diméthyl-2-heptèn-4-ynyl)-N-méthylamino]acétophénone ;
- trans-3-{1-[N-(6,6-diméthyl-2-heptèn-4-ynyl)-N-méthylamino]méthyl}vinylbenzonitrile ;
- trans-4'-cyano-2-[N-(6,6-diméthyl-2-heptèn-4-ynyl)-N-méthylamino]acétophénone ;
- trans-4-{1-[N-(6,6-diméthyl-2-heptèn-4-ynyl)-N-méthylamino]méthyl}vinylbenzonitrile ;
- trans-4-{2-[N-(6,6-diméthyl-2-heptèn-4-ynyl)-N-méthylamino]acétyl}benzoate d'éthyle ;
- trans-4-{1-(N-(6,6-diméthyl-2-heptèn-4-ynyl)-N-méthylamino]méthyl}vinylbenzoate d'éthyle ;
- trans-2',4'-dichloro-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(2,4-dichlorophényl)-2-propényl]amine ;
- trans-3',4'-dichloro-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(3,4-dichlorophényl)-2-propényl]amine ;
- trans-2',4'-diméthyl-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(2,4-diméthylphényl)-2-propényl]amine ;
- trans-3',4'-diméthyl-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(3,4-diméthylphényl)-2-propényl]amme ;
- trans-3',4'-difluoro-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(3,4-difluorophényl)-2-propényl]amine ;
- trans-3',5'-difluoro-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(3,5-difluorophényl)-2-propényl]amine ;
- trans-4'-tert-butyl-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(4-tert-butylphényl)-2-propényl]amine ;
- trans-2-[N-(6,6-diméthy]-2-heptèn-4-ynyl)-N-méthylamino]-2'-hydroxyacétophénone ;
- trans-4'-tert-butyldiméthylsilyloxy-2-[N-(6,6-diméthyl-2-heptèn-4-ynyl)-N-méthylamino]acétophénone ;
- trans-N-(6,6-diméthyl-2-heptèn-4-ynyl)-N-méthyl-[2-(4-tert-butyldiméthylsilyloxyphényl)-2-propényl]amine ;
- trans-N-(6,6-diméthyl-2-heptèn-4-ynyl)-N-méthyl-[2-(4-hydroxyphényl)-2-propényl]amine ;
- trans-N-(6,6-diméthyl-2-heptèn-4-ynyl)-N-méthyl-[2-(2-aminophényl)-2-propényl]amine ;
- trans-N-(6,6-diméthyl-2-heptèn-4-ynyl)-N-méthyl-[2-(3-aminophényl)-2-propényl]amine ;
- trans-N-(6,6-diméthyl-2-heptèn-4-ynyl)-N-éthyl-2-phényl-2-propénylamine ;
- trans-N-(6,6-diméthyl-2-heptèn-4-ynyl)-N-isopropyl-2-phényl-2-propénylamine ; et
- trans-N-cyclopropyl-N-(6,6-diméthyl-2-heptèn-4-ynyl)-2-phényl-2-propénylamine.

5. Sels de chlorhydrate des dérivés d'amine selon l'une quelconque des revendications 1 à 4.

6. Procédé pour la production des dérivés d'amine ou des sels de ceux-ci selon la revendication 1, comprenant la condensation d'un composé représenté par la formule générale (2) 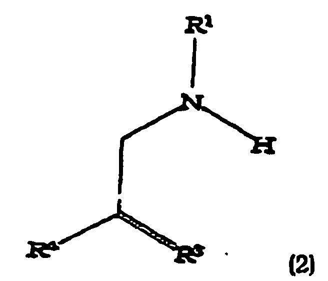 avec un composé représenté par la formule générale (3) 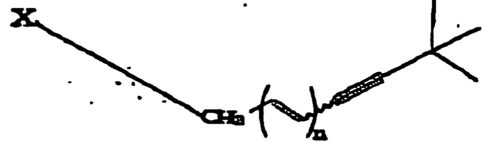 dans laquelle X représente un atome d'halogène.

7. Procédé pour la production des dérives d'amine ou des sels de ceux-ci selon la revendication 1, comprenant la condensation d'un composé représenté par la formule générale (4) 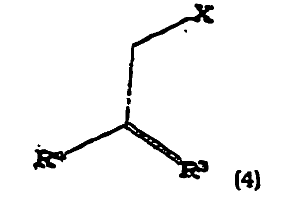 avec un composé représenté par la formule générale (5) 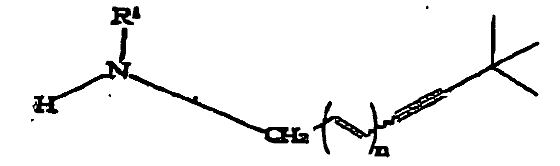 dans laquelle X représente un atome d'halogène.

8. Agent antifongique consistant en les dérivés d'amine ou les sels de ceux-ci selon l'une quelconque des revendications 1 à 4.

9. Composition antifongique comprenant les dérivés d'amine ou les sels de ceux-ci selon l'une quelconque des revendications 1 à 4.

10. Composition pharmaceutique comprenant, comme ingrédient actif, des dérivés d'amine ou des sels de ceux-ci selon l'une quelconque des revendications 1 à 4.
